(19) Europäisches Patentamt
European Patent Office
Office européen des brevets

(11) EP 4 603 492 A1

(12) **EUROPEAN PATENT APPLICATION**
published in accordance with Art. 153(4) EPC

(43) Date of publication:
**20.08.2025 Bulletin 2025/34**

(21) Application number: **23877355.0**

(22) Date of filing: **13.10.2023**

(51) International Patent Classification (IPC):
***C07D 471/04*** *(2006.01)* ***A01M 1/20*** *(2006.01)*
***A01N 43/40*** *(2006.01)* ***A01N 43/50*** *(2006.01)*
***A01N 43/54*** *(2006.01)* ***A01N 43/56*** *(2006.01)*
***A01N 43/58*** *(2006.01)* ***A01N 43/60*** *(2006.01)*
***A01N 43/653*** *(2006.01)* ***A01N 43/90*** *(2006.01)*
***A01N 47/02*** *(2006.01)* ***A01N 53/14*** *(2006.01)*
***A01P 7/02*** *(2006.01)* ***A01P 7/04*** *(2006.01)*
***A61K 31/497*** *(2006.01)* ***A61K 31/501*** *(2006.01)*
***A61P 33/14*** *(2006.01)*

(52) Cooperative Patent Classification (CPC):
**A01M 1/20; A01N 43/40; A01N 43/50; A01N 43/54; A01N 43/56; A01N 43/58; A01N 43/60; A01N 43/653; A01N 43/90; A01N 47/02; A01N 53/00; A01P 7/02; A01P 7/04; A61K 31/497; A61K 31/501;** (Cont.)

(86) International application number:
**PCT/JP2023/037148**

(87) International publication number:
**WO 2024/080354 (18.04.2024 Gazette 2024/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.10.2022 JP 2022165593**

(71) Applicant: **Sumitomo Chemical Company, Limited**
**Tokyo 103-6020 (JP)**

(72) Inventors:
- **SHIODA, Takayuki**
  **Takarazuka-shi, Hyogo 665-8555 (JP)**
- **DOTA, Koichiro**
  **Takarazuka-shi, Hyogo 665-8555 (JP)**
- **NISHIBU, Saki**
  **Takarazuka-shi, Hyogo 665-8555 (JP)**
- **SAITO, Yasumasa**
  **Narashino-shi, Chiba 275-0026 (JP)**

(74) Representative: **Vossius & Partner**
**Patentanwälte Rechtsanwälte mbB**
**Siebertstrasse 3**
**81675 München (DE)**

(54) **SULFONAMIDE COMPOUND AND HARMFUL-ARTHROPOD-CONTROLLING COMPOSITION CONTAINING SAME**

(57) The present invention provides a compound which has excellent control efficacy against harmful arthropods and is represented by formula (I) wherein Q represents a group represented by formula Q1, etc., $R^{2a}$ and $R^{2b}$ are identical to or different from each other, and each represents a C1-C6 alkyl group optionally substituted with one or more halogen atoms, etc., and Het represents a group represented by formula Het1, etc., or an N-oxide thereof.

EP 4 603 492 A1

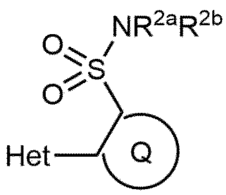

( I )

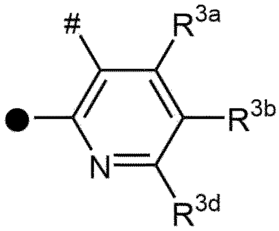

Q1

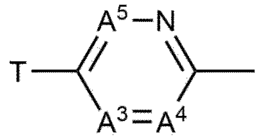

Het1

(52) Cooperative Patent Classification (CPC): (Cont.)
**A61P 33/14; C07D 471/04**

## Description

[Technical Field]

This patent application claims priority to and the benefits under the Paris convention of Japanese Patent Application No. 2022-165593 filed on October 14, 2022, the entire contents of which are incorporated herein by reference.
The present invention relates to sulfonamide compounds and harmful arthropod control compositions comprising the same.

[Background Art]

Heretofore, various compounds have been studied in order to control harmful arthropods. For example, Patent Document 1 discloses that certain kinds of compounds have control effects on pests.

[Prior Art Document]

[Patent Document]

[Patent Document 1] WO 2016/052455 A

[Disclosure of the Invention]

[Problems to be Solved by the Invention]

An object of the present invention is to provide compounds having excellent control efficacy against harmful arthropods.

[Means for Solving the Problems]

The present inventors have studied to find a compound having excellent control efficacy against harmful arthropods, and found that a compound represented by following formula (I) has excellent control efficacy against harmful arthropods.
Namely, the present invention is as follows.

[1] A compound represented by formula (I):

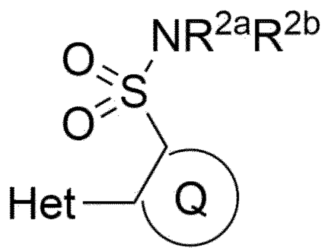

( I )

[wherein,

Q represented by the following formula:

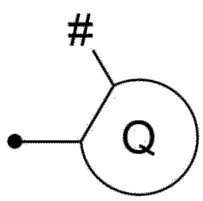

represents a group represented by formula Q1, a group represented by formula Q2, a group represented by formula Q4, a group represented by formula Q5, a group represented by formula Q6, or a group represented by

formula Q7 (wherein # represents the binding site to the sulfur atom, and • represents the binding site to Het):

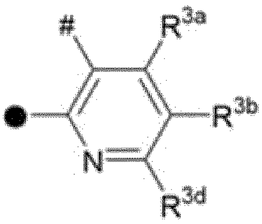

Q1

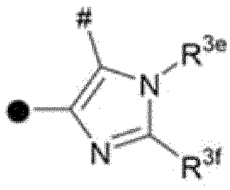

Q2

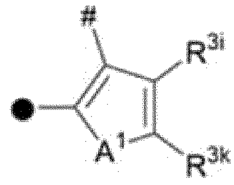

Q4

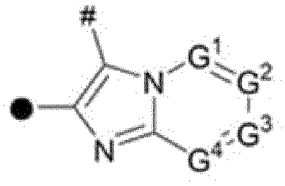

Q5

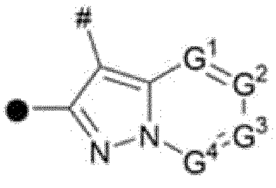

Q6

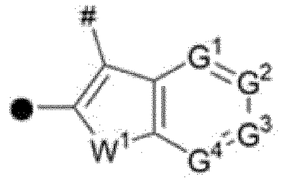

Q7

$A^1$ represents $NR^{5a}$, an oxygen atom or a sulfur atom,
the combination of $G^1$, $G^2$, $G^3$ and $G^4$ represents:

a combination wherein $G^1$ is a nitrogen atom or $CR^{3a}$, $G^2$ is $CR^{3b}$, $G^3$ is $CR^{3d}$, and $G^4$ is $CR^{3c}$;
a combination wherein $G^1$ is $CR^{3a}$, $G^2$ is a nitrogen atom, $G^3$ is $CR^{3d}$, and $G^4$ is $CR^{3c}$;
a combination wherein $G^1$ is $CR^{3a}$, $G^2$ is $CR^{3b}$, $G^3$ is a nitrogen atom, and $G^4$ is $CR^{3c}$; or
a combination wherein $G^1$ is $CR^{3a}$, $G^2$ is $CR^{3b}$, $G^3$ is $CR^{3d}$, and $G^4$ is a nitrogen atom,
$W^1$ represents an oxygen atom or a sulfur atom,
$R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom,
$R^{2a}$ and $R^{2b}$ may be combined with the nitrogen atom to which they are attached to form an aziridinyl group, an azetidinyl group, a pyrrolidinyl group, or a piperidyl group,
$R^{3a}$, $R^{3b}$, $R^{3c}$, $R^{3d}$, $R^{3f}$, $R^{3i}$ and $R^{3k}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5- or 6-membered aromatic heterocyclic group optionally substituted with one or more substituents selected from Group H, $OR^{12}$, $NR^{11}R^{12}$, $NR^{11a}R^{12a}$, $NR^{24}NR^{11}R^{12}$, $NR^{24}OR^{11}$, $NR^{11}C(O)R^{13}$, $NR^{24}NR^{11}C(O)R^{13}$, $NR^{11}C(O)OR^{14}$, $NR^{24}NR^{11}C(O)OR^{14}$, $NR^{11}C(O)NR^{31}R^{32}$, $NR^{24}NR^{11}C(O)NR^{31}R^{32}$, $N{=}CHNR^{31}R^{32}$, $N{=}S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}{=}NOR^{17}$, $NR_{11}CR^{24}{=}NOR^{17}$, $S(O)_qR^{23}$, a cyano group, a nitro group, a hydrogen atom, or a halogen atom,
$R^{3e}$ represents a C1-C6 alkyl group optionally substituted with one or more halogen atoms, a phenyl group optionally substituted with one or more substituents selected from Group H, or a hydrogen atom,
when Q is a group represented by formula Q1, the two adjacent substituents among $R^{3a}$, $R^{3b}$ and $R^{3d}$ may be combined with two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {the benzene ring, the pyrrole ring, the furan ring, the thiophene ring, the pyrazole ring, the imidazole ring, the oxazole ring, the isoxazole ring, the thiazole ring, the isothiazole ring, the pyridine ring, the pyridazine ring, the pyrimidine ring and the pyrazine ring may be optionally substituted with one or more substituents selected from Group H}, or a triazole ring optionally substituted with one or more substituents selected from Group I,
p represents 0 or 1,
q represents 0, 1 or 2,
$R^{30}$ represents a C1-C6 chain hydrocarbon group optionally substituted with one or more halogen atoms, a halogen atom, $OR^{35}$, $NR^{36}R^{37}$, or a hydrogen atom,

$R^{17}$ represents a C1-C6 chain hydrocarbon group optionally substituted with one or more halogen atoms, a phenyl group optionally substituted with one or more substituents selected from Group D, or a hydrogen atom,

$R^{12}$ represents a C1-C6 chain hydrocarbon group optionally substituted with one or more substituents selected from Group F, a C3-C7 cycloalkyl group optionally substituted with one or more substituents selected from Group J, a C3-C7 cycloalkenyl group optionally substituted with one or more substituents selected from Group J, a phenyl group optionally substituted with one or more substituents selected from Group D, a 6-membered aromatic heterocyclic group optionally substituted with one or more substituents selected from Group D, a hydrogen atom, or $S(O)_2R^{23}$,

$R^{23}$ represents a C1-C6 chain hydrocarbon group optionally substituted with one or more halogen atoms, or a phenyl group optionally substituted with one or more substituents selected from Group D,

$R^{11a}$ and $R^{12a}$ , together with the nitrogen atoms to which they are attached, represent a 3- to 7-membered nonaromatic heterocyclic group optionally substituted with one or more substituents selected from Group E,

$R^{13}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, a phenyl group optionally substituted with one or more substituents selected from Group D, a 5- or 6-membered aromatic heterocyclic group optionally substituted with one or more substituents selected from Group D, or a hydrogen atom,

$R^{14}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, or a phenyl C1-C3 alkyl group {the phenyl moiety in the phenyl C1-C3 alkyl group may be optionally substituted with one or more substituents selected from Group D},

$R^{15}$ and $R^{16}$ are identical to or different from each other, and each represents a C1-C6 alkyl group optionally substituted with one or more halogen atoms,

$R^{31}$ represents a C1-C6 alkyl group optionally substituted with one or more halogen atoms, or a hydrogen atom,

$R^{32}$ represents a C1-C6 chain hydrocarbon group optionally substituted with one or more substituents selected from Group F, a C3-C7 cycloalkyl group optionally substituted with one or more substituents selected from Group J, or a hydrogen atom,

Het represents a group represented by formula Het1, a group represented by formula Het2, a group represented by formula Het3, a group represented by formula Het4, a group represented by formula Het5, or a group represented by formula Het6:

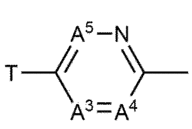
Het1

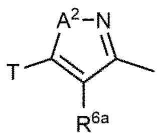
Het2

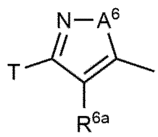
Het3

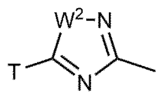
Het4

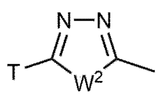
Het5

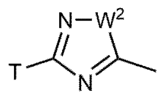
Het6

$A^2$ represents an oxygen atom, a sulfur atom, or $NR^{5b}$,

$A^3$ represents a nitrogen atom or $CR^{4b}$,

$A^4$ represents a nitrogen atom or $CR^{4c}$,

$A^5$ represents a nitrogen atom or $CR^{4d}$,

$A^6$ represents an oxygen atom, a sulfur atom, or $NR^{20}$,

$W^2$ represents an oxygen atom or a sulfur atom,

$R^{4b}$, $R^{4c}$, $R^{4d}$ and $R^{6a}$ are identical to or different from each other, and each represents a C1-C6 chain hydrocarbon group optionally substituted with one or more halogen atoms, a nitro group, $OR^{18}$, $NR^{18}R^{19}$, a cyano group, an amino group, a halogen atom, or a hydrogen atom,

$R^{20}$ is identical to or different from each other, and represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, or a (C3-C7 cycloalkyl) C1-C6 alkyl group optionally substituted with one or more halogen atoms,

T represents a C1-C10 chain hydrocarbon group, a (C1-C5 alkoxy) C2-C5 alkyl group, a (C3-C5 alkenyloxy) C2-C5 alkyl group, a (C3-C5 alkynyloxy) C2-C5 alkyl group, a (C1-C5 alkyl)-$S(O)_w$-(C2-C5 alkyl) group, a (C3-C5 alkenyl)-$S(O)_w$-(C2-C5 alkyl) group, a (C3-C5 alkynyl)-$S(O)_w$-(C2-C5 alkyl) group, a (C1-C5 alkyl)-C(O)-(C1-C5 alkyl) group {the C1-C10 chain hydrocarbon group, the (C1-C5 alkoxy) C2-C5 alkyl

group, the (C3-C5 alkenyloxy) C2-C5 alkyl group, the (C3-C5 alkynyloxy) C2-C5 alkyl group, the (C1-C5 alkyl)-$S(O)_w$-(C2-C5 alkyl) group, the (C3-C5 alkenyl)-$S(O)_w$-(C2-C5 alkyl) group, the (C3-C5 alkynyl)-$S(O)_w$-(C2-C5 alkyl) group, and the (C1-C5 alkyl)-C(O)-(C1-C5 alkyl) group are substituted with one or more substituents selected from the group consisting of a cyano group and a halogen atom}, a (C3-C7 cycloalkyl) C1-C3 alkyl group which is substituted with one or more substituents selected from Group G, a C3-C7 cycloalkyl group which is substituted with one or more substituents selected from Group G, $OR^1$, $S(O)_vR^1$, $OS(O)_2R^1$, $CH_2OR^1$, $NR^1R^{29}$, $C(O)R^1$, $C(O)NR^1R^{29}$, $NR^{29}C(O)R^1$, $N{=}CR^1R^{30}$, a group represented by formula T-1, a group represented by formula T-2, a group represented by formula T-3, a group represented by formula T-4, a group represented by formula T-5, a group represented by formula T-6, a group represented by formula T-7, a group represented by formula T-8, a group represented by formula T-9, a group represented by formula T-10, a group represented by formula T-11, or a group represented by formula T-12:

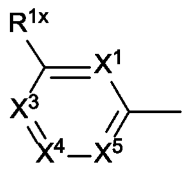
T-1

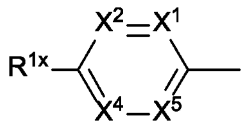
T-2

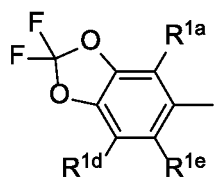
T-3

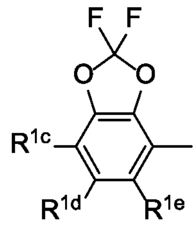
T-4

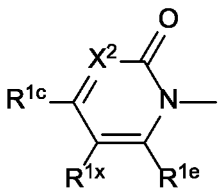
T-5

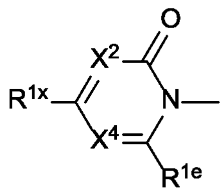
T-6

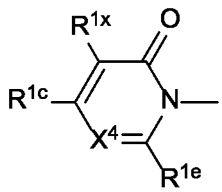
T-7

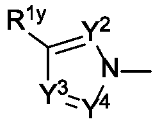
T-8

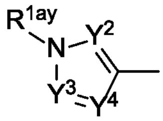
T-9

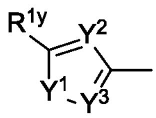
T-10

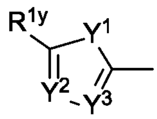
T-11

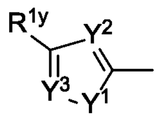
T-12

$X^1$ represents a nitrogen atom or $CR^{1a}$,
$X^2$ represents a nitrogen atom or $CR^{1b}$,
$X^3$ represents a nitrogen atom or $CR^{1c}$,
$X^4$ represents a nitrogen atom or $CR^{1d}$,
$X^5$ represents a nitrogen atom or $CR^{1e}$,
$R^{1x}$ represents a C1-C5 chain hydrocarbon group which is substituted with one or more halogen atoms, $OR^7$, $OS(O)_2R^7$, $S(O)_mR^7$, $NR^8S(O)_2R^7$, or a halogen atom,
$R^{1a}$, $R^{1b}$, $R^1$, $R^{1d}$ and $R^{1e}$ are identical to or different from each other, and each represents a C1-C6 chain hydrocarbon group optionally substituted with one or more halogen atoms, a C3-C6 cycloalkyl group optionally substituted with one or more halogen atoms, a halogen atom, or a hydrogen atom,
$Y^1$ represents $NR^{25}$, an oxygen atom or a sulfur atom,
$Y^2$ represents a nitrogen atom or $CR^{26}$,
$Y^3$ represents a nitrogen atom or $CR^{27}$,
$Y^4$ represents a nitrogen atom or $CR^{28}$,
$R^{5a}$, $R^{5b}$ and $R^{25}$ are identical to or different from each other, and each represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom,
$R^{26}$, $R^{27}$ and $R^{28}$ are identical to or different from each other, and each represents a C1-C6 chain hydrocarbon group optionally substituted with one or more halogen atoms, a C3-C6 cycloalkyl group optionally substituted

with one or more halogen atoms, a halogen atom, or a hydrogen atom,
$R^{1y}$ represents a C1-C5 chain hydrocarbon group which is substituted with one or more halogen atoms, $OR^7$, $OS(O)_2R^7$, $S(O)_mR^7$, $NR^8S(O)_2R^7$, a cyano group, or a halogen atom,
$R^{1ay}$ and $R^7$ are identical to or different from each other, and each represents a C1-C6 chain hydrocarbon group which is substituted with one or more halogen atoms,
m and v are identical to or different from each other, and each represents 0, 1 or 2,
w and t are identical to or different from each other, and each represents 0, 1 or 2,
$R^1$ represents a C1-C10 chain hydrocarbon group, a (C1-C5 alkoxy) C2-C5 alkyl group, a (C3-C5 alkenyloxy) C2-C5 alkyl group, a (C3-C5 alkynyloxy) C2-C5 alkyl group, a (C1-C5 alkyl)-$S(O)_t$-(C2-C5 alkyl) group, a (C3-C5 alkenyl)-S(O)t-(C2-C5 alkyl) group, a (C3-C5 alkynyl)-S(O)t-(C2-C5 alkyl) group, a (C1-C5 alkyl)-C(O)-(C1-C5 alkyl) group {the C1-C10 chain hydrocarbon group, the (C1-C5 alkoxy) C2-C5 alkyl group, the (C3-C5 alkenyloxy) C2-C5 alkyl group, the (C3-C5 alkynyloxy) C2-C5 alkyl group, the (C1-C5 alkyl)-S(O)t-(C2-C5 alkyl) group, the (C3-C5 alkenyl)-S(O)t-(C2-C5 alkyl) group, the (C3-C5 alkynyl)-S(O)t-(C2-C5 alkyl) group, and the (C1-C5 alkyl)-C(O)-(C1-C5 alkyl) group are substituted with one or more substituents selected from the group consisting of a cyano group and a halogen atom}, a (C3-C7 cycloalkyl) C1-C3 alkyl group which is substituted with one or more substituents selected from Group G, or a C3-C7 cycloalkyl group which is substituted with one or more substituents selected from Group G,
$R^{18}$ and $R^{35}$ are identical to or different from each other, and each represents a C1-C6 chain hydrocarbon group optionally substituted with one or more halogen atoms, and
$R^{88}$, $R^{11}$, $R^{19}$, $R^{24}$, $R^{29}$, $R^{36}$ and $R^{37}$ are identical to or different from each other, and each represents a C1-C6 chain hydrocarbon group optionally substituted with one or more halogen atoms, or a hydrogen atom.
Group B: the group consisting of a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a C3-C6 cycloalkyl group optionally substituted with one or more halogen atoms, a cyano group, a hydroxy group, and a halogen atom.
Group C: the group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, and a halogen atom.
Group D: the group consisting of a C1-C6 chain hydrocarbon group optionally substituted with one or more halogen atoms, a hydroxy group, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a sulfanyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom.
$R^{21}$ and $R^{22}$ are identical to or different from each other, and each represents a C1-C6 alkyl group optionally substituted with one or more halogen atoms.
Group E: the group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a halogen atom, an oxo group, a hydroxy group, a cyano group, and a nitro group.
Group F: the group consisting of a C1-C6 alkoxy group optionally substituted with one or more halogen atoms, a phenyl group optionally substituted with one or more substituents selected from Group D, a 5- or 6-membered aromatic heterocyclic group optionally substituted with one or more substituents selected from Group D, a C3-C7 cycloalkyl group optionally substituted with one or more halogen atoms, a 3- to 7-membered nonaromatic heterocyclic group optionally substituted with one or more substituents selected from Group C, an amino group, $NHR^{21}$, $NR^{21}R^{22}$, a halogen atom, and a cyano group.
Group G: the group consisting of a halogen atom, a C1-C6 haloalkyl group and a cyano group.
Group H: the group consisting of a C1-C6 alkyl group optionally substituted with one or more halogen atoms, $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{10}$, a halogen atom, a nitro group, a cyano group, an amino group, and a 5- or 6-membered aromatic heterocyclic group.

$R^9$ represents a C1-C6 alkyl group optionally substituted with one or more halogen atoms, or a C3-C6 cycloalkyl group optionally substituted with one or more halogen atoms, and

$R^{10}$ represents a C1-C6 alkyl group optionally substituted with one or more halogen atoms, a C3-C6 cycloalkyl group optionally substituted with one or more halogen atoms, or a hydrogen atom.

Group I: the group consisting of a C2-C6 chain hydrocarbon group optionally substituted with one or more halogen atoms, a C3-C6 cycloalkyl group optionally substituted with one or more halogen atoms, a phenyl group optionally substituted with one or more substituents selected from Group D, a 5- or 6-membered aromatic heterocyclic group optionally substituted with one or more substituents selected from Group D, a C2-C6 alkylcarbonyl group optionally substituted with one or more halogen atoms, a C2-C6 alkoxycarbonyl group optionally substituted with one or more halogen atoms, an aminocarbonyl group, a (C1-C6 alkyl)aminocarbonyl group optionally substituted with one or more halogen atoms, a methyl group, and a di(C1-C4 alkyl)aminocarbonyl group optionally substituted with one or more halogen atoms.

Group J: the group consisting of a C1-C6 alkyl group optionally substituted with one or more halogen atoms, a halogen atom, and a cyano group]

(hereinafter referred to as present compound N) or an N-oxide thereof (hereinafter a compound represented by formula (I) or an N-oxide thereof is referred to as present compound).

[2] The compound according to [1], wherein Q is a group represented by formula Q4 or a group represented by formula Q7, or an N-oxide thereof.

[3] The compound according to [1], wherein Q is a group represented by formula Q5 or a group represented by formula Q6, or an N-oxide thereof.

[4] The compound according to [1], wherein Q is a group represented by formula Q1 or a group represented by formula Q2, or an N-oxide thereof.

[5] The compound according to [1], wherein Q is a group represented by formula Q5, or an N-oxide thereof.

[6] The compound according to [5], wherein $G^1$ is $CR^{3a}$, $G^2$ is $CR^{3b}$, $G^3$ is $CR^{3d}$, and $G^4$ is $CR^{3c}$, or an N-oxide thereof.

[7] The compound according to any one of [1] to [6], wherein Het is a group represented by formula Het1, $A^3$ is a nitrogen atom or $CR^{4b}$, $A^4$ is $CR^{4c}$, and $A^5$ is a nitrogen atom or $CR^{4d}$, or an N-oxide thereof.

[8] A harmful arthropod control composition comprising the compound according to any one of [1] to [7], or an N-oxide thereof, and an inert carrier.

[9] A composition comprising one or more ingredients selected from the group consisting of Group (a), Group (b), Group (c) and Group (d), and the compound according to any one of [1] to [7], or an N-oxide thereof:

Group (a): the group consisting of insecticidal active ingredients, miticidal active ingredients and nematicidal active ingredients;

Group (b): fungicidal active ingredients;

Group (c): plant growth regulatory ingredients; and

Group (d): repellent ingredients.

[10] A method for controlling harmful arthropods, which comprises applying an effective amount of the compound according to any one of [1] to [7], or an N-oxide thereof, or an effective amount of the composition according to [9] to harmful arthropods or habitats where harmful arthropods live.

[11] A seed or a vegetative reproduction organ holding an effective amount of the compound according to any one of [1] to [7], or an N-oxide thereof, or an effective amount of the composition according to [9].

[Effects of the Invention]

According to the present invention, harmful arthropods can be controlled.

[Mode for Carrying Out the Invention]

The substituents in the present invention will be described below.

A halogen atom means a fluorine atom, a chlorine atom, a bromine atom, or an iodine atom.

When the substituent is substituted with two or more halogen atoms or substituents, those halogen atoms or substituents may be identical to or different from each other.

The expression "CX-CY" as used herein means that the number of carbon atoms is X to Y. For example, the expression "C1-C6" means that the number of carbon atoms is 1 to 6.

The chain hydrocarbon group represents an alkyl group, an alkenyl group, or an alkynyl group.

Examples of the alkyl group include a methyl group, an ethyl group, a propyl group, an isopropyl group, a 1,1-dimethylpropyl group, a 1,2-dimethylpropyl group, a 1-ethylpropyl group, a butyl group, a sec-butyl group, a tert-butyl group, a pentyl group, and a hexyl group.

Examples of the alkenyl group include a vinyl group, a 1-propenyl group, a 2-propenyl group, a 1-methyl-1-propenyl group, a 1-methyl-2-propenyl group, a 1,2-dimethyl-1-propenyl group, a 1-ethyl-2-propenyl group, a 3-butenyl group, a 4-pentenyl group, and a 5-hexenyl group.

Examples of the alkynyl group include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, and a 5-hexynyl group.

Examples of the alkoxy group include a methoxy group, an ethoxy group, a propoxy group, an isopropoxy group, a butoxy group, a tert-butoxy group, a pentyloxy group, and a hexyloxy group.

Examples of the alkenyloxy group include a 2-propenyloxy group, a 2-butenyloxy group, and a 5-hexenyloxy group.

Examples of the alkynyloxy group include a 2-propynyloxy group, a 2-buthynyloxy group, and a 5-hexynyloxy group.

Examples of the cycloalkyl group include a cyclopropyl group, a cyclobutyl group, a cyclopentyl group, a cyclohexyl group, and a cycloheptyl group.

Examples of the cycloalkenyl group include a cyclopropenyl group, a cyclobutenyl group, a cyclopentenyl group, a cyclohexenyl group, and a cycloheptenyl group.

Examples of the 3- to 7-membered nonaromatic heterocyclic group include an aziridinyl group, an oxiranyl group, a thiiranyl group, an azetidinyl group, an oxetanyl group, a thietanyl group, a pyrrolidinyl group, a tetrahydrofuranyl group, a tetrahydrothienyl group, a piperidyl group, a pyranyl group, a dihydropyranyl group, a tetrahydropyranyl group, a tetrahydrothiopyranyl group, an azepanyl group, an oxepanyl group, a thiepanyl group, a pyrazolinyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolyl group, a dioxolanyl group, a dioxanyl group, a morpholinyl group, a thiomorpholinyl group, and a piperazinyl group.

Examples of the 5- or 6-membered aromatic heterocyclic group include a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a pyridyl group, a pyridazinyl group, a pyrimidinyl group, a pyrazinyl group, a triazinyl group, and a tetrazinyl group.

Examples of the 6-membered aromatic heterocyclic group include a pyridyl group, a pyridazinyl group, a pyrimidinyl group, a pyrazinyl group, a triazinyl group, and a tetrazinyl group.

Examples of the (C3-C6 cycloalkyl) C1-C3 alkyl group optionally substituted with one or more halogen atoms include a cyclopropylmethyl group, a (2-fluorocyclopropyl)methyl group, a cyclopropyl(fluoro)methyl group, and a (2-fluorocyclopropyl) (fluoro)methyl group.

Examples of the (C1-C5 alkoxy) C2-C5 alkyl group include a 2-methoxyethyl group, a 3-methoxypropyl group, and a 3-ethoxypropyl group.

The (C3-C7 cycloalkyl) C1-C6 alkyl group optionally substituted with one or more halogen atoms represents a group wherein (C3-C7 cycloalkyl) and/or (C1-C6 alkyl) may be optionally substituted with one or more halogen atoms, and examples thereof include a (2,2-difluorocyclopropyl)methyl group, a 2-cyclopropyl-1,1,2,2-tetrafluoroethyl group, and a 2-(2,2-difluorocyclopropyl)-1,1,2,2-tetrafluoroethyl group, a (2,2-difluorocyclopropyl)propyl group, a (2,2-difluorocyclopropyl)butyl group, a (2,2-difluorocyclopropyl)pentyl group, and a (2,2-difluorocyclopropyl)hexyl group.

The (C3-C7 cycloalkyl) C1-C3 alkyl group which is substituted with one or more substituents selected from Group G represents a group wherein (C3-C7 cycloalkyl) and/or (C1-C3 alkyl) is/are substituted with one or more substituents selected from Group G, and examples thereof include a (2,2-difluorocyclopropyl)methyl group, a [1-(trifluoromethyl) cyclopropyl]methyl group, a [2-(trifluoromethyl)cyclopropyl]methyl group, a 2-cyclopropyl-1,1,2,2-tetrafluoroethyl group, a 2-cyclopropyl-3,3,3-trifluoropropyl group, and a 1,1,2,2-tetrafluoro-2-[2-(trifluoromethyl)cyclopropyl]ethyl group.

Examples of the alkylsulfanyl group include a methylsulfanyl group, an ethylsulfanyl group, a propylsulfanyl group, and an isopropylsulfanyl group.

Examples of the alkylsulfinyl group include a methylsulfinyl group, an ethylsulfinyl group, a propylsulfinyl group, and an isopropylsulfinyl group.

Examples of the alkylsulfonyl group include a methylsulfonyl group, an ethylsulfonyl group, a propylsulfonyl group, and an isopropylsulfonyl group.

Examples of the alkylcarbonyl group include an acetyl group, a propanoyl group, a 2-methylpropanoyl group, and a hexanoyl group.

Examples of the alkoxycarbonyl group include a methoxycarbonyl group, an ethoxycarbonyl group, an isopropoxycarbonyl group, and a pentyloxycarbonyl group.

Examples of the N-oxide of the compound represented by formula (I) include a compound represented by the following formula:

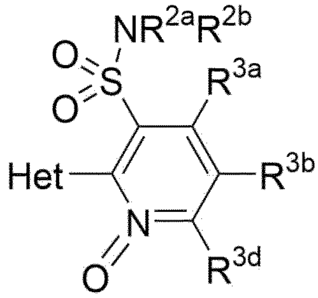

[wherein symbols are the same as defined above.]

The present compound may have one or more stereoisomers. Examples of the stereoisomer include enantiomers, diastereomers, and geometric isomers. The present compound includes each stereoisomer and mixtures of stereoisomers in any ratio.

The present compound may form an acid addition salt. Examples of the acid to form the acid addition salt include inorganic acids such as hydrogen chloride, phosphoric acid and sulfuric acid; and organic acids such as acetic acid, trifluoroacetic acid benzoic acid and p-toluenesulfonic acid. The acid addition salt can be obtained by mixing the present compound with the acid.

Examples of aspects of the present compound N include the following compounds.

[Aspect 1] The present compound N wherein Q is a group represented by formula Q4 or a group represented by formula Q7.

[Aspect 2] The present compound N wherein Q is a group represented by formula Q5 or a group represented by formula Q6.

[Aspect 3] The present compound N wherein Q is a group represented by formula Q1 or a group represented by formula Q2.

[Aspect 4] The present compound N wherein Q is a group represented by formula Q5.

[Aspect 5] The present compound N wherein Q is formula Q5, $G^1$ is $CR^{3a}$, $G^2$ is $CR^{3b}$, $G^3$ is $CR^{3d}$, and $G^4$ is $CR^{3c}$.

[Aspect 6] The present compound N wherein Q is formula Q5, $G^1$ is $CR^{3a}$, $G^2$ is $CR^{3b}$, $G^3$ is $CR^{3d}$, $G^4$ is $CR^{3c}$, $R^{3a}$ is a hydrogen atom, $R^{3b}$ is a C1-C6 chain hydrocarbon group optionally substituted with one or more substituents selected from Group B, a hydrogen atom, or a halogen atom, $R^{3c}$ is a hydrogen atom, and $R^{3d}$ is a C1-C6 chain hydrocarbon group optionally substituted with one or more substituents selected from Group B, a hydrogen atom, or a halogen atom.

[Aspect 7] The present compound N wherein Q is formula Q5, $G^1$ is $CR^{3a}$, $G^2$ is $CR^{3b}$, $G^3$ is $CR^{3d}$, $G^4$ is $CR^{3c}$, $R^{3a}$ is a hydrogen atom, $R^{3b}$ is a C1-C6 chain hydrocarbon group optionally substituted with one or more substituents selected from Group B, or a halogen atom, $R^{3c}$ is a hydrogen atom, and $R^{3d}$ is a hydrogen atom.

[Aspect 8] The present compound N wherein Q is formula Q5, $G^1$ is $CR^{3a}$, $G^2$ is $CR^{3b}$, $G^3$ is $CR^{3d}$, $G^4$ is $CR^{3c}$, $R^{3a}$, $R^{3c}$, and $R^{3d}$ are a hydrogen atom, and $R^{3b}$ is a halogen atom.

[Aspect 9] The present compound N wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 10] The present compound N wherein Het is a group represented by formula Het4, or a group represented by formula Het5, and $W^2$ is an oxygen atom.

[Aspect 11] The present compound N wherein Het is a group represented by formula Het2, or a group represented by formula Het3, $A^2$ and $A^6$ are an oxygen atom, and $R^{6a}$ is a hydrogen atom.

[Aspect 12] The present compound N wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 13] The compound according to Aspect 1 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 14] The compound according to Aspect 2 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 15] The compound according to Aspect 3 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 16] The compound according to Aspect 4 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 17] The compound according to Aspect 5 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 18] The compound according to Aspect 6 wherein Het is a group represented by formula Het1, $A^3$ is a nitrogen

atom or $CR^{4b}$, $A^4$ is $CR^{4c}$, and $A^5$ is a nitrogen atom or $CR^{4d}$.

[Aspect 19] The compound according to Aspect 7 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 20] The compound according to Aspect 8 wherein Het is a group represented by formula Het1, $A^3$ is a nitrogen atom or $CR^{4b}$, $A^4$ is $CR^{4c}$, and $A^5$ is a nitrogen atom or $CR^{4d}$.

[Aspect 21] The compound according to Aspect 1 wherein Het is a group represented by formula Het4, or a group represented by formula Het5, and $W^2$ is an oxygen atom.

[Aspect 22] The compound according to Aspect 2 wherein Het is a group represented by formula Het4, or a group represented by formula Het5, and $W^2$ is an oxygen atom.

[Aspect 23] The compound according to Aspect 3 wherein Het is a group represented by formula Het4, or a group represented by formula Het5, and $W^2$ is an oxygen atom.

[Aspect 24] The compound according to Aspect 4 wherein Het is a group represented by formula Het4, or a group represented by formula Het5, and $W^2$ is an oxygen atom.

[Aspect 25] The compound according to Aspect 5 wherein Het is a group represented by formula Het4, or a group represented by formula Het5, and $W^2$ is an oxygen atom.

[Aspect 26] The compound according to Aspect 6 wherein Het is a group represented by formula Het4, or a group represented by formula Het5, and $W^2$ is an oxygen atom.

[Aspect 27] The compound according to Aspect 7 wherein Het is a group represented by formula Het4, or a group represented by formula Het5, and $W^2$ is an oxygen atom.

[Aspect 28] The compound according to Aspect 8 wherein Het is a group represented by formula Het4, or a group represented by formula Het5, and $W^2$ is an oxygen atom.

[Aspect 29] The compound according to Aspect 1 wherein Het is a group represented by formula Het2, or a group represented by formula Het3, $A^2$ and $A^6$ are an oxygen atom, and $R^{6a}$ is a hydrogen atom.

[Aspect 30] The compound according to Aspect 2 wherein Het is a group represented by formula Het2, or a group represented by formula Het3, $A^2$ and $A^6$ are an oxygen atom, and $R^{6a}$ is a hydrogen atom.

[Aspect 31] The compound according to Aspect 3 wherein Het is a group represented by formula Het2, or a group represented by formula Het3, $A^2$ and $A^6$ are an oxygen atom, and $R^{6a}$ is a hydrogen atom.

[Aspect 32] The compound according to Aspect 4 wherein Het is a group represented by formula Het2, or a group represented by formula Het3, $A^2$ and $A^6$ are an oxygen atom, and $R^{6a}$ is a hydrogen atom.

[Aspect 33] The compound according to Aspect 5 wherein Het is a group represented by formula Het2, or a group represented by formula Het3, $A^2$ and $A^6$ are an oxygen atom, and $R^{6a}$ is a hydrogen atom.

[Aspect 34] The compound according to Aspect 6 wherein Het is a group represented by formula Het2, or a group represented by formula Het3, $A^2$ and $A^6$ are an oxygen atom, and $R^{6a}$ is a hydrogen atom.

[Aspect 35] The compound according to Aspect 7 wherein Het is a group represented by formula Het2, or a group represented by formula Het3, $A^2$ and $A^6$ are an oxygen atom, and $R^{6a}$ is a hydrogen atom.

[Aspect 36] The compound according to Aspect 8 wherein Het is a group represented by formula Het2, or a group represented by formula Het3, $A^2$ and $A^6$ are an oxygen atom, and $R^{6a}$ is a hydrogen atom.

[Aspect 37] The compound according to Aspect 1 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$, and $R^{4d}$ are a hydrogen atom.

[Aspect 38] The compound according to Aspect 2 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 39] The compound according to Aspect 3 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 40] The compound according to Aspect 4 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 41] The compound according to Aspect 5 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 42] The compound according to Aspect 6 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 43] The compound according to Aspect 7 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 44] The compound according to Aspect 8 wherein Het is a group represented by formula Het1, $A^3$ is a nitrogen atom or $CR^{4b}$, $A^4$ is $CR^{4c}$, $A^5$ is a nitrogen atom or $CR^{4d}$, and $R^{4b}$, $R^{4c}$ and $R^{4d}$ are a hydrogen atom.

[Aspect 45] The present compound N wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 46] The present compound N wherein $R^{2a}$ and $R^{2b}$ are identical to or different from each other, and are a C1-C6 alkyl group optionally substituted with one or more halogen atoms, or a hydrogen atom.

[Aspect 47] The compound according to Aspect 13 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 48] The compound according to Aspect 14 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 49] The compound according to Aspect 15 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 50] The compound according to Aspect 16 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 51] The compound according to Aspect 17 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 52] The compound according to Aspect 18 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 53] The compound according to Aspect 19 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 54] The compound according to Aspect 20 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 55] The compound according to Aspect 21 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 56] The compound according to Aspect 22 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 57] The compound according to Aspect 23 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 58] The compound according to Aspect 24 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 59] The compound according to Aspect 25 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 60] The compound according to Aspect 26 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 61] The compound according to Aspect 27 wherein $R^{2a}$ and $R^{2b}$ are identical to or different from each other,

and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 62] The compound according to Aspect 28 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 63] The compound according to Aspect 29 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 64] The compound according to Aspect 30 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 65] The compound according to Aspect 31 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 66] The compound according to Aspect 32 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 67] The compound according to Aspect 33 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 68] The compound according to Aspect 34 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 69] The compound according to Aspect 35 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$. together with the nitrogen atoms to which they are attached, form a pyrrolidinyl group or a piperidyl group.

[Aspect 70] The compound according to Aspect 36 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 71] The compound according to Aspect 37 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 72] The compound according to Aspect 38 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 73] The compound according to Aspect 39 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 74] The compound according to Aspect 40 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 75] The compound according to Aspect 41 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen

atoms to which they are attached, form a pyrrolidinyl group or a piperidyl group.

[Aspect 76] The compound according to Aspect 42 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 77] The compound according to Aspect 43 wherein $R^{2a}$ and $R^{2b}$ are identical to or different from each other, and are a C1-C6 alkyl group optionally substituted with one or more halogen atoms, a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 78] The compound according to Aspect 44 wherein $R^{2a}$ and $R^{2b}$ are a C1-C6 alkyl group optionally substituted with one or more halogen atoms, and are a C3-C6 cycloalkyl group optionally substituted with one or more halogen atoms, or a hydrogen atom, or $R^{2a}$ and $R^{2b}$, together with the nitrogen atoms to which they are attached, form a pyrrolidinyl group or a piperidyl group.

[Aspect 79] The compound according to Aspect 13 wherein $R^{2a}$ and $R^{2b}$ are identical to or different from each other, and are a C1-C6 alkyl group optionally substituted with one or more halogen atoms, or a hydrogen atom.

[Aspect 80] The compound according to Aspect 14 wherein $R^{2a}$ and $R^{2b}$ are identical to or different from each other, and are a C1-C6 alkyl group optionally substituted with one or more halogen atoms, or a hydrogen atom.

[Aspect 81] The compound according to Aspect 15 wherein $R^{2a}$ and $R^{2b}$ are identical to or different from each other, and are a C1-C6 alkyl group optionally substituted with one or more halogen atoms, or a hydrogen atom.

[Aspect 82] The compound according to Aspect 16 wherein $R^{2a}$ and $R^{2b}$ are identical to or different from each other, and are a C1-C6 alkyl group optionally substituted with one or more halogen atoms, or a hydrogen atom.

[Aspect 83] The compound according to Aspect 17 wherein $R^{2a}$ and $R^{2b}$ are identical to or different from each other, and are a C1-C6 alkyl group optionally substituted with one or more halogen atoms, or a hydrogen atom.

[Aspect 84] The compound according to Aspect 18 wherein $R^{2a}$ and $R^{2b}$ are identical to or different from each other, and are a C1-C6 alkyl group optionally substituted with one or more halogen atoms, or a hydrogen atom.

[Aspect 85] The compound according to Aspect 19 wherein $R^{2a}$ and $R^{2b}$ are identical to or different from each other, and are a C1-C6 alkyl group optionally substituted with one or more halogen atoms, or a hydrogen atom.

[Aspect 86] The compound according to Aspect 20 wherein $R^{2a}$ and $R^{2b}$ are identical to or different from each other, and are a C1-C6 alkyl group optionally substituted with one or more halogen atoms, or a hydrogen atom.

[Aspect 87] The compound according to Aspect 21 wherein $R^{2a}$ and $R^{2b}$ are identical to or different from each other, and are a C1-C6 alkyl group optionally substituted with one or more halogen atoms, or a hydrogen atom.

[Aspect 88] The compound according to Aspect 22 wherein $R^{2a}$ and $R^{2b}$ are identical to or different from each other, and are a C1-C6 alkyl group optionally substituted with one or more halogen atoms, or a hydrogen atom.

[Aspect 89] The compound according to Aspect 23 wherein $R^{2a}$ and $R^{2b}$ are identical to or different from each other, and are a C1-C6 alkyl group optionally substituted with one or more halogen atoms, or a hydrogen atom.

[Aspect 90] The compound according to Aspect 24 wherein $R^{2a}$ and $R^{2b}$ are identical to or different from each other, and are a C1-C6 alkyl group optionally substituted with one or more halogen atoms, or a hydrogen atom.

[Aspect 91] The compound according to Aspect 25 wherein $R^{2a}$ and $R^{2b}$ are identical to or different from each other, and are a C1-C6 alkyl group optionally substituted with one or more halogen atoms, or a hydrogen atom.

[Aspect 92] The compound according to Aspect 26 wherein $R^{2a}$ and $R^{2b}$ are identical to or different from each other, and are a C1-C6 alkyl group optionally substituted with one or more halogen atoms, or a hydrogen atom.

[Aspect 93] The compound according to Aspect 27 wherein $R^{2a}$ and $R^{2b}$ are identical to or different from each other, and are a C1-C6 alkyl group optionally substituted with one or more halogen atoms, or a hydrogen atom.

[Aspect 94] The compound according to Aspect 28 wherein $R^{2a}$ and $R^{2b}$ are identical to or different from each other, and are a C1-C6 alkyl group optionally substituted with one or more halogen atoms, or a hydrogen atom.

[Aspect 95] The compound according to Aspect 29 wherein $R^{2a}$ and $R^{2b}$ are identical to or different from each other, and are a C1-C6 alkyl group optionally substituted with one or more halogen atoms, or a hydrogen atom.

[Aspect 96] The compound according to Aspect 30 wherein $R^{2a}$ and $R^{2b}$ are identical to or different from each other, and are a C1-C6 alkyl group optionally substituted with one or more halogen atoms, or a hydrogen atom.

[Aspect 97] The compound according to Aspect 31 wherein $R^{2a}$ and $R^{2b}$ are identical to or different from each other, and are a C1-C6 alkyl group optionally substituted with one or more halogen atoms, or a hydrogen atom.

[Aspect 98] The compound according to Aspect 32 wherein $R^{2a}$ and $R^{2b}$ are identical to or different from each other, and are a C1-C6 alkyl group optionally substituted with one or more halogen atoms, or a hydrogen atom.

[Aspect 99] The compound according to Aspect 33 wherein $R^{2a}$ and $R^{2b}$ are identical to or different from each other, and are a C1-C6 alkyl group optionally substituted with one or more halogen atoms, or a hydrogen atom.

[Aspect 100] The compound according to Aspect 34 wherein $R^{2a}$ and $R^{2b}$ are identical to or different from each other, and are a C1-C6 alkyl group optionally substituted with one or more halogen atoms, or a hydrogen atom.

[Aspect 101] The compound according to Aspect 35 wherein $R^{2a}$ and $R^{2b}$ are identical to or different from each other,

and are a C1-C6 alkyl group optionally substituted with one or more halogen atoms, or a hydrogen atom.
[Aspect 102] The compound according to Aspect 36 wherein $R^{2a}$ and $R^{2b}$ are identical to or different from each other, and are a C1-C6 alkyl group optionally substituted with one or more halogen atoms, or a hydrogen atom.
[Aspect 103] The compound according to Aspect 37 wherein $R^{2a}$ and $R^{2b}$ are identical to or different from each other, and are a C1-C6 alkyl group optionally substituted with one or more halogen atoms, or a hydrogen atom.
[Aspect 104] The compound according to Aspect 38 wherein $R^{2a}$ and $R^{2b}$ are identical to or different from each other, and are a C1-C6 alkyl group optionally substituted with one or more halogen atoms, or a hydrogen atom.
[Aspect 105] The compound according to Aspect 39 wherein $R^{2a}$ and $R^{2b}$ are identical to or different from each other, and are a C1-C6 alkyl group optionally substituted with one or more halogen atoms, or a hydrogen atom.
[Aspect 106] The compound according to Aspect 40 wherein $R^{2a}$ and $R^{2b}$ are identical to or different from each other, and are a C1-C6 alkyl group optionally substituted with one or more halogen atoms, or a hydrogen atom.
[Aspect 107] The compound according to Aspect 41 wherein $R^{2a}$ and $R^{2b}$ are identical to or different from each other, and are a C1-C6 alkyl group optionally substituted with one or more halogen atoms, or a hydrogen atom.
[Aspect 108] The compound according to Aspect 42 wherein $R^{2a}$ and $R^{2b}$ are identical to or different from each other, and are a C1-C6 alkyl group optionally substituted with one or more halogen atoms, or a hydrogen atom.
[Aspect 109] The compound according to Aspect 43 wherein $R^{2a}$ and $R^{2b}$ are identical to or different from each other, and are a C1-C6 alkyl group optionally substituted with one or more halogen atoms, or a hydrogen atom.
[Aspect 110] The compound according to Aspect 44 wherein $R^{2a}$ and $R^{2b}$ are identical to or different from each other, and are a C1-C6 alkyl group optionally substituted with one or more halogen atoms, or a hydrogen atom.
[Aspect 111] The compound according to any one of Aspects 1 to 110 or the present compound N wherein T is a group represented by formula T-1 , $X^1$ is $CR^{1a}$, $X^3$ is $CR^{10}$, $X^4$ is a nitrogen atom or $CR^{1d}$, $X^5$ is a nitrogen atom or $CR^{1e}$, $R^{1a}$, $R^{1c}$ and $R^{1e}$ are a hydrogen atom, $R^{1d}$ is a C1-C6 chain hydrocarbon group optionally substituted with one or more halogen atoms, or a halogen atom, $R^{1x}$ is a C1-C5 chain hydrocarbon group which is substituted with one or more halogen atoms, $OR^7$, $OS(O)_2R^7$, $S(O)_mR^7$, $NR^8S(O)_2R^7$, or a halogen atom.
[Aspect 112] The compound according to any one of Aspects 1 to 110 or the present compound N wherein T is a C1-C10 chain hydrocarbon group which is substituted with one or more substituents selected from the group consisting of a cyano group and a halogen atom, or $OR^1$, and $R^1$ is a C1-C10 chain hydrocarbon group which is substituted with one or more substituents selected from the group consisting of a cyano group and a halogen atom, a C3-C7 cycloalkyl group which is substituted with one or more substituents selected from Group G, or a (C3-C7 cycloalkyl) C1-C3 alkyl group which is substituted with one or more substituents selected from Group G.
[Aspect 113] The compound according to any one of Aspects 1 to 110 or the present compound N wherein T is a group represented by formula T-8, $Y^2$ is a nitrogen atom or $CR^{26}$, $Y^3$ is $CR^{27}$, $Y^4$ is a nitrogen atom or $CR^{28}$, and $R^{1y}$ is a C1-C5 chain hydrocarbon group which is substituted with one or more halogen atoms, a cyano group, or a halogen atom.
[Aspect 114] The compound according to any one of Aspects 1 to 110 or the present compound N wherein T is $OR^1$, and $R^1$ is a C1-C10 chain hydrocarbon group which is substituted with one or more substituents selected from the group consisting of a cyano group and a halogen atom.

Next, a method for producing the present compound will be described.

Production Method 1

The compound represented by formula (I) (present compound N) can be produced by reacting a compound represented by formula (M-1) (hereinafter referred to as compound (M-1)) with a compound represented by formula (M-2) (hereinafter referred to as compound (M-2)):

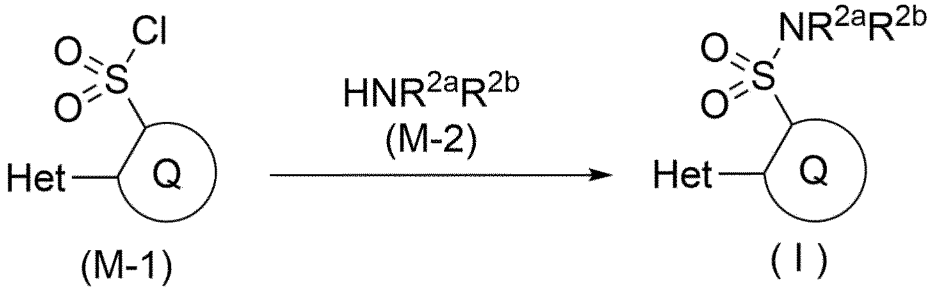


[wherein symbols are the same as defined above.]
The reaction is usually carried out in a solvent. Examples of the solvent include ethers such as tetrahydrofuran

(hereinafter referred to as THF), 1,4-dioxane, 1,2-dimethoxyethane (hereinafter referred to as DME), methyl tert-butyl ether (hereinafter referred to as MTBE) and diethyl ether (hereinafter referred to as ethers); halogenated hydrocarbons such as dichloromethane and chloroform (hereinafter referred to as halogenated hydrocarbons); aromatic hydrocarbons such as toluene and xylene (hereinafter referred to as aromatic hydrocarbons); nitriles such as acetonitrile (hereinafter referred to as nitriles); water, and mixtures of two or more thereof.

In the reaction, the compound (M-2) is usually used in the ratio of 0.8 to 10 mols relative to 1 mol of the compound (M-1).

The reaction can be carried out by mixing the compound (M-1) with the compound (M-2). The reaction may be carried out in the presence of a base as necessary. Examples of the base include alkali metal carbonates such as sodium carbonate and potassium carbonate (hereinafter referred to as alkali metal carbonates); tertiary amines such as triethylamine (hereinafter referred to as tertiary amines); and nitrogen-containing aromatic compounds such as pyridine (hereinafter referred to as nitrogen-containing aromatic compounds). When the base is used in the reaction, the base is usually used in the ratio of 1 to 3 mols relative to 1 mol of the compound (M-1).

The reaction temperature is usually within a range of - 20 to 200°C. The reaction time is usually within a range of 0.1 to 24 hours.

After completion of the reaction, a compound N of the present invention can be obtained by carrying out workup operations such as pouring water into the reaction mixture, extraction with an organic solvent, and drying and concentration of the organic layer.

The compound (M-2) is a commercially available compound or can be produced using a known method.

Production Method 2

The compound represented by formula (I-b) (present compound N) can be produced by reacting a compound represented by formula (M-6) (hereinafter referred to as compound (M-6)) with a compound (M-5) in the presence of a metal catalyst:

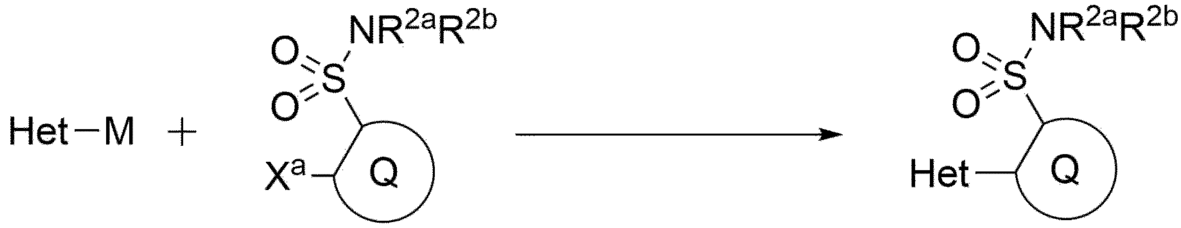

(M-6) (M-5) (I-b)

[wherein M represents a 9-borabicyclo[3.3.1]nonan-9-yl group, a borono group, a 4,4,5,5-tetramethyl-1,3,2-dioxaborolan-2-yl group, a tributylstannyl group or ZnCl; $X^a$ represents a chlorine atom, a bromine atom or an iodine atom; and the other symbols are as the same as defined above.]

The reaction is usually carried out in a solvent. Solvents include, for example, ethers; aromatic hydrocarbons; aprotic polar solvents; water, and mixtures of two or more thereof.

Examples of the metal catalyst used in the reaction include palladium catalysts such as tetrakis(triphenylphosphine)palladium(0), 1,1'-bis(diphenylphosphino)ferrocenepalladium(II) dichloride, tris(dibenzylideneacetone)dipalladium(0), and palladium(II) acetate; nickel catalysts such as bis(cyclooctadiene)nickel(0) and nickel(II) chloride; iron catalysts such as iron(III) chloride and iron(III) acetylacetonate; and copper catalysts such as copper(I) iodide and copper(I) chloride.

The reaction may be carried out as necessary by adding a ligand, a base, and/or an inorganic halide.

Examples of the ligand used in the reaction include triphenylphosphine, xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline.

Examples of the base used in the reaction include alkali metal hydrides, alkali metal carbonates, and organic bases.

Examples of the inorganic halide used in the reaction include alkali metal fluorides such as potassium fluoride and sodium fluoride; and alkali metal chlorides such as lithium chloride and sodium chloride.

In the reaction, the compound (M-5) is usually used in the ratio of 1 to 10 mols, the metal catalyst is usually used in the ratio of 0.01 to 0.5 mol, the ligand is usually used in the ratio of 0.01 to 1 mol, the base is usually used in the ratio of 0.1 to 5 mols, and the inorganic halide is usually used in the ratio of 0.1 to 5 mols, relative to 1 mol of the compound (M-6).

The reaction temperature is usually within a range of - 20°C to 200°C. The reaction time is usually within a range of 0.1 to 24 hours.

After completion of the reaction, a compound represented by formula (I-b) can be obtained by carrying out workup operations such as addition of water to the reaction mixture, extraction with an organic solvent, and drying and concentration of the organic layer.

The compound (M-6) is known or can be produced in accordance with the method mentioned in, for example, WO 2018/221720 A.

Reference Production Method 1

The compound (M-1) can be produced by reacting a compound represented by formula (M-7) (hereinafter referred to as compound (M-7)) with a chlorinating agent in the presence of chlorosulfonic acid:

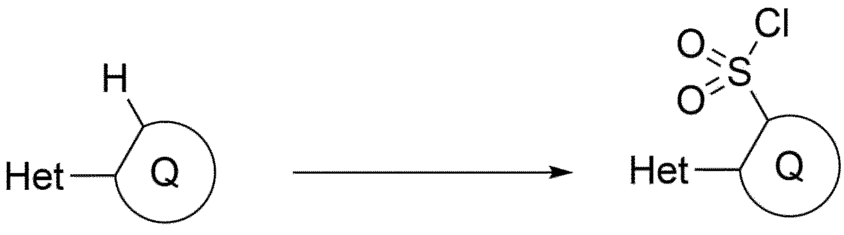

(M-7) (M-1)

[wherein symbols are the same as defined above.]

The reaction may be carried out in a solvent as necessary. Examples of the solvent used in the reaction include ethers; aromatic hydrocarbons; aromatic hydrocarbons, and mixtures of two or more thereof.

Examples of the chlorinating agent used in the reaction include phosphorus oxychloride and thionyl chloride.

In the reaction, the chlorosulfonic acid is usually used in the ratio of 1 to 10 mols and the chlorinating agent is usually used in the ratio of 1 to 10 mols, relative to 1 mol of the compound (M-7).

The reaction temperature is usually within a range of -20°C to 200°C. The reaction time is usually within a range of 0.1 to 24 hours.

After completion of the reaction, a compound represented by formula (M-1) can be obtained by carrying out workup operations such as addition of water to the reaction mixture, extraction with an organic solvent, and drying and concentration of the organic layer.

The compound (M-7) is known or can be produced in accordance with the method mentioned in, for example, WO 2015/163478 A, WO 2016/052455 A, WO 2016/121969 A, WO 2017/069105 A, WO 2017/065228 A, WO 2017/077911 A, WO 2016/121970 A, WO 2017/164108 A, WO 2017/150209 A, WO 2017/169894 A, WO 2018/052119 A, WO 2018/101424 A, or WO 2019/189731 A.

Reference Production Method 2

The compound represented by formula (M-9) (hereinafter referred to as compound (M-9)) can be produced by reacting a compound represented by formula (M-8) (hereinafter referred to as compound (M-8)) with a chlorinating agent in the presence of chlorosulfonic acid:

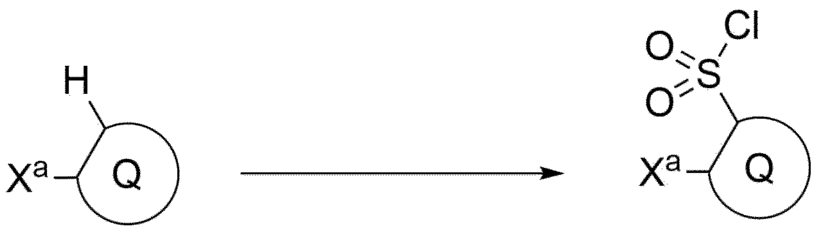

(M-8) (M-9)

[wherein the symbols have the same meanings as defined above.]

The reaction can be carried out in accordance with the method mentioned in Reference Production Method 1, except that a compound (M-8) is used instead of the compound (M-7).

The compound (M-8) is a commercially available compound or can be prepared using a known method.

Reference Production Method 3

The compound (M-5) can be produced by reacting a compound (M-9) with a compound (M-2):

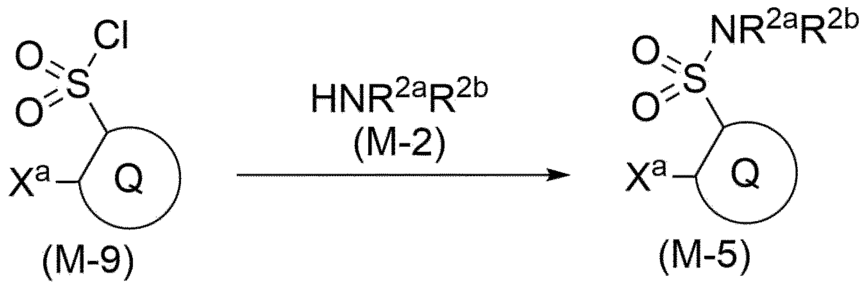


[wherein symbols are the same as defined above.]

The reaction can be carried out in accordance with the method mentioned in Production Method 1, except that a compound (M-9) is used instead of the compound (M-1).

The present compound may be mixed with or used in combination with one or more ingredient(s) selected from the group consisting of the following Group (a), Group (b), Group (c), and Group (d) (hereinafter referred to as "Present ingredient").

When the present compound is mixed with or used in combination with the Present ingredient, they are used simultaneously, separately, or at time intervals with each other.

When the present compound is used simultaneously with the Present ingredient, the present compound and the Present ingredient may be contained in separate formulations or contained in one formulation.

One aspect of the present invention provides a composition comprising one or more ingredient(s) selected from the group consisting of Group (a), Group (b), Group (c), and Group (d), and the present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides, and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the present compound are described. For example, "alanycarb + SX" indicates a combination of alanycarb and SX.

The abbreviation of "SX" indicates any one of the present compound selected from the Compound groups SX1 to SX928 described in Examples. Also, all of the following Present ingredient are known ingredients, and may be obtained from commercially available formulations, or may be prepared by known methods. When the Present ingredient is a microorganism, it may also be available from a bacterial authority depository. Further, the number in parentheses

represents the CAS RN (registered trademark).

Combinations of the Present ingredient in the above Group (a) and the present compound:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilonmetofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, fluralaner + SX, fluvalinate + SX, fluxametamide + SX, formetanate + SX, fosthiazate + SX, furamethrin + SX, furathiocarb + SX, gammacyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, thetacypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX,

triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis MNPV(multiple nucleocapsid nucleopolyhedrovirus) + SX, Autographa californica MNPV + SX, Cydia pomonella GV(granulovirus) strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the present compound:

acibenzolar-S-methyl + SX, aldimorph + SX, ametoctradin + SX, aminopyrifen + SX, amisulbrom + SX, anilazine + SX,

azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, Agrobacterium radiobactor strain K1026 + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl) (2,6-difluoropyridin-4-yl)amino]-N-(2,2-dimethylcyclobutyl)-5-methylthiazole-4-carboxamide + SX, 2-[(2-methylpropanoyl) (2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl] pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-1[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl) methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, Agrobacterium radiobactor strain K84 + SX, Bacillus amyloliquefaciens strain PTA-4838 (Aveo(registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 +

SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadionecalcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the present compound:
anthraquinone + SX, deet + SX, icaridin + SX.

The ratio of the present compound to the Present ingredient includes, but not limited thereto, as a ratio by weight (the present compound : the Present ingredient) 1,000:1 to 1:1,000, 500:1 to 1:500, 100:1 to 1:100, 50:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, and the others.

The present compound has an efficacy against pests. Examples of the pests include plant phytopathogenic microorganism, harmful arthropods such as harmful insects and harmful mites, harmful nematicides, and harmful mollusks.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), and Tagosodes orizicolus;

from the family Cicadellidae, for example, green rice leafhopper (Nephotettix cincticeps), green paddy leafhopper (Nephotettix virescens), rice leafhopper (Nephotettix nigropictus), zigzag-striped leafhopper (Recilia dorsalis),

tea green leafhopper (Empoasca onukii), potato leafhopper (Empoasca fabae), corn leafhopper (Dalbulus maidis), rice leafhopper (Cofana spectra), and Amrasca biguttula biguttula;

from the family Aphrophoridae, for example, European spittlebug (Philaenus spumarius);

from the family Cercopidae, for example, Mahanarva posticata and Mahanarva fimbriolata;

from the family Aphididae, for example, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid(Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);

from the family Phylloxeridae, for example, grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);

from the family Adelgidae, for example, hemlock woolly aphid (Adelges tsugae), balsam woolly aphid (Adelges piceae), and Aphrastasia pectinatae;

from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug(Euschistus heros), red banded stink bug(Piezodorus guildinii), Oebalus pugnax, and Dichelops melacanthus;

from the family Cydnidae, for example, Scaptocoris castanea;

from the family Alydidae, for example, bean bug (Riptortus clavatus), corbett rice bug (Leptocorisa chinensis), and rice bug (Leptocorisa acuta);

from the family Coreidae, for example, Cletus punctiger and Australian leaf-footed bug (Leptoglossus australis);

from the family Lygaeidae, for example, oriental chinch bug (Cavelerius saccharivorus), seed bug (Togo hemipterus), and chinch bug (Blissus leucopterus);

from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);

from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;

from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);

from the family Coccidae, for example, pink wax scale (Ceroplastes rubens);

from the family Margarodidae, for example, fluted scale (Icerya purchasi) and seychelles fluted scale (Icerya seychellarum);

from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);

from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);

from the family Tingidae, for example, sycamore lace bug (Corythucha ciliata), aster tingid (Corythucha marmorata), Japanese pear lace bug (Stephanitis nashi), and azalea lace bug (Stephanitis pyrioides);

from the family Cimicidae, for example, common bed bug (Cimex lectularius) and tropical bed bug (Cimex hemipterus); from the family Cicadidae, for example, Quesada gigas;

from the family Reduviidae, for example, Triatoma infestans, large kissing bug (Triatoma rubrofasciata), Triatoma dimidiata, and Rhodonius prolixus;

and the others.

Lepidoptera:

from the family Crambidae, for example, rice stem borer (Chilo suppressalis), Dark-headed stem borer (Chilo

polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);

from the family Pyralidae, for example, lesser cornstalk borer (Elasmopalpus lignosellus), mealworm moth (Plodia interpunctella), persimmon bark borer (Euzophera batangensis), and fig moth (Cadra cautella);

from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), semitropical armyworm (Spodoptera eridania), black cutworm (Agrotis ipsilon), beet worm (Autographa nigrisigna), rice looper (Plusia festucae), Soybean looper (Chrysodeixis includens), Trichoplusia spp., Heliothis spp.(such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);

from the family Pieridae, for example, common cabbage worm (Pieris rapae);

from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);

from the family Gracillariidae, for example, tea leaf roller (Caloptilia theivora) and Asiatic apple leaf miner (Phyllonorycter ringoniella);

from the family Carposinidae, for example, peach fruit moth (Carposina sasakii);

from the family Lyonetiidae, for example, coffee leaf miner (Leucoptera coffeella), peach leaf miner (Lyonetia clerkella), and Lyonetia prunifoliella;

from the family Lymantriidae, for example, Lymantria spp. (such as gypsy moth (Lymantria dispar)) and Euproctis spp. (such as tea lymantriid (Euproctis pseudoconspersa));

from the family Plutellidae, for example, diamondback moth (Plutella xylostella);

from the family Gelechiidae, for example, peach worm (Anarsia lineatella), sweetpotato leaf folder (Helcystogramma triannulella), pink bollworm (Pectinophora gossypiella), potato moth (Phthorimaea operculella), and Tuta absoluta;

from the family Arctiidae, for example, American white moth (Hyphantria cunea);

from the family Castniidae, for example, giant sugarcane borer (Telchin licus);

from the family Cossidae, for example, Cossus insularis;

from the family Geometridae, for example, Ascotis selenaria;

from the family Limacodidae, for example, blue-striped nettle grub (Parasa lepida) ;

from the family Stathmopodidae, for example, persimmon fruit moth (Stathmopoda masinissa);

from the family Sphingidae, for example, tobacco hornworm (Acherontia lachesis);

from the family Sesiidae, for example, Nokona feralis, cherry borer (Synanthedon hector), and Synanthedon tenuis:

from the family Hesperiidae, for example, rice skipper (Parnara guttata);

from the family Tineidae, for example, casemaking clothes moth (Tinea translucens), common clothes moth (Tineola bisselliella);

and the others.

Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae);

from the family Phlaeothripidae, for example, aculeated rice thrips (Haplothrips aculeatus);

and the others.

Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis;
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus ,Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus;
from the family Muscidae, for example, house fly (Musca domestica), false stable fly (Muscina stabulans), biting house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;
and the others.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle(Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, and tobacco flea beetle (Epitrix hirtipennis);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala

rufocuprea), Anomala albopilosa, Japanese beetle (Popillia japonica), yellowish elongate chafer (Heptophylla picea), European Chafer(Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus hampei) ;
from the family Curculionidae, for example, West Indian sweet-potato weevil (Euscepes postfasciatus), alfalfa weevil (Hypera postica), maize weevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powderpost beetle (Lyctus brunneus), and lesser grain borer (Rhizopertha dominica);
from the family Ptinidae;
from the family Cerambycidae, for example, citrus longhorned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum);
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);
and the others.

Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust(Locustana pardalina), tree locust (Anacridium melanorhodon), Italian locust (Calliptamus italicus), differential grasshopper (Melanoplus differentialis), twostriped grasshopper (Melanoplus bivittatus), migratory grasshopper (Melanoplus sanguinipes), red-legged grasshopper (Melanoplus femurrubrum), clearwinged grasshopper (Camnula pellucida), desert locust (Schistocerca gregaria), Yellowwinged locust (Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma);
from the family Tettigoniidae, for example, mormon cricket (Anabrus simplex);
and the others.

Hymenoptera :

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta), tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine

ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia, Vespa simillima, Vespa analis, Asian hornet(Vespa velutina), and Polistes jokahamae;
from the family Siricidae, for example, pine wood wasp (Urocerus gigas);
from the family Bethylidae;
and the others.

Blattodea :

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;
and the others.

Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp;
from the family Trimenoponidae, for example, Cummingsia spp.;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;
and the other.

Thysanura:

from the family Lepismatidae, for example, oriental silverfish (Ctenolepisma villosa) andmoth fish (Lepisma saccharina);
and the others.

Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example,bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;
and the others.

Araneae:

from the family Eutichuridae, for example, Cheiracanthium japonicum;
from the family Theridiidae, for example, red-back spider (Latrodectus hasseltii);
and the others

Polydesmida:

from the family Paradoxosomatidae, for example, flat-backed millipede (Oxidus gracilis) and Nedyopus tambanus;
and the others.

Isopoda:

from the family Armadillidiidae, common pill bug (Armadillidium vulgare);
and the others.

Chilopoda:

from the family Scutigeridae, for example, Thereuonema hilgendorfi;

from the family Scolopendridae, giant tropical centipede (Scolopendra subspinipes);
from the family Ethopolyidae, Bothropolys rugosus;
and the others.

Gastropoda:

from the family Limacidae, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;
and the others.

Nematoda:

from the family Aphelenchoididae, for example, rice whitetip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);
and the others.

The harmful arthropods such as harmful insects and harmful mites, harmful mollusks and harmful nematodes may be those having a reduced susceptibility to or a developed resistance to an insecticide, a mitecide, a molluscicide or a nematicide.

The method for controlling harmful arthropods of the present invention is carried out by applying an effective amount of the present compound or the Composition A to a harmful arthropod directly and/or a habitat thereof (for example, plant bodies, soil, an interior of a house, animal bodies). Examples of the method for controlling harmful arthropods of the present invention include foliage treatment, soil treatment, root treatment, shower treatment, smoking treatment, water surface treatment and seed treatment.

The present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example,

acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the present compound. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), N092P-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the present compound or the Composition A to soils include a method of applying an effective amount of the present compound or the Composition A to soils before planting plants or after planting plants, a method of applying an effective amount of the present compound or the Composition A to a root part of a crop to be protected from damage such as ingestion by harmful arthropods, and a method of controlling harmful arthropods that ingest a plant by permeating and transferring an effective amount of the present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the present compound or the Composition A to seeds or vegetative reproductive organs, and specific examples thereof include spraying treatment in

which a suspension of the present compound or the Composition A is sprayed onto seed surface or the vegetative reproductive organ surface in the form of mist; smearing treatment in which the present compound or the Composition A is coated a surface of seeds or the vegetative reproductive organ; a soaking treatment in which the seeds are soaked into the solution of the present compound or the Composition A for a certain time; and a method for coating the seeds or the vegetative reproductive organ with a carrier containing the present compound or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present ingredients other than the alreadyapplied Present ingredients, are included.

As used herein, seeds or vegetative reproductive organs carrying the present compound or the Composition A means seeds or vegetative reproductive organs in the state where the present compound or the Composition A is adhered to a surface of the seeds or the vegetative reproductive organ. The above-described seeds or vegetative reproductive organs carrying the present compound or the Composition A may be adhered by any other materials that are different from the present compound or the Composition A before or after being adhered the present compound or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound or the Composition A can be obtained, for example, by applying the formulations comprising the present compound or the Composition A by the above-described application method to seeds to seeds or vegetative reproductive organs.

When the present compound or the Composition A is applied for harmful arthropods control in agricultural fields, the application dose thereof is usually within a range of 1 to 10,000 g of the present compound per 10,000 $m^2$. In the case of being applied to seeds or vegetative reproductive organs, the dose of application dose thereof is usually within a range of 0.001 to 100 g of the present compound per 1 Kg of seeds. When the present compound or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the present compound or the Composition A is used to control harmful arthropods that live inside a house, the application dose as an amount of the present compound is usually within a range from 0.01 to 1,000 mg per 1 $m^2$ of an area to be treated, in the case of using it on a planar area. In the case of using it spatially, the application dose as an amount of the present compound is usually within a range from 0.01 to 500 mg per 1 $m^3$ of the space to be treated. When the present compound or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the present compound or the composition is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the compound of the present compound is usually within a range from 0.1 to 1,000 mg per 1 kg of an animal body weight.

Also, the composition of the present compound or the Composition A may be used as an agent for controlling

harmful arthropods in agricultural lands such as paddy fields, fields, turfs, and orchards. Examples of the plants include the followings:
corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

[Examples]

Hereinafter, the present invention will be described in more detail by way of Preparation Examples, Formulation Examples and Test Examples, but the present invention is not limited only to these Examples.

As used herein, Me represents a methyl group, Et represents an ethyl group, Pr represents a propyl group, i-Pr represents an isopropyl group, t-Bu represents a tert-butyl group, c-Pr represents a cyclopropyl group, Ph represents a phenyl group, Py2 represents a 2-pyridyl group, Py3 represents a 3-pyridyl group, and Py4 represents a 4-pyridyl group. When c-Pr, Ph, Py2, Py3 and Py4 are substituted with a substituent, the substituent is described together with a substitution position before the symbol. For example, 1-CN-c-Pr represents a 1-cyanocyclopropyl group, 3,4-$F_2$-Ph represents a 3,4-difluorophenyl group, 4-$S(O)_2CF_3$-Ph represents a 4-(trifluoromethanesulfonyl)phenyl group, 3-$SCF_3$-Ph represents a 3-[(trifluoromethyl)thio]phenyl group, 4-$CF_3$-Py2 represents a 4-(trifluoromethyl)-2-pyridyl group, and 5-$NMeCH_2CF_3$-Py2 represents a 5-(methyl 2,2,2-trifluoroethylamino)-2-pyridyl group.

First, Production Examples of the present compound and intermediates thereof are shown.

Reference Production Example 1

To a mixture of 2.2 g of 2-(2-bromoacetyl)-5-(2,2,3,3,3-pentafluoropropoxy)pyridine produced by the method mentioned in WO 2018/052119 A and 5 mL of acetonitrile, 0.9 g of 5-(trifluoromethyl)-2-aminopyridine was added at room temperature, followed by stirring under reflux for 3 hours. The mixture thus obtained was cooled to room temperature and water was added, followed by extraction with ethyl acetate. The organic layer thus obtained was dried over sodium sulfate and concentrated under reduced pressure. The residue thus obtained was subjected to silica gel column chromatography to obtain 0.43 g of an intermediate 1 represented by the following formula.

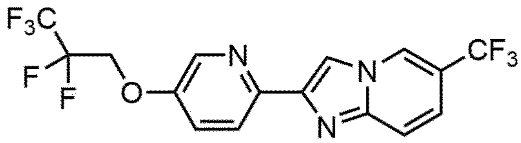

Intermediate 1: $^1$H-NMR ($CDCl_3$) δ: 8.52 (1H, t), 8.40 (1H, d), 8.27 (1H, d), 8.18 (1H, d), 7.73 (1H, dd), 7.38 (1H, dd), 7.37-7.33 (1H, dd), 4.53 (2H, td).

Reference Production Example 2

The compounds produced in accordance with Reference Production Example 1 and physical properties thereof are shown below.

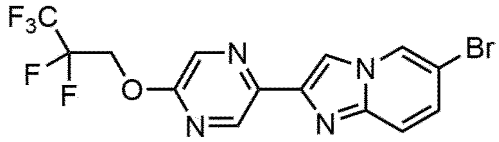

Intermediate 2: $^1$H-NMR ($CDCl_3$) δ: 8.93-8.92 (1H, m), 8.36 (1H, s), 8.33 (1H, s), 8.13 (1H, s), 7.56 (1H, d), 7.30 (1H, d), 4.96-4.88 (2H, m).

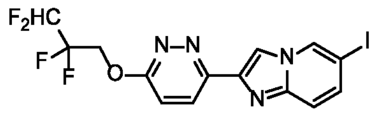

Intermediate 3: $^1$H-NMR ($CDCl_3$) δ: 8.51-8.49 (1H, m), 8.36 (1H, d), 8.33 (1H, s), 7.47-7.40 (2H, m), 7.21 (1H, d), 6.21-5.92 (1H, m), 5.06-4.97 (2H, m).

Production Example 1

A mixture of 1.0 g of the intermediate 2, 1.0 mL of chlorosulfonic acid and 6 mL of phosphorus oxychloride was stirred at 120°C for 10 hours. The mixture thus obtained was concentrated under reduced pressure, and 1 mL of triethylamine, 6 mL of acetonitrile and 6 mL of methylamine (7% tetrahydrofuran solution) were added to the residue thus obtained, followed by stirring at room temperature for 1 hour. To the mixture thus obtained, water was added, followed by extraction with ethyl acetate. The organic layer thus obtained was dried over anhydrous sodium sulfate and concentrated under reduced pressure. The residue thus obtained was subjected to silica gel column chromatography to obtain 0.40 g of the present compound 1 represented by the following formula.

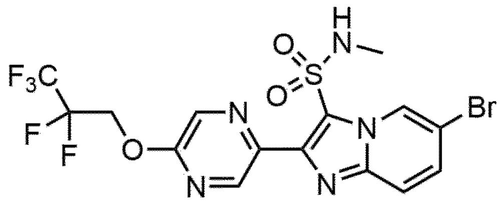

Present compound 1: $^1$H-NMR ($CDCl_3$) δ: 9.42 (1H, s), 9.07 (1H, s), 8.38 (1H, s), 7.63 (1H, d), 7.53 (1H, d), 7.19-7.14 (1H, m), 4.94 (2H, t), 2.72 (3H, d).

Production Example 2

The compounds produced in accordance with Reference Production Example 1 and physical properties thereof are shown below.

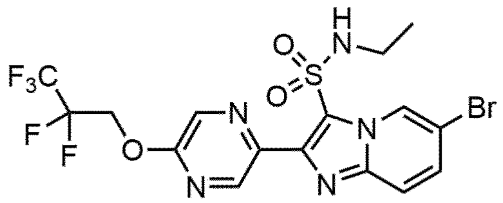

Present compound 2: $^1$H-NMR ($CDCl_3$) δ: 9.41 (1H, s), 9.07 (1H, s), 8.39 (1H, s), 7.63 (1H, d), 7.52 (1H, d), 7.20-7.15 (1H, m), 4.93 (2H, t), 3.12-3.04 (2H, m), 1.13 (3H, t).

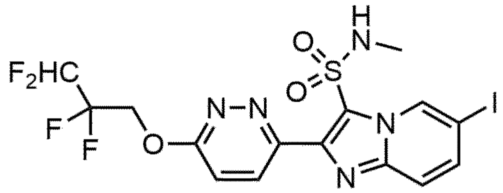

Present compound 3: $^1$H-NMR ($CDCl_3$) δ: 9.57 (1H, s), 8.41 (1H, d), 7.64 (1H, d), 7.61-7.56 (1H, m), 7.51 (1H, d), 7.28 (1H, d), 6.04 (1H, t), 5.01 (2H, t), 2.75 (3H, d).

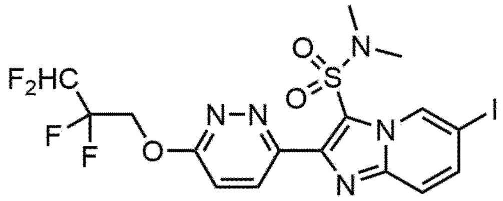

Present compound 4: $^1$H-NMR ($CDCl_3$) δ: 9.22 (1H, s), 7.93 (1H, d), 7.68 (1H, d), 7.56 (1H, d), 7.19 (1H, d), 6.06 (1H, t), 5.02 (2H, t), 2.89 (6H, d).

Next, examples of the present compounds produced in accordance with any of Production Examples mentioned in Examples and Production Methods mentioned herein are shown below.

A compound represented by formula (L-1):

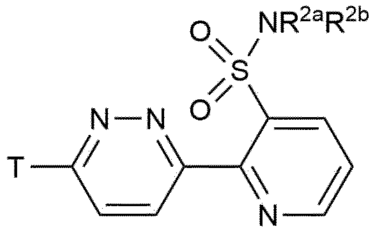

( L-1 )

(hereinafter referred to as compound (L-1)) wherein $R^{2a}$ and $R^{2b}$ are a hydrogen atom, and T is any one substituent mentioned in [Table 1A] to [Table 6A] (hereinafter referred to as compound group SX1).

**Table 1A**

| |
|---|
| $CF_3$ |
| $CHF_2$ |
| $CH_2CF_3$ |
| $CF_2CF_3$ |
| $CH_2CF_2CF_3$ |
| $CF_2CF_2CF_3$ |
| $CF_2CF_2CF_2CF_3$ |
| $CF_2CF_2CF_2CF_2CF_3$ |

(continued)

| |
|---|
| $OCF_3$ |
| $OCHF_2$ |
| $OCH_2CF_3$ |
| $OCH_2CHF_2$ |
| $OCF_2CF_3$ |
| $OCH(CH_3)CF_3$ |
| $OCH_2CF_2CHF_2$ |
| $OCH_2CF_2CF_3$ |
| $OCF_2CF_2CF_3$ |
| $OCH_2CF_2CHFCF_3$ |
| $OCH_2CF_2CF_2CF_3$ |
| $OCF_2CF_2CF_2CF_3$ |
| $OCH_2CF_2CF_2CF_2CF_3$ |
| $OCH_2CMe_2CN$ |
| $OCH_2$-1-CN-c-Pr |
| OH |

**Table 2A**

| |
|---|
| $SCF_3$ |
| $SCH_2CF_3$ |
| $SCF_2CF_3$ |
| $SCH_2CF_2CF_3$ |
| $SCF_2CF_2CF_3$ |
| $SCH_2CF_2CF_2CF_3$ |
| $SCF_2CF_2CF_2CF_3$ |
| $S(O)CF_3$ |
| $S(O)CH_2CF_3$ |
| $S(O)CF_2CF_3$ |
| $S(O)CH_2CF_2CF_3$ |
| $S(O)CF_2CF_2CF_3$ |
| $S(O)CH_2CF_2CF_2CF_3$ |
| $S(O)CF_2CF_2CF_2CF_3$ |
| $S(O)_2CF_3$ |
| $S(O)_2CH_2CF_3$ |
| $S(O)_2CF_2CF_3$ |
| $S(O)_2CH_2CF_2CF_3$ |
| $S(O)_2CF_2CF_2CF_3$ |
| $S(O)_2CH_2CF_2CF_2CF_3$ |
| $S(O)_2CF_2CF_2CF_2CF_3$ |
| $SCH_2CMe_2CN$ |

(continued)

| |
|---|
| $SCH_2$-1-CN-c-Pr |
| Cl |

**Table 3A**

| |
|---|
| $NHCH_2CF_3$ |
| $NHCH_2CF_2CF_3$ |
| $NHCH_2CF_2CF_2CF_3$ |
| $NMeCH_2CF_3$ |
| $NMeCH_2CF_2CF_3$ |
| $NMeCH_2CF_2CF_2CF_3$ |
| $NEtCH_2CF_3$ |
| $NEtCH_2CF_2CF_3$ |
| $NEtCH_2CF_2CF_2CF_3$ |
| $OS(O)_2CF_3$ |
| $OS(O)_2CF_2CF_3$ |
| $OS(O)_2CF_2CF_2CF_3$ |
| $CH_2OCF_3$ |
| $CH_2OCH_2CF_3$ |
| $CH_2OCF_2CF_3$ |
| $C(O)CF_3$ |
| $C(O)CF_2CF_3$ |
| $C(O)CF_2CF_2CF_3$ |
| $C(O)NMeCH_2CF_3$ |
| $NMeC(O)CF_3$ |
| $N=CEtCH_2CF_3$ |
| $CH_2CH_2CMe_2CN$ |
| $CH_2CH_2$-1-CN-c-Pr |
| |

**Table 4A**

| |
|---|
| 3-$CF_3$-Ph |
| 4-$CF_3$-Ph |
| 3, 5- $(CF_3)_2$-Ph |
| 3-$SCF_3$-Ph |
| 3-$S(O)CF_3$-Ph |
| 3-S $(O)_2CF_3$-Ph |
| 4-$SCF_3$-Ph |
| 4-$S(O)CF_3$-Ph |
| 4-$S(O)_2CF_3$-Ph |

(continued)

| |
|---|
| 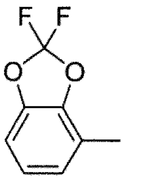 |
| 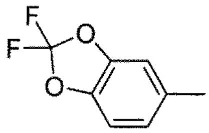 |
| 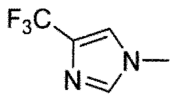 |

**Table 5A**

| |
|---|
| 4-$CF_3$-Py2 |
| 5-$CF_3$-Py2 |
| 4-$SCF_3$-Py2 |
| 4-$S(O)CF_3$-Py2 |
| 4-$S(O)_2CF_3$-Py2 |
| 5-$SCF_3$-Py2 |
| 5-$S(O)CF_3$-Py2 |
| 5-$S(O)_2CF_3$-Py2 |
| 5-$NMeCH_2CF_3$-Py2 |
| 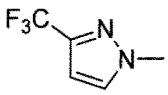 |
| 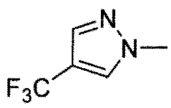 |
| 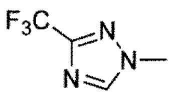 |

**Table 6A**

| |
|---|
| 5-$CF_3$-Py3 |
| 6-$CF_3$-Py3 |
| 5-$SCF_3$-Py3 |
| 5-$S(O)CF_3$-Py3 |
| 5-$S(O)_2CF_3$-Py3 |
| 6-$SCF_3$-Py3 |
| 6-$S(O)CF_3$-Py3 |
| 6-$S(O)_2CF_3$-Py3 |

(continued)

| $6\text{-NMeCH}_2\text{CF}_3\text{-Py3}$ |
|---|
| 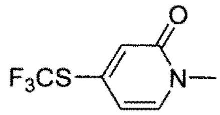 |
| 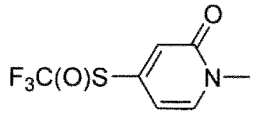 |
| 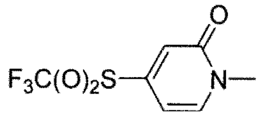 |

The compound (L-1) wherein $R^{2a}$ is a methyl group, $R^{2b}$ is a hydrogen atom, and T is any one substituent mentioned in [Table 1A] to [Table 6A] (hereinafter referred to as compound group SX2).
The compound (L-1) wherein $R^{2a}$ is a methyl group, $R^{2b}$ is a methyl group, and T is any one substituent mentioned in [Table 1A] to [Table 6A] (hereinafter referred to as compound group SX3).
The compound (L-1) wherein $R^{2a}$ is an ethyl group, $R^{2b}$ is a hydrogen atom, and T is any one substituent mentioned in [Table 1A] to [Table 6A] (hereinafter referred to as compound group SX4).
The compound (L-1) wherein $R^{2a}$ is an ethyl group, $R^{2b}$ is an ethyl group, and T is any one substituent mentioned in [Table 1A] to [Table 6A] (hereinafter referred to as compound group SX5).
The compound (L-1) wherein $R^{2a}$ is a propyl group, $R^{2b}$ is a hydrogen atom, and T is any one substituent mentioned in [Table 1A] to [Table 6A] (hereinafter referred to as compound group SX6).
The compound (L-1) wherein $R^{2a}$ is a propyl group, $R^{2b}$ is a propyl group, and T is any one substituent mentioned in [Table 1A] to [Table 6A] (hereinafter referred to as compound group SX7).
The compound (L-1) wherein $R^{2a}$ is a methyl group, $R^{2b}$ is an ethyl group, and T is any one substituent mentioned in [Table 1A] to [Table 6A] (hereinafter referred to as compound group SX8).
A compound represented by formula (L-2):

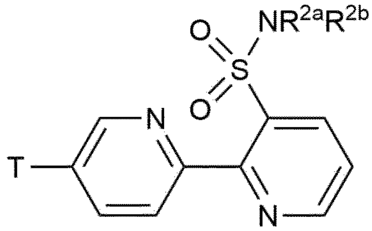

( L-2 )

(hereinafter referred to as compound (L-2)) wherein $R^{2a}$ and $R^{2b}$ are a hydrogen atom, and T is any one substituent mentioned in [Table 1A] to [Table 6A] (hereinafter referred to as compound group SX9).
The compound (L-2) wherein $R^{2a}$ is a methyl group, $R^{2b}$ is a hydrogen atom, and T is any one substituent mentioned in [Table 1A] to [Table 6A] (hereinafter referred to as compound group SX10).
The compound (L-2) wherein $R^{2a}$ is a methyl group, $R^{2b}$ is a methyl group, and T is any one substituent mentioned in [Table 1A] to [Table 6A] (hereinafter referred to as compound group SX11).
The compound (L-2) wherein $R^{2a}$ is an ethyl group, $R^{2b}$ is a hydrogen atom, and T is any one substituent mentioned in [Table 1A] to [Table 6A] (hereinafter referred to as compound group SX12).
The compound (L-2) wherein $R^{2a}$ is an ethyl group, $R^{2b}$ is an ethyl group, and T is any one substituent mentioned in [Table 1A] to [Table 6A] (hereinafter referred to as compound group SX13).
The compound (L-2) wherein $R^{2a}$ is a propyl group, $R^{2b}$ is a hydrogen atom, T is any one substituent mentioned in [Table 1A] to [Table 6A] (hereinafter referred to as compound group SX14).
The compound (L-2) wherein $R^{2a}$ is a propyl group, $R^{2b}$ is a propyl group, and T is any one substituent mentioned in [Table 1A] to [Table 6A] (hereinafter referred to as compound group SX15).
The compound (L-2) wherein $R^{2a}$ is a methyl group, $R^{2b}$ is an ethyl group, and T is any one substituent mentioned

in [Table 1A] to [Table 6A] (hereinafter referred to as compound group SX16).

A compound represented by formula (L-3):

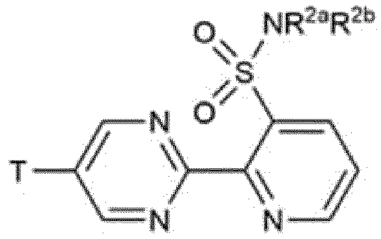

( L-3 )

(hereinafter referred to as compound (L-3)) wherein $R^{2a}$ and $R^{2b}$ are a hydrogen atom, and T is any one substituent mentioned in [Table 1A] to [Table 6A] (hereinafter referred to as compound group SX17).

The compound (L-3) wherein $R^{2a}$ is a methyl group, $R^{2b}$ is a hydrogen atom, and T is any one substituent mentioned in [Table 1A] to [Table 6A] (hereinafter referred to as compound group SX18).

The compound (L-3) wherein $R^{2a}$ is a methyl group, $R^{2b}$ is a methyl group, and T is any one substituent mentioned in [Table 1A] to [Table 6A] (hereinafter referred to as compound group SX19).

The compound (L-3) wherein $R^{2a}$ is an ethyl group, $R^{2b}$ is a hydrogen atom, and T is any one substituent mentioned in [Table 1A] to [Table 6A] (hereinafter referred to as compound group SX20).

The compound (L-3) wherein $R^{2a}$ is an ethyl group, $R^{2b}$ is an ethyl group, and T is any one substituent mentioned in [Table 1A] to [Table 6A] (hereinafter referred to as compound group SX21).

The compound (L-3) wherein $R^{2a}$ is a propyl group, $R^{2b}$ is a hydrogen atom, and T is any one substituent mentioned in [Table 1A] to [Table 6A] (hereinafter referred to as compound group SX22).

The compound (L-3) wherein $R^{2a}$ is a propyl group, $R^{2b}$ is a propyl group, and T is any one substituent mentioned in [Table 1A] to [Table 6A] (hereinafter referred to as compound group SX23).

The compound (L-3) wherein $R^{2a}$ is a methyl group, $R^{2b}$ is an ethyl group, and T is any one substituent mentioned in [Table 1A] to [Table 6A] (hereinafter referred to as compound group SX24).

A compound represented by formula (L-4):

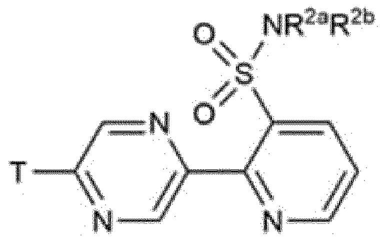

( L-4 )

(hereinafter referred to as compound (L-4)) wherein $R^{2a}$ and $R^{2b}$ are a hydrogen atom, and T is any one substituent mentioned in [Table 1A] to [Table 6A] (hereinafter referred to as compound group SX25).

The compound (L-4) wherein $R^{2a}$ is a methyl group, $R^{2b}$ is a hydrogen atom, and T is any one substituent mentioned in [Table 1A] to [Table 6A] (hereinafter referred to as compound group SX26).

The compound (L-4) wherein $R^{2a}$ is a methyl group, $R^{2b}$ is a methyl group, and T is any one substituent mentioned in [Table 1A] to [Table 6A] (hereinafter referred to as compound group SX27).

The compound (L-4) wherein $R^{2a}$ is an ethyl group, $R^{2b}$ is a hydrogen atom, and T is any one substituent mentioned in [Table 1A] to [Table 6A] (hereinafter referred to as compound group SX28).

The compound (L-4) wherein $R^{2a}$ is an ethyl group, $R^{2b}$ is an ethyl group, and T is any one substituent mentioned in [Table 1A] to [Table 6A] (hereinafter referred to as compound group SX29).

The compound (L-4) wherein $R^{2a}$ is a propyl group, $R^{2b}$ is a hydrogen atom, and T is any one substituent mentioned in [Table 1A] to [Table 6A] (hereinafter referred to as compound group SX30).

The compound (L-4) wherein $R^{2a}$ is a propyl group, $R^{2b}$ is a propyl group, and T is any one substituent mentioned in [Table 1A] to [Table 6A] (hereinafter referred to as compound group SX31).

The compound (L-4) wherein $R^{2a}$ is a methyl group, $R^{2b}$ is an ethyl group, and T is any one substituent mentioned in [Table 1A] to [Table 6A] (hereinafter referred to as compound group SX32).

A compound represented by formula (L-5):

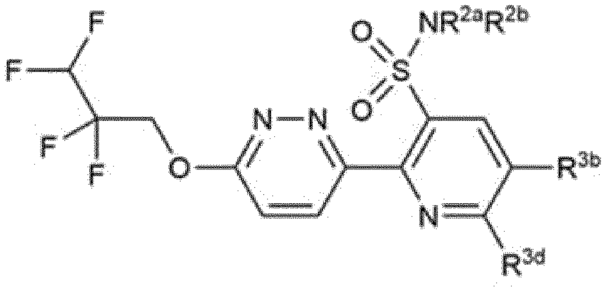

( L-5 )

(hereinafter referred to as compound (L-5)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX33).

**Table 7A**

| |
|---|
| F |
| Cl |
| Br |
| Me |
| Et |
| Pr |
| i-Pr |
| c-Pr |
| 1-CN-c-Pr |
| OMe |
| OEt |
| OPr |
| Oi-Pr |
| $CF_3$ |
| $NH_2$ |
| $NHCH_2CF_3$ |
| CN |
| C(O)OEt |
| NHC(O)c-Pr |
| NMeC(O)c-Pr |
| CH=N-OH |
| CH=N-OMe |

**Table 8A**

| |
|---|
| Ph |
| 3-F-Ph |
| 4-F-Ph |
| 3-Cl-Ph |
| 4-Cl-Ph |
| 3-$CF_3$-Ph |
| 4-$CF_3$-Ph |

(continued)

| |
|---|
| 3-$NMe_2$-Ph |
| 4-$NMe_2$-Ph |
| 3-CN-Ph |
| 4-CN-Ph |
| 4-C(O)$NMe_2$-Ph |
| 4-NHC(O)Me-Ph |
| 3, 4-$F_2$-Ph |
| 3, 5-$F_2$-Ph |
| 2, 4-$F_2$-Ph |
| 3, 4, 5-$F_3$-Ph |
| 3, 4-$Cl_2$-Ph |
| 3, 5-$Cl_2$-Ph |
| 3, 5-$Cl_2$-4-F-Ph |
| OPh |
| O-2-F-Ph |

**Table 9A**

| |
|---|
| Py2 |
| 4-F-Py2 |
| 5-F-Py2 |
| 4-Cl-Py2 |
| 5-Cl-Py2 |
| 4-$CF_3$-Py2 |
| 5-$CF_3$-Py2 |
| 3-Me-Py2 |
| 4-Me--Py2 |
| 5-Me-Py2 |
| 6-Me-Py2 |
| 5-CN-Py2 |
| 5-$OCH_2CF_2CF_3$-Py2 |
| 3, 5-$F_2$-Py2 |
| Py3 |
| 6-$CF_3$-Py3 |
| 5-$CF_3$-Py3 |
| 6-F-Py3 |
| 6-Cl-Py3 |
| Py4 |
| OPy2 |
| OPy3 |

**Table 10A**

| |
|---|
| 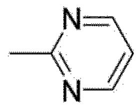 |
| 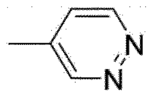 |
| 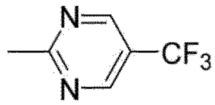 |
| 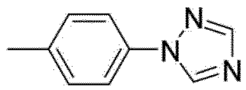 |
| 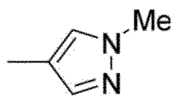 |
| 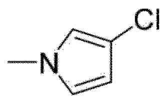 |
| 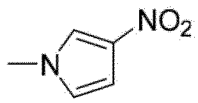 |

**Table 11A**

| |
|---|
| 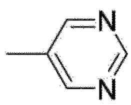 |
| 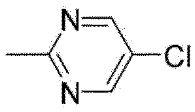 |
| 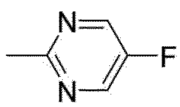 |
| 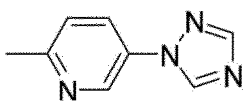 |
| 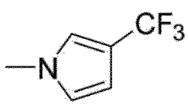 |
| 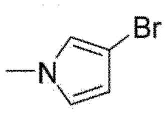 |

(continued)

| |
|---|
| 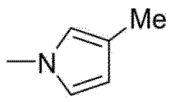 |

**Table 12A**

| |
|---|
| 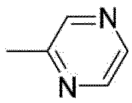 |
| 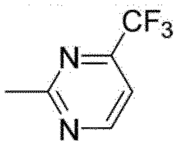 |
| 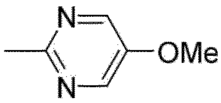 |
| 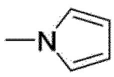 |
| 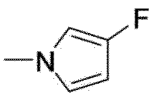 |
| 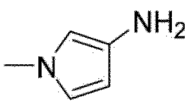 |
| 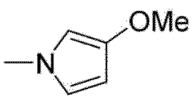 |

**Table 13A**

| |
|---|
| 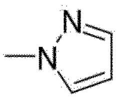 |
| 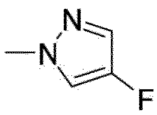 |
| 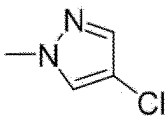 |
| 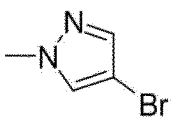 |

(continued)

| |
|---|
| 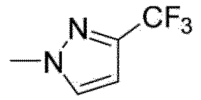 |
| 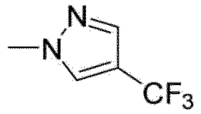 |
| 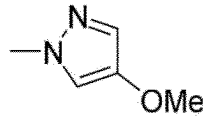 |
| 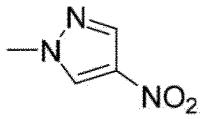 |
| 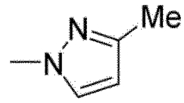 |

**Table 14A**

| |
|---|
| 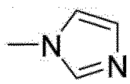 |
| 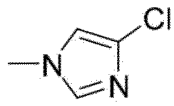 |
| 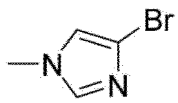 |
| 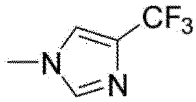 |
| 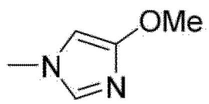 |
| 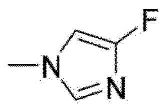 |
| 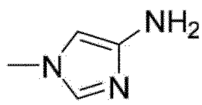 |

(continued)

| |
|---|
| 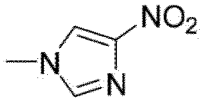 |
| 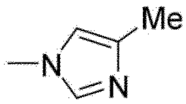 |

**Table 15A**

| |
|---|
| 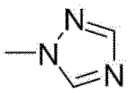 |
| 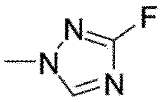 |
| 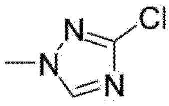 |
| 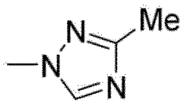 |
| 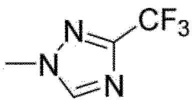 |
| 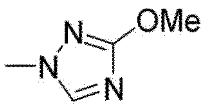 |
| 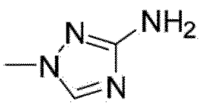 |
| 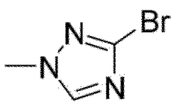 |
| 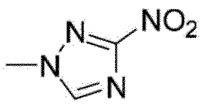 |

The compound (L-5) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX34).

The compound (L-5) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX35).

The compound (L-5) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX36).
The compound (L-5) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX37).
The compound (L-5) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX38).
The compound (L-5) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX39).
The compound (L-5) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX40).
The compound (L-5) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX41).
The compound (L-5) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX42).
The compound (L-5) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX43).
The compound (L-5) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX44).
The compound (L-5) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX45).
The compound (L-5) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX46).
The compound (L-5) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX47).
The compound (L-5) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX48).
A compound represented by formula (L-6):

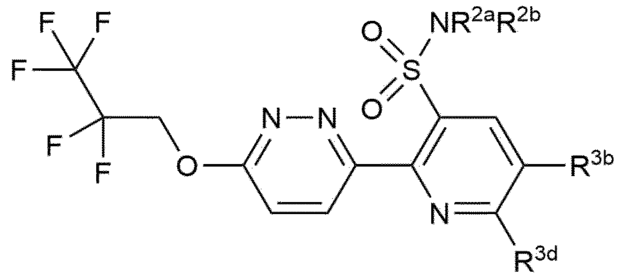

(L-6)

(hereinafter referred to as compound (L-6)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX49).
The compound (L-6) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX50).
The compound (L-6) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX51).
The compound (L-6) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX52).
The compound (L-6) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX53).
The compound (L-6) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX54).
The compound (L-6) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX55).
The compound (L-6) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX56).
The compound (L-6) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX57).
The compound (L-6) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX58).

The compound (L-6) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX59).
The compound (L-6) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX60).
The compound (L-6) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX61).
The compound (L-6) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX62).
The compound (L-6) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX63).
The compound (L-6) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX64).
A compound represented by formula (L-7):

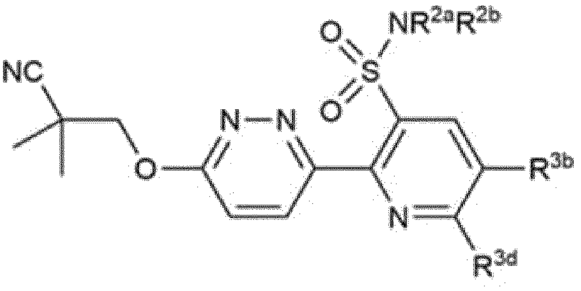

(L-7)

(hereinafter referred to as compound (L-7)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX65).
The compound (L-7) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX66).
The compound (L-7) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX67).
The compound (L-7) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX68).
The compound (L-7) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX69).
The compound (L-7) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX70).
The compound (L-7) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX71).
The compound (L-7) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX72).
The compound (L-7) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX73).
The compound (L-7) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX74).
The compound (L-7) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX75).
The compound (L-7) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX76).
The compound (L-7) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX77).
The compound (L-7) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX78).
The compound (L-7) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX79).
The compound (L-7) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX80).
A compound represented by formula (L-8):

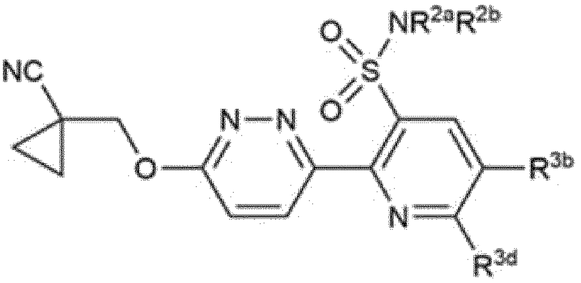

( L-8 )

(hereinafter referred to as compound (L-8)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX81).

The compound (L-8) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX82).

The compound (L-8) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX83).

The compound (L-8) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX84).

The compound (L-8) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX85).

The compound (L-8) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX86).

The compound (L-8) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX87).

The compound (L-8) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX88).

The compound (L-8) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX89).

The compound (L-8) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX90).

The compound (L-8) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX91).

The compound (L-8) wherein $R^{2a}$ is an ethyl group, $R^{2b}$ is a hydrogen atom, $R^{3d}$ is a hydrogen atom, $R^{3b}$ is any one substituent mentioned in [Table 7A] to [Table 15A] (hereinafter referred to as compound group SX92).

The compound (L-8) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX93).

The compound (L-8) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX94).

The compound (L-8) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX95).

The compound (L-8) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX96).

A compound represented by formula (L-9):

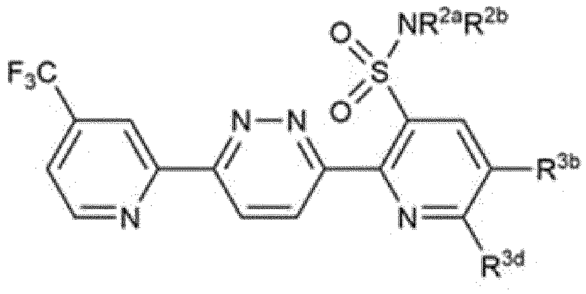

( L-9 )

(hereinafter referred to as compound (L-9)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX97).

The compound (L-9) wherein $R^{2a}$ is a methyl group, $R^{2b}$ is a hydrogen atom, $R^{3b}$ is a hydrogen atom, and $R^{3d}$ is

any one substituent mentioned in [Table 7A] to [Table 15A] (hereinafter referred to as compound group SX98).

The compound (L-9) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX99).

The compound (L-9) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX100).

The compound (L-9) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX101).

The compound (L-9) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX102).

The compound (L-9) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX103).

The compound (L-9) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX104).

The compound (L-9) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX105).

The compound (L-9) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX106).

The compound (L-9) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX107).

The compound (L-9) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX108).

The compound (L-9) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX109).

The compound (L-9) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX110).

The compound (L-9) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX111).

The compound (L-9) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX112).

A compound represented by formula (L-10):

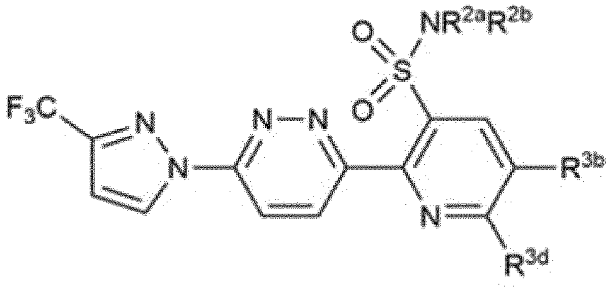

(L-10)

(hereinafter referred to as compound (L-10)) wherein $R^{2a}$ and $R^{2b}$ area hydrogen atom, $R^{3b}$ is a hydrogen atom, and $R^{3d}$ is any one substituent mentioned in [Table 7A] to [Table 15A] (hereinafter referred to as compound group SX113).

The compound (L-10) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX114).

The compound (L-10) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX115).

The compound (L-10) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX116).

The compound (L-10) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX117).

The compound (L-10) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX118).

The compound (L-10) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX119).

The compound (L-10) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX120).

The compound (L-10) wherein $R^{2a}$ and $R^{2b}$ are a hydrogen atom, $R^{3d}$ is a hydrogen atom, and $R^{3b}$ is any one

substituent mentioned in [Table 7A] to [Table 15A] (hereinafter referred to as compound group SX121).

The compound (L-10) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX122).

The compound (L-10) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX123).

The compound (L-10) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX124).

The compound (L-10) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX125).

The compound (L-10) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX126).

The compound (L-10) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX127).

The compound (L-10) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX128).

A compound represented by formula (L-11):

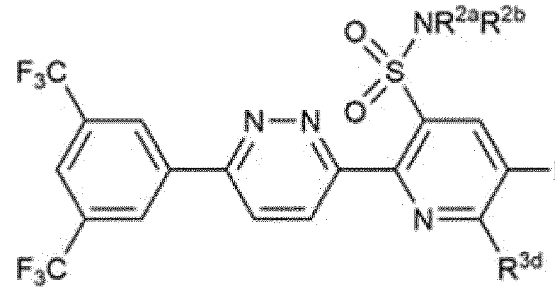

(L-11)

(hereinafter referred to as compound (L-11)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX129).

The compound (L-11) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX130).

The compound (L-11) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX131).

The compound (L-11) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX132).

The compound (L-11) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX133).

The compound (L-11) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX134).

The compound (L-11) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX135).

The compound (L-11) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX136).

The compound (L-11) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX137).

The compound (L-11) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX138).

The compound (L-11) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX139).

The compound (L-11) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX140).

The compound (L-11) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX141).

The compound (L-11) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX142).

The compound (L-11) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX143).

The compound (L-11) wherein $R^{2a}$ is a methyl group, $R^{2b}$ is an ethyl group, $R^{3d}$ is a hydrogen atom, and $R^{3b}$ is any

one substituent mentioned in [Table 7A] to [Table 15A] (hereinafter referred to as compound group SX144).

A compound represented by formula (L-12):

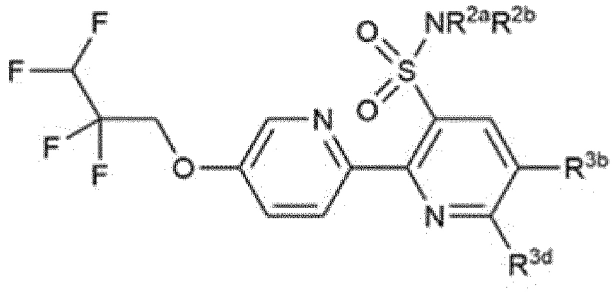

(L-12)

(hereinafter referred to as compound (L-12)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX145).

The compound (L-12) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX146).

The compound (L-12) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX147).

The compound (L-12) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX148).

The compound (L-12) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX149).

The compound (L-12) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX150).

The compound (L-12) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX151).

The compound (L-12) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX152).

The compound (L-12) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX153).

The compound (L-12) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX154).

The compound (L-12) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX155).

The compound (L-12) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX156).

The compound (L-12) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX157).

The compound (L-12) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX158).

The compound (L-12) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX159).

The compound (L-12) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX160).

A compound represented by formula (L-13):

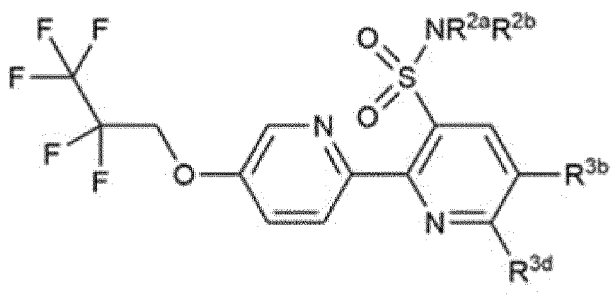

(L-13)

(hereinafter referred to as compound (L-13)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX161).

The compound (L-13) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX162).

The compound (L-13) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX163).

The compound (L-13) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX164).

The compound (L-13) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX165).

The compound (L-13) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX166).

The compound (L-13) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX167).

The compound (L-13) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX168).

The compound (L-13) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX169).

The compound (L-13) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX170).

The compound (L-13) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX171).

The compound (L-13) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX172).

The compound (L-13) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX173).

The compound (L-13) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX174).

The compound (L-13) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX175).

The compound (L-13) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX176).

A compound represented by formula (L-14):

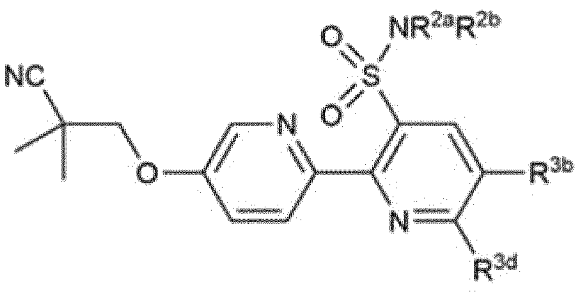

(L-14)

(hereinafter referred to as compound (L-14)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX177).

The compound (L-14) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX178).

The compound (L-14) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX179).

The compound (L-14) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX180).

The compound (L-14) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX181).

The compound (L-14) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX182).

The compound (L-14) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX183).

The compound (L-14) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX184).

The compound (L-14) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX185).

The compound (L-14) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX186).

The compound (L-14) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX187).

The compound (L-14) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX188).

The compound (L-14) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX189).

The compound (L-14) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX190).

The compound (L-14) wherein $R^{2a}$ is a propyl group, $R^{2b}$ is a propyl group, $R^{3d}$ is a hydrogen atom, $R^{3b}$ is any one substituent mentioned in [Table 7A] to [Table 15A] (hereinafter referred to as compound group SX191).

The compound (L-14) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX192).

A compound represented by formula (L-15):

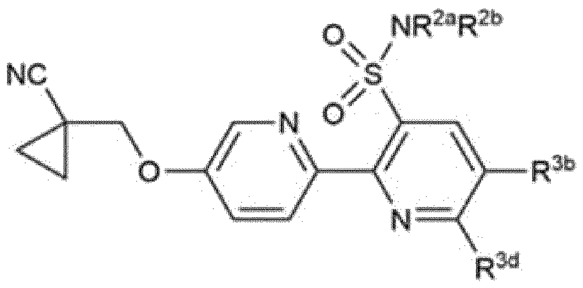

(L-15)

(hereinafter referred to as compound (L-15)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX193).

The compound (L-15) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX194).

The compound (L-15) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX195).

The compound (L-15) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX196).

The compound (L-15) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX197).

The compound (L-15) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX198).

The compound (L-15) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX199).

The compound (L-15) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX200).

The compound (L-15) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX201).

The compound (L-15) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX202).

The compound (L-15) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX203).

The compound (L-15) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX204).

The compound (L-15) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX205).

The compound (L-15) wherein $R^{2a}$ is a propyl group, $R^{2b}$ is a hydrogen atom, $R^{3d}$ is a hydrogen atom, $R^{3b}$ is any one substituent mentioned in [Table 7A] to [Table 15A] (hereinafter referred to as compound group SX206).

The compound (L-15) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX207).
The compound (L-15) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX208).
A compound represented by formula (L-16):

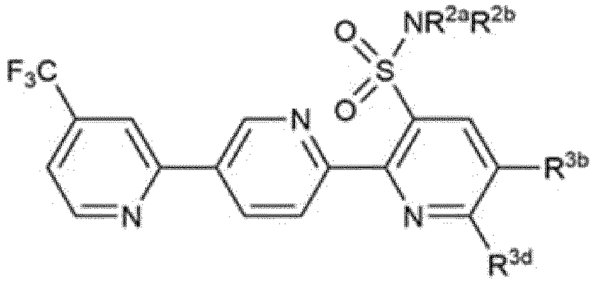

(L-16)

(hereinafter referred to as compound (L-16)) wherein $R^{2a}$ and $R^{2b}$ are a hydrogen atom, $R^{3b}$ is a hydrogen atom, $R^{3d}$ is any one substituent mentioned in [Table 7A] to [Table 15A] (hereinafter referred to as compound group SX209).
The compound (L-16) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX210).
The compound (L-16) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX211).
The compound (L-16) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX212).
The compound (L-16) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX213).
The compound (L-16) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX214).
The compound (L-16) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX215).
The compound (L-16) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX216).
The compound (L-16) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX217).
The compound (L-16) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX218).
The compound (L-16) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX219).
The compound (L-16) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX220).
The compound (L-16) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX221).
The compound (L-16) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX222).
The compound (L-16) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX223).
The compound (L-16) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX224).
A compound represented by formula (L-17):

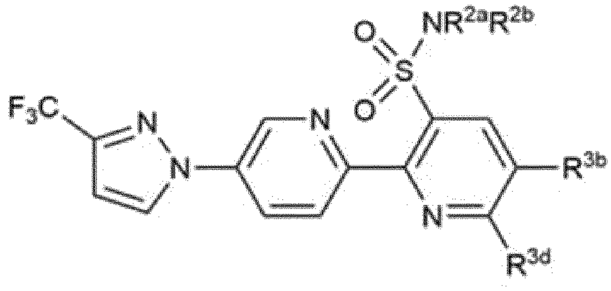

(L-17)

(hereinafter referred to as compound (L-17)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX225).

The compound (L-17) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX226).

The compound (L-17) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX227).

The compound (L-17) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX228).

The compound (L-17) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX229).

The compound (L-17) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX230).

The compound (L-17) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX231).

The compound (L-17) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX232).

The compound (L-17) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX233).

The compound (L-17) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX234).

The compound (L-17) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX235).

The compound (L-17) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX236).

The compound (L-17) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX237).

The compound (L-17) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX238).

The compound (L-17) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX239).

The compound (L-17) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX240).

A compound represented by formula (L-18):

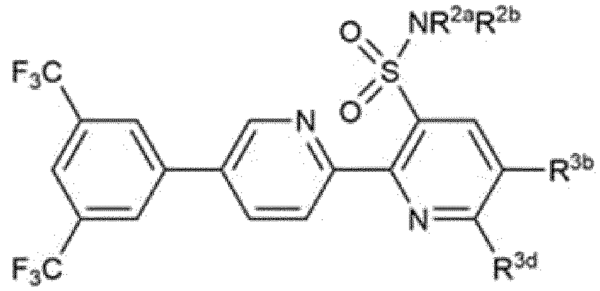

(L-18)

(hereinafter referred to as compound (L-18)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX241).

The compound (L-18) wherein $R^{2a}$ is a methyl group, $R^{2b}$ is a hydrogen atom, $R^{3b}$ is a hydrogen atom, and $R^{3d}$ is

any one substituent mentioned in [Table 7A] to [Table 15A] (hereinafter referred to as compound group SX242).

The compound (L-18) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX243).

The compound (L-18) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX244).

The compound (L-18) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX245).

The compound (L-18) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX246).

The compound (L-18) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX247).

The compound (L-18) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX248).

The compound (L-18) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX249).

The compound (L-18) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX250).

The compound (L-18) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX251).

The compound (L-18) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX252).

The compound (L-18) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX253).

The compound (L-18) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX254).

The compound (L-18) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX255).

The compound (L-18) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX256).

A compound represented by formula (L-19):

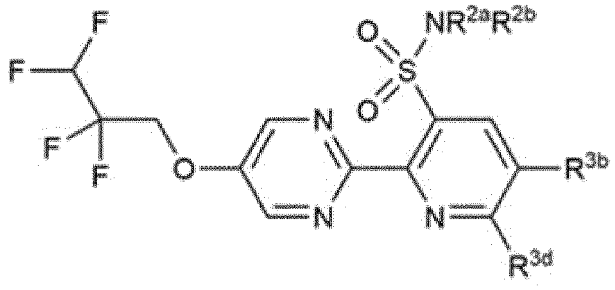

(L-19)

(hereinafter referred to as compound (L-19)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX257).

The compound (L-19) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX258).

The compound (L-19) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX259).

The compound (L-19) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX260).

The compound (L-19) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX261).

The compound (L-19) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX262).

The compound (L-19) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX263).

The compound (L-19) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX264).

The compound (L-19) wherein $R^{2a}$ and $R^{2b}$ are a hydrogen atom, $R^{3d}$ is a hydrogen atom, and $R^{3b}$ is any one

substituent mentioned in [Table 7A] to [Table 15A] (hereinafter referred to as compound group SX265).

The compound (L-19) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX266).

The compound (L-19) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX267).

The compound (L-19) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX268).

The compound (L-19) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX269).

The compound (L-19) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX270).

The compound (L-19) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX271).

The compound (L-19) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX272).

A compound represented by formula (L-20):

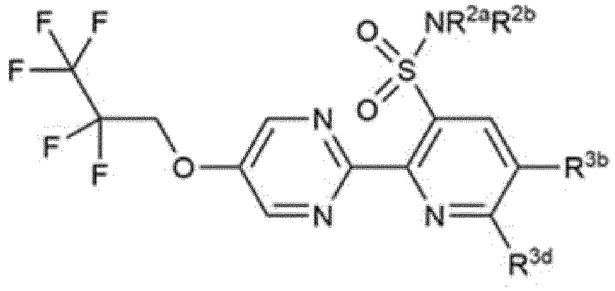

(L-20)

(hereinafter referred to as compound (L-20)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX273).

The compound (L-20) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX274).

The compound (L-20) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX275).

The compound (L-20) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX276).

The compound (L-20) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX277).

The compound (L-20) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX278).

The compound (L-20) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX279).

The compound (L-20) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX280).

The compound (L-20) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX281).

The compound (L-20) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX282).

The compound (L-20) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX283).

The compound (L-20) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX284).

The compound (L-20) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX285).

The compound (L-20) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX286).

The compound (L-20) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX287).

The compound (L-20) wherein $R^{2a}$ is a methyl group, $R^{2b}$ is an ethyl group, $R^{3d}$ is a hydrogen atom, and $R^{3b}$ is any

one substituent mentioned in [Table 7A] to [Table 15A] (hereinafter referred to as compound group SX288).

A compound represented by formula (L-21):

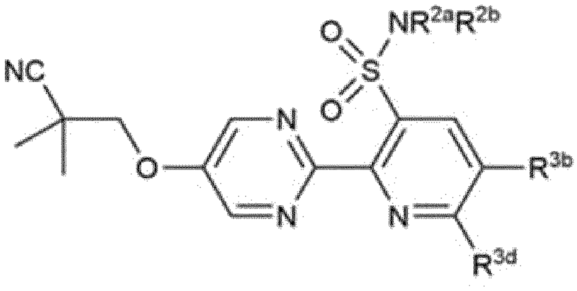

(L-21)

(hereinafter referred to as compound (L-21)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX289).

The compound (L-21) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX290).

The compound (L-21) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX291).

The compound (L-21) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX292).

The compound (L-21) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX293).

The compound (L-21) wherein $R^{2a}$ is a propyl group, $R^{2b}$ is a hydrogen atom, $R^{3b}$ is a hydrogen atom, $R^{3d}$ is any one substituent mentioned in [Table 7A] to [Table 15A] (hereinafter referred to as compound group SX294).

The compound (L-21) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX295).

The compound (L-21) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX296).

The compound (L-21) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX297).

The compound (L-21) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX298).

The compound (L-21) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX299).

The compound (L-21) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX300).

The compound (L-21) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX301).

The compound (L-21) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX302).

The compound (L-21) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX303).

The compound (L-21) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX304).

A compound represented by formula (L-22):

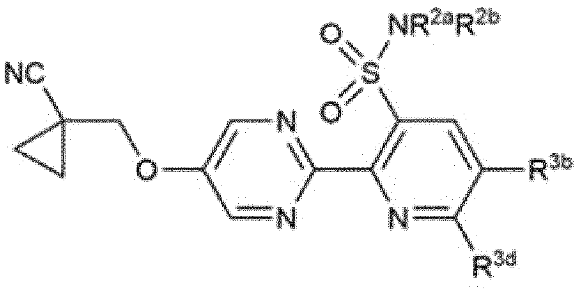

(L-22)

(hereinafter referred to as compound (L-22)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX305).

The compound (L-22) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX306).

The compound (L-22) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX307).

The compound (L-22) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX308).

The compound (L-22) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX309).

The compound (L-22) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX310).

The compound (L-22) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX311).

The compound (L-22) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX312).

The compound (L-22) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX313).

The compound (L-22) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX314).

The compound (L-22) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX315).

The compound (L-22) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX316).

The compound (L-22) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX317).

The compound (L-22) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX318).

The compound (L-22) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX319).

The compound (L-22) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX320).

A compound represented by formula (L-23):

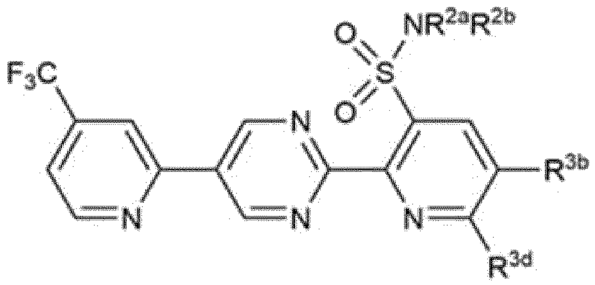

(L-23)

(hereinafter referred to as compound (L-23)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX321).

The compound (L-23) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX322).

The compound (L-23) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX323).

The compound (L-23) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX324).

The compound (L-23) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX325).

The compound (L-23) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX326).

The compound (L-23) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX327).

The compound (L-23) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX328).

The compound (L-23) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX329).

The compound (L-23) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX330).

The compound (L-23) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX331).

The compound (L-23) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX332).

The compound (L-23) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX333).

The compound (L-23) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX334).

The compound (L-23) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX335).

The compound (L-23) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX336).

A compound represented by formula (L-24):

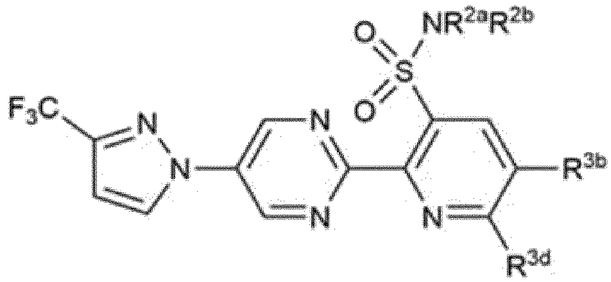

(L-24)

(hereinafter referred to as compound (L-24)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX337).

The compound (L-24) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX338).

The compound (L-24) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX339).

The compound (L-24) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX340).

The compound (L-24) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX341).

The compound (L-24) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX342).

The compound (L-24) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX343).

The compound (L-24) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX344).

The compound (L-24) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX345).

The compound (L-24) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX346).

The compound (L-24) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX347).

The compound (L-24) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX348).

The compound (L-24) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX349).

The compound (L-24) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX350).

The compound (L-24) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX351).
The compound (L-24) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX352).
A compound represented by formula (L-25):

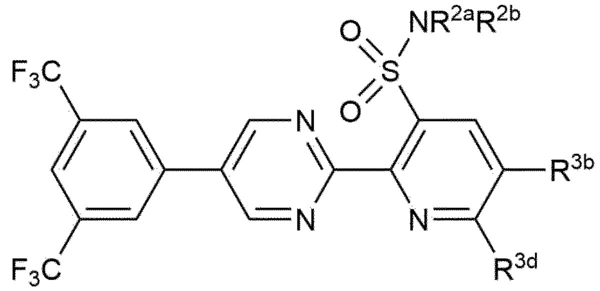

( L-25 )

(hereinafter referred to as compound (L-25)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX353).
The compound (L-25) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX354).
The compound (L-25) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX355).
The compound (L-25) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX356).
The compound (L-25) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX357).
The compound (L-25) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX358).
The compound (L-25) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX359).
The compound (L-25) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX360).
The compound (L-25) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX361).
The compound (L-25) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX362).
The compound (L-25) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX363).
The compound (L-25) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX364).
The compound (L-25) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX365).
The compound (L-25) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX366).
The compound (L-25) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX367).
The compound (L-25) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX368).
A compound represented by formula (L-26):

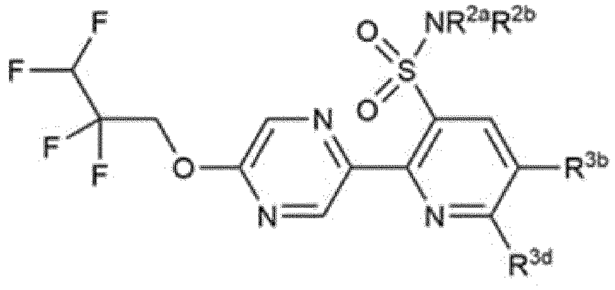

( L-26 )

(hereinafter referred to as compound (L-26)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX369).

The compound (L-26) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX370).

The compound (L-26) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX371).

The compound (L-26) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX372).

The compound (L-26) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX373).

The compound (L-26) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX374).

The compound (L-26) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX375).

The compound (L-26) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX376).

The compound (L-26) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX377).

The compound (L-26) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX378).

The compound (L-26) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX379).

The compound (L-26) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX380).

The compound (L-26) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX381).

The compound (L-26) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX382).

The compound (L-26) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX383).

The compound (L-26) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX384).

A compound represented by formula (L-27):

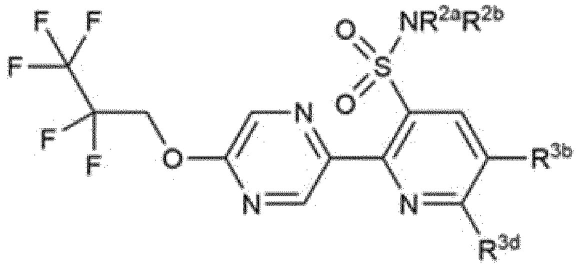

( L-27 )

(hereinafter referred to as compound (L-27)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX385).

The compound (L-27) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX386).

The compound (L-27) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX387).

The compound (L-27) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX388).

The compound (L-27) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX389).

The compound (L-27) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX390).

The compound (L-27) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX391).

The compound (L-27) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX392).

The compound (L-27) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX393).

The compound (L-27) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX394).

The compound (L-27) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX395).

The compound (L-27) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX396).

The compound (L-27) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX397).

The compound (L-27) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX398).

The compound (L-27) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX399).

The compound (L-27) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX400).

A compound represented by formula (L-28):

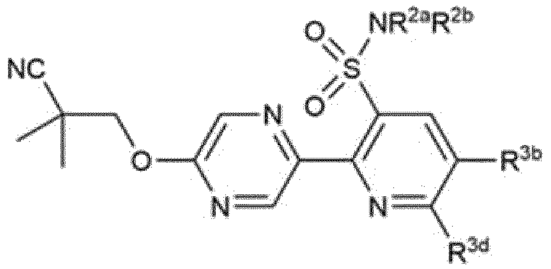

( L-28 )

(hereinafter referred to as compound (L-28)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX401).

The compound (L-28) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX402).

The compound (L-28) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX403).

The compound (L-28) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX404).

The compound (L-28) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX405).

The compound (L-28) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX406).

The compound (L-28) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX407).

The compound (L-28) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX408).

The compound (L-28) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX409).

The compound (L-28) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX410).
The compound (L-28) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX411).
The compound (L-28) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX412).
The compound (L-28) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX413).
The compound (L-28) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX414).
The compound (L-28) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX415).
The compound (L-28) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX416).
A compound represented by formula (L-29):

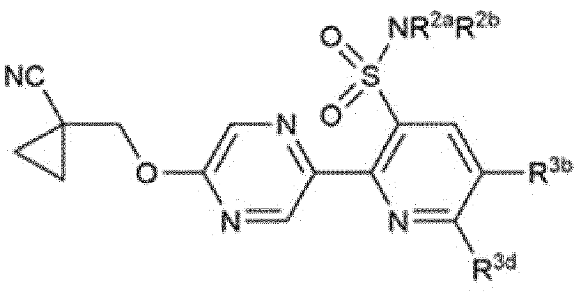

(L-29)

(hereinafter referred to as compound (L-29)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX417).
The compound (L-29) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX418).
The compound (L-29) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX419).
The compound (L-29) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX420).
The compound (L-29) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX421).
The compound (L-29) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX422).
The compound (L-29) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX423).
The compound (L-29) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX424).
The compound (L-29) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX425).
The compound (L-29) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX426).
The compound (L-29) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX427).
The compound (L-29) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX428).
The compound (L-29) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX429).
The compound (L-29) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX430).
The compound (L-29) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX431).
The compound (L-29) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX432).

A compound represented by formula (L-30):

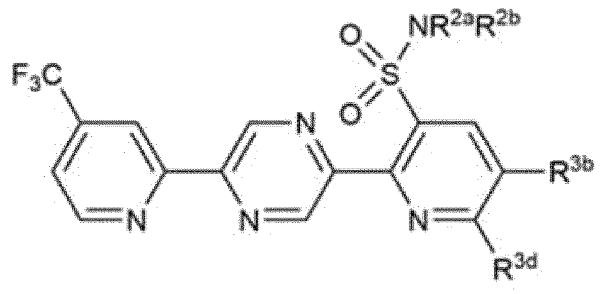

( L-30 )

(hereinafter referred to as compound (L-30)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX433).
The compound (L-30) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX434).
The compound (L-30) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX435).
The compound (L-30) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX436).
The compound (L-30) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX437).
The compound (L-30) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX438).
The compound (L-30) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX439).
The compound (L-30) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX440).
The compound (L-30) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX441).
The compound (L-30) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX442).
The compound (L-30) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX443).
The compound (L-30) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX444).
The compound (L-30) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX445).
The compound (L-30) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX446).
The compound (L-30) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX447).
The compound (L-30) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX448).
A compound represented by formula (L-31):

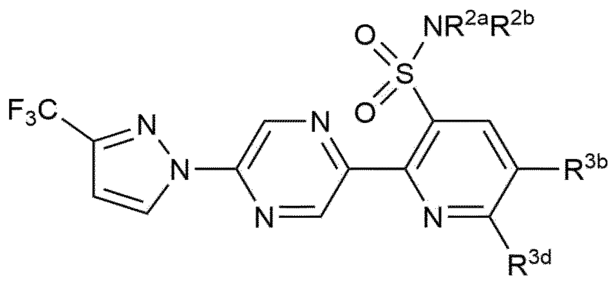

( L-31 )

(hereinafter referred to as compound (L-31)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX449).

The compound (L-31) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX450).
The compound (L-31) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX451).
The compound (L-31) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX452).
The compound (L-31) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX453).
The compound (L-31) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX454).
The compound (L-31) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX455).
The compound (L-31) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX456).
The compound (L-31) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX457).
The compound (L-31) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX458).
The compound (L-31) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX459).
The compound (L-31) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX460).
The compound (L-31) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX461).
The compound (L-31) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX462).
The compound (L-31) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX463).
The compound (L-31) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX464).
A compound represented by formula (L-32):

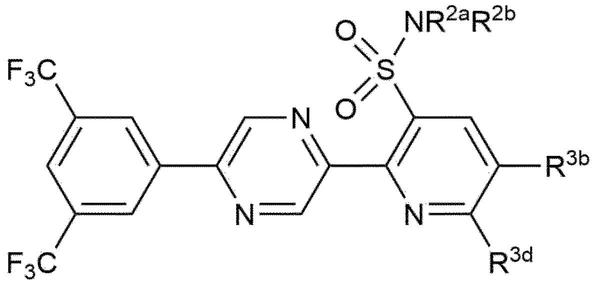

( L-32 )

(hereinafter referred to as compound (L-32)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX465).
The compound (L-32) wherein $R^{2a}$ is a methyl group, $R^{2b}$ is a hydrogen atom, $R^{3b}$ is a hydrogen atom, $R^{3d}$ is any one substituent mentioned in [Table 7A] to [Table 15A] (hereinafter referred to as compound group SX466).
The compound (L-32) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX467).
The compound (L-32) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX468).
The compound (L-32) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX469).
The compound (L-32) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX470).
The compound (L-32) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX471).
The compound (L-32) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX472).

The compound (L-32) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX473).
The compound (L-32) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX474).
The compound (L-32) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX475).
The compound (L-32) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX476).
The compound (L-32) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX477).
The compound (L-32) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX478).
The compound (L-32) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX479).
The compound (L-32) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX480).
A compound represented by formula (L-33):

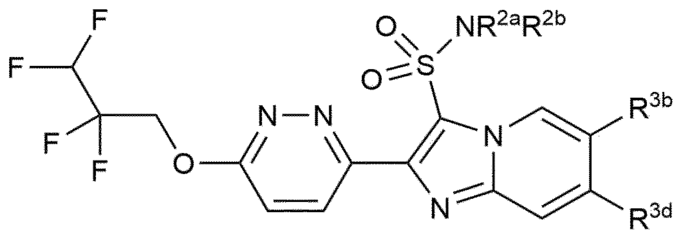

( L-33 )

(hereinafter referred to as compound (L-33)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX481).
The compound (L-33) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX482).
The compound (L-33) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX483).
The compound (L-33) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX484).
The compound (L-33) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX485).
The compound (L-33) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX486).
The compound (L-33) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX487).
The compound (L-33) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX488).
The compound (L-33) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX489).
The compound (L-33) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX490).
The compound (L-33) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX491).
The compound (L-33) wherein $R^{2a}$ is an ethyl group, $R^{2b}$ is a hydrogen atom, $R^{3d}$ is a hydrogen atom, $R^{3b}$ is any one substituent mentioned in [Table 7A] to [Table 15A] (hereinafter referred to as compound group SX492).
The compound (L-33) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX493).
The compound (L-33) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX494).
The compound (L-33) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX495).
The compound (L-33) wherein $R^{2a}$ is a methyl group, $R^{2b}$ is an ethyl group, $R^{3d}$ is a hydrogen atom, and $R^{3b}$ is any

one substituent mentioned in [Table 7A] to [Table 15A] (hereinafter referred to as compound group SX496).

A compound represented by formula (L-34):

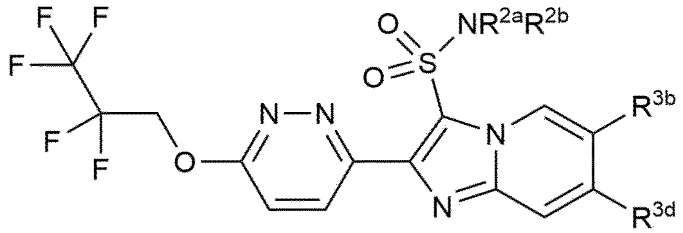

( L-34 )

(hereinafter referred to as compound (L-34)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX497).

The compound (L-34) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX498).

The compound (L-34) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX499).

The compound (L-34) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX500).

The compound (L-34) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX501).

The compound (L-34) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX502).

The compound (L-34) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX503).

The compound (L-34) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX504).

The compound (L-34) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX505).

The compound (L-34) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX506).

The compound (L-34) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX507).

The compound (L-34) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX508).

The compound (L-34) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX509).

The compound (L-34) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX510).

The compound (L-34) wherein $R^{2a}$ is a propyl group, $R^{2b}$ is a propyl group, $R^{3d}$ is a hydrogen atom, $R^{3b}$ is any one substituent mentioned in [Table 7A] to [Table 15A] (hereinafter referred to as compound group SX511).

The compound (L-34) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX512).

A compound represented by formula (L-35):

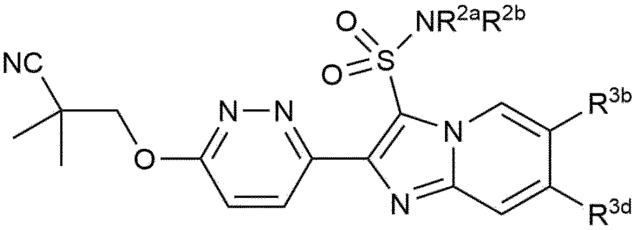

( L-35 )

(hereinafter referred to as compound (L-35)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX513).

The compound (L-35) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX514).

The compound (L-35) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX515).
The compound (L-35) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX516).
The compound (L-35) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX517).
The compound (L-35) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX518).
The compound (L-35) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX519).
The compound (L-35) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX520).
The compound (L-35) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX521).
The compound (L-35) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX522).
The compound (L-35) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX523).
The compound (L-35) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX524).
The compound (L-35) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX525).
The compound (L-35) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX526).
The compound (L-35) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX527).
The compound (L-35) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX528).
A compound represented by formula (L-36):

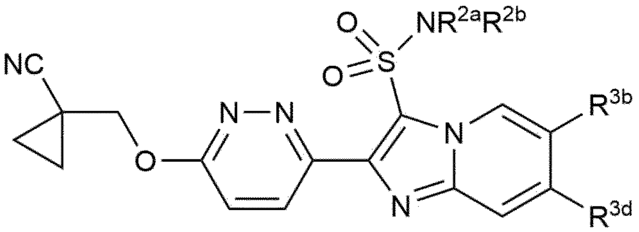

( L-36 )

(hereinafter referred to as compound (L-36)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX529).
The compound (L-36) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX530).
The compound (L-36) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX531).
The compound (L-36) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX532).
The compound (L-36) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX533).
The compound (L-36) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX534).
The compound (L-36) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX535).
The compound (L-36) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX536).
The compound (L-36) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX537).
The compound (L-36) wherein $R^{2a}$ is a methyl group, $R^{2b}$ is a hydrogen atom, $R^{3d}$ is a hydrogen atom, and $R^{3b}$ is

any one substituent mentioned in [Table 7A] to [Table 15A] (hereinafter referred to as compound group SX538).

The compound (L-36) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX539).

The compound (L-36) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX540).

The compound (L-36) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX541).

The compound (L-36) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX542).

The compound (L-36) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX543).

The compound (L-36) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX544).

A compound formula (L-37):

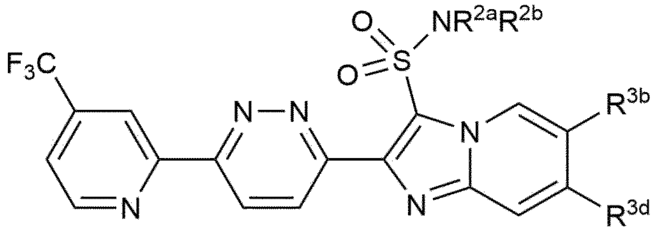

( L-37 )

(hereinafter referred to as compound (L-37)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX545).

The compound (L-37) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX546).

The compound (L-37) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX547).

The compound (L-37) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX548).

The compound (L-37) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX549).

The compound (L-37) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX550).

The compound (L-37) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX551).

The compound (L-37) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX552).

The compound (L-37) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX553).

The compound (L-37) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX554).

The compound (L-37) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX555).

The compound (L-37) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX556).

The compound (L-37) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX557).

The compound (L-37) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX558).

The compound (L-37) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX559).

The compound (L-37) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX560).

A compound represented by formula (L-38):

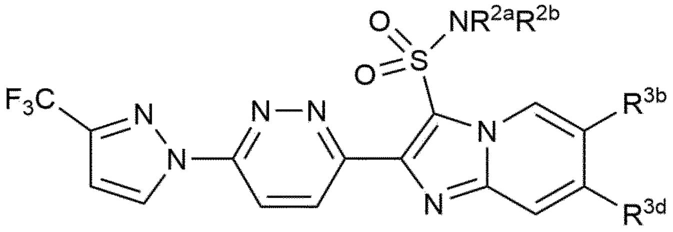

( L-38 )

(hereinafter referred to as compound (L-38)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX561).

The compound (L-38) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX562).

The compound (L-38) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX563).

The compound (L-38) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX564).

The compound (L-38) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX565).

The compound (L-38) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX566).

The compound (L-38) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX567).

The compound (L-38) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX568).

The compound (L-38) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX569).

The compound (L-38) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX570).

The compound (L-38) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX571).

The compound (L-38) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX572).

The compound (L-38) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX573).

The compound (L-38) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX574).

The compound (L-38) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX575).

The compound (L-38) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX576).

A compound represented by formula (L-39):

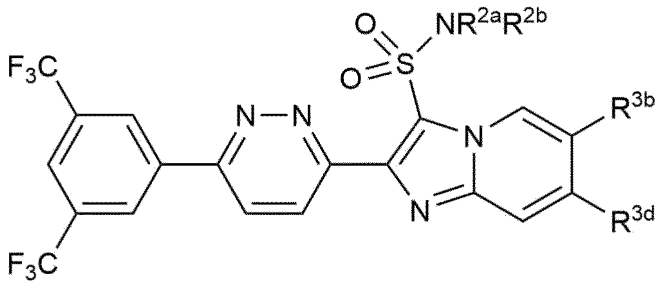

( L-39 )

(hereinafter referred to as compound (L-39)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX577).

The compound (L-39) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX578).

The compound (L-39) wherein $R^{2a}$ is a methyl group, $R^{2b}$ is a methyl group, $R^{3b}$ is a hydrogen atom, and $R^{3d}$ is

any one substituent mentioned in [Table 7A] to [Table 15A] (hereinafter referred to as compound group SX579).

The compound (L-39) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX580).

The compound (L-39) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX581).

The compound (L-39) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX582).

The compound (L-39) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX583).

The compound (L-39) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX584).

The compound (L-39) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX585).

The compound (L-39) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX586).

The compound (L-39) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX587).

The compound (L-39) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX588).

The compound (L-39) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX589).

The compound (L-39) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX590).

The compound (L-39) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX591).

The compound (L-39) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX592).

A compound represented by formula (L-40):

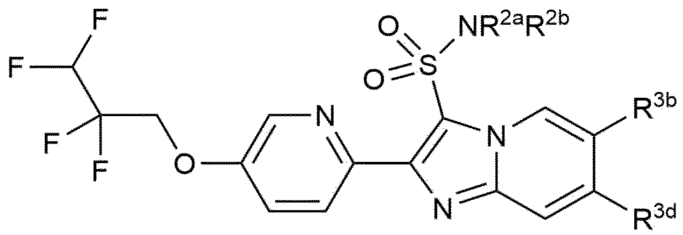

( L-40 )

(hereinafter referred to as compound (L-40)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX593).

The compound (L-40) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX594).

The compound (L-40) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX595).

The compound (L-40) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX596).

The compound (L-40) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX597).

The compound (L-40) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX598).

The compound (L-40) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX599).

The compound (L-40) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX600).

The compound (L-40) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX601).

The compound (L-40) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX602).

The compound (L-40) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX603).
The compound (L-40) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX604).
The compound (L-40) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX605).
The compound (L-40) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX606).
The compound (L-40) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX607).
The compound (L-40) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX608).
A compound represented by formula (L-41):

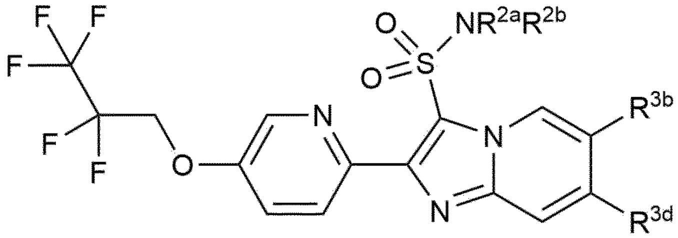

( L-41 )

(hereinafter referred to as compound (L-41)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX609).
The compound (L-41) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX610).
The compound (L-41) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX611).
The compound (L-41) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX612).
The compound (L-41) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX613).
The compound (L-41) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX614).
The compound (L-41) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX615).
The compound (L-41) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX616).
The compound (L-41) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX617).
The compound (L-41) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX618).
The compound (L-41) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX619).
The compound (L-41) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX620).
The compound (L-41) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX621).
The compound (L-41) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX622).
The compound (L-41) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX623).
The compound (L-41) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX624).
A compound represented by formula (L-42):

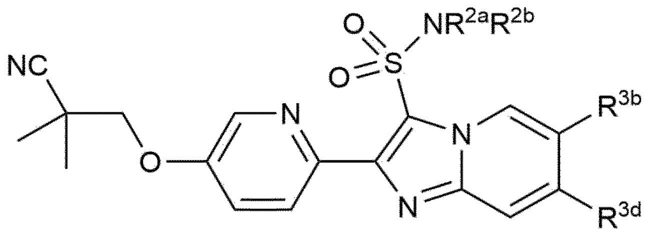

( L-42 )

(hereinafter referred to as compound (L-42)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX625).

The compound (L-42) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX626).

The compound (L-42) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX627).

The compound (L-42) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX628).

The compound (L-42) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX629).

The compound (L-42) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX630).

The compound (L-42) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX631).

The compound (L-42) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX632).

The compound (L-42) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX633).

The compound (L-42) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX634).

The compound (L-42) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX635).

The compound (L-42) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX636).

The compound (L-42) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX637).

The compound (L-42) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX638).

The compound (L-42) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX639).

The compound (L-42) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX640).

A compound represented by formula (L-43):

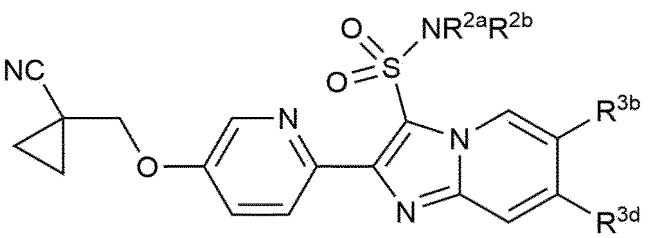

( L-43 )

(hereinafter referred to as compound (L-43)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX641).

The compound (L-43) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX642).

The compound (L-43) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX643).

The compound (L-43) wherein $R^{2a}$ is an ethyl group, $R^{2b}$ is a hydrogen atom, $R^{3b}$ is a hydrogen atom, and $R^{3d}$ is

any one substituent mentioned in [Table 7A] to [Table 15A] (hereinafter referred to as compound group SX644).

The compound (L-43) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX645).

The compound (L-43) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX646).

The compound (L-43) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX647).

The compound (L-43) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX648).

The compound (L-43) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX649).

The compound (L-43) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX650).

The compound (L-43) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX651).

The compound (L-43) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX652).

The compound (L-43) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX653).

The compound (L-43) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX654).

The compound (L-43) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX655).

The compound (L-43) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX656).

A compound represented by formula (L-44):

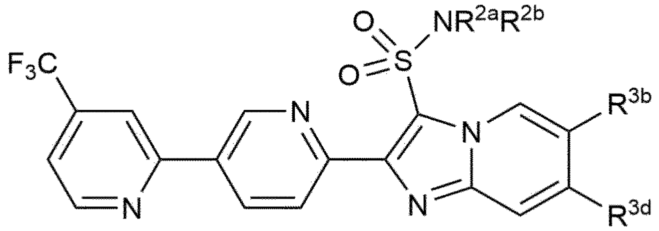

( L-44 )

(hereinafter referred to as compound (L-44)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX657).

The compound (L-44) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX658).

The compound (L-44) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX659).

The compound (L-44) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX660).

The compound (L-44) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX661).

The compound (L-44) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX662).

The compound (L-44) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX663).

The compound (L-44) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX664).

The compound (L-44) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX665).

The compound (L-44) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX666).

The compound (L-44) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX667).

The compound (L-44) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX668).
The compound (L-44) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX669).
The compound (L-44) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX670).
The compound (L-44) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX671).
The compound (L-44) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX672).
A compound represented by formula (L-45):

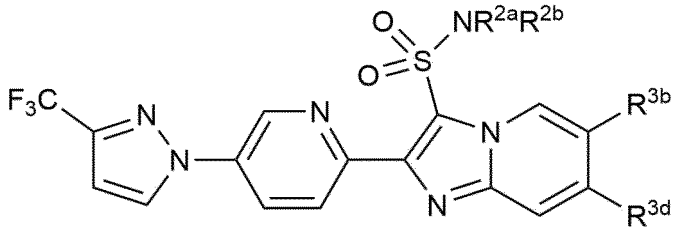

( L-45 )

(hereinafter referred to as compound (L-45)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX673).
The compound (L-45) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX674).
The compound (L-45) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX675).
The compound (L-45) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX676).
The compound (L-45) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX677).
The compound (L-45) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX678).
The compound (L-45) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX679).
The compound (L-45) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX680).
The compound (L-45) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX681).
The compound (L-45) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX682).
The compound (L-45) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX683).
The compound (L-45) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX684).
The compound (L-45) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX685).
The compound (L-45) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX686).
The compound (L-45) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX687).
The compound (L-45) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX688).
A compound represented by formula (L-46):

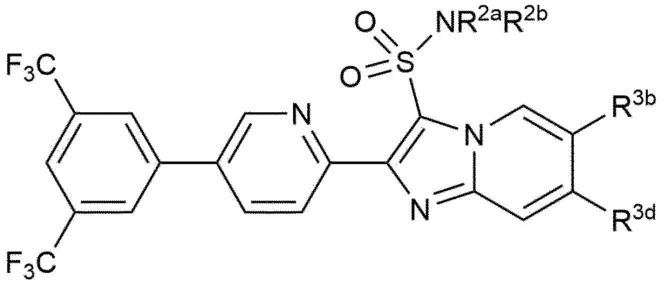

( L-46 )

(hereinafter referred to as compound (L-46)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX689).

The compound (L-46) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX690).

The compound (L-46) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX691).

The compound (L-46) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX692).

The compound (L-46) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX693).

The compound (L-46) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX694).

The compound (L-46) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX695).

The compound (L-46) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX696).

The compound (L-46) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX697).

The compound (L-46) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX698).

The compound (L-46) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX699).

The compound (L-46) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX700).

The compound (L-46) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX701).

The compound (L-46) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX702).

The compound (L-46) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX703).

The compound (L-46) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX704).

A compound represented by formula (L-47):

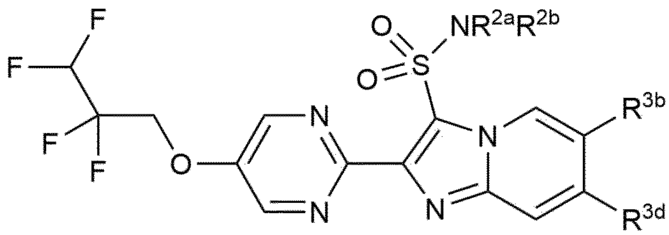

( L-47 )

(hereinafter referred to as compound (L-47)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX705).

The compound (L-47) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX706).

The compound (L-47) wherein $R^{2a}$ is a methyl group, $R^{2b}$ is a methyl group, $R^{3b}$ is a hydrogen atom, and $R^{3d}$ is

any one substituent mentioned in [Table 7A] to [Table 15A] (hereinafter referred to as compound group SX707).

The compound (L-47) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX708).

The compound (L-47) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX709).

The compound (L-47) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX710).

The compound (L-47) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX711).

The compound (L-47) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX712).

The compound (L-47) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX713).

The compound (L-47) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX714).

The compound (L-47) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX715).

The compound (L-47) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX716).

The compound (L-47) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX717).

The compound (L-47) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX718).

The compound (L-47) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX719).

The compound (L-47) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX720).

A compound represented by formula (L-48):

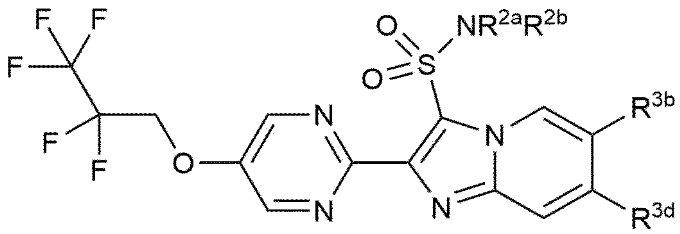

( L-48 )

(hereinafter referred to as compound (L-48)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX721).

The compound (L-48) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX722).

The compound (L-48) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX723).

The compound (L-48) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX724).

The compound (L-48) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX725).

The compound (L-48) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX726).

The compound (L-48) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX727).

The compound (L-48) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX728).

The compound (L-48) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX729).

The compound (L-48) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX730).

The compound (L-48) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX731).

The compound (L-48) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX732).

The compound (L-48) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX733).

The compound (L-48) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX734).

The compound (L-48) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX735).

The compound (L-48) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX736).

A compound represented by formula (L-49):

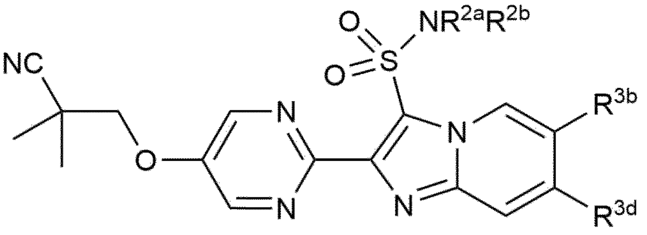

( L-49 )

(hereinafter referred to as compound (L-49)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX737).

The compound (L-49) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX738).

The compound (L-49) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX739).

The compound (L-49) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX740).

The compound (L-49) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX741).

The compound (L-49) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX742).

The compound (L-49) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX743).

The compound (L-49) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX744).

The compound (L-49) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX745).

The compound (L-49) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX746).

The compound (L-49) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX747).

The compound (L-49) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX748).

The compound (L-49) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX749).

The compound (L-49) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX750).

The compound (L-49) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX751).

The compound (L-49) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX752).

A compound represented by formula (L-50):

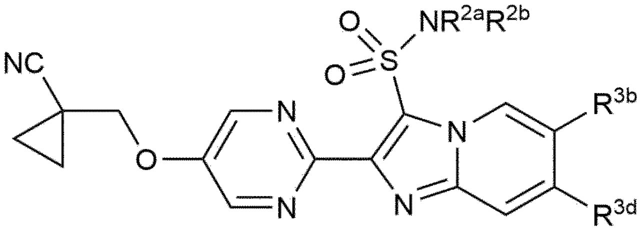

( L-50 )

(hereinafter referred to as compound (L-50)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX753).

The compound (L-50) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX754).

The compound (L-50) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX755).

The compound (L-50) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX756).

The compound (L-50) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX757).

The compound (L-50) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX758).

The compound (L-50) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX759).

The compound (L-50) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX760).

The compound (L-50) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX761).

The compound (L-50) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX762).

The compound (L-50) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX763).

The compound (L-50) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX764).

The compound (L-50) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX765).

The compound (L-50) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX766).

The compound (L-50) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX767).

The compound (L-50) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX768).

A compound represented by formula (L-51):

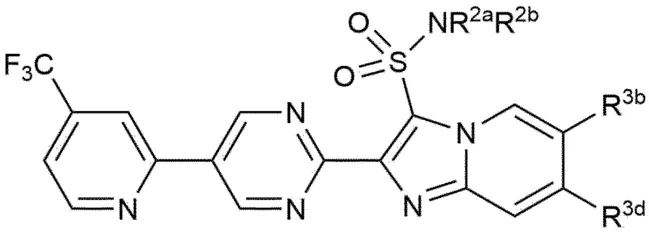

( L-51 )

(hereinafter referred to as compound (L-51)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX769).

The compound (L-51) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX770).

The compound (L-51) wherein $R^{2a}$ is a methyl group, $R^{2b}$ is a methyl group, $R^{3b}$ is a hydrogen atom, $R^{3d}$ is any one substituent mentioned in [Table 7A] to [Table 15A] (hereinafter referred to as compound group SX771).

The compound (L-51) wherein $R^{2a}$ is an ethyl group, $R^{2b}$ is a hydrogen atom, $R^{3b}$ is a hydrogen atom, and $R^{3d}$ is

any one substituent mentioned in [Table 7A] to [Table 15A] (hereinafter referred to as compound group SX772).

The compound (L-51) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX773).

The compound (L-51) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX774).

The compound (L-51) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX775).

The compound (L-51) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX776).

The compound (L-51) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX777).

The compound (L-51) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX778).

The compound (L-51) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX779).

The compound (L-51) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX780).

The compound (L-51) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX781).

The compound (L-51) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX782).

The compound (L-51) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX783).

The compound (L-51) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX784).

A compound represented by formula (L-52):

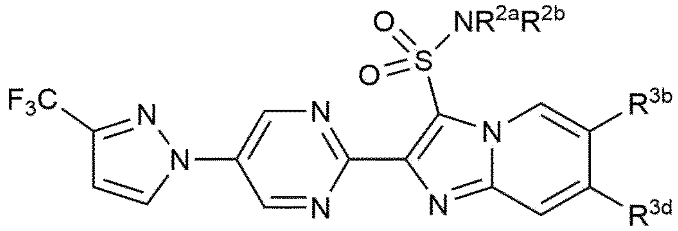

( L-52 )

(hereinafter referred to as compound (L-52)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX785).

The compound (L-52) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX786).

The compound (L-52) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX787).

The compound (L-52) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX788).

The compound (L-52) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX789).

The compound (L-52) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX790).

The compound (L-52) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX791).

The compound (L-52) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX792).

The compound (L-52) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX793).

The compound (L-52) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX794).

The compound (L-52) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX795).

The compound (L-52) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX796).
The compound (L-52) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX797).
The compound (L-52) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX798).
The compound (L-52) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX799).
The compound (L-52) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX800).
A compound represented by formula (L-53):

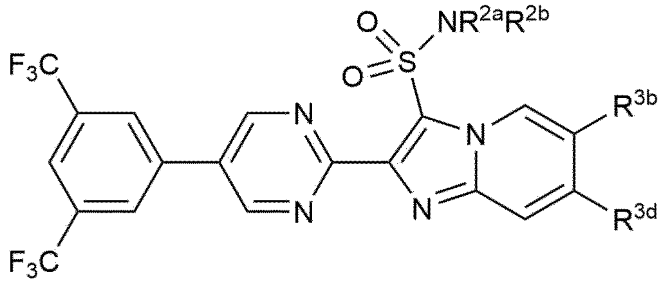

( L-53 )

(hereinafter referred to as compound (L-53)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX801).
The compound (L-53) wherein $R^{2a}$ is a methyl group, $R^{2b}$ is a hydrogen atom, $R^{3b}$ is a hydrogen atom, $R^{3d}$ is any one substituent mentioned in [Table 7A] to [Table 15A] (hereinafter referred to as compound group SX802).
The compound (L-53) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX803).
The compound (L-53) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX804).
The compound (L-53) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX805).
The compound (L-53) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX806).
The compound (L-53) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX807).
The compound (L-53) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX808).
The compound (L-53) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX809).
The compound (L-53) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX810).
The compound (L-53) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX811).
The compound (L-53) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX812).
The compound (L-53) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX813).
The compound (L-53) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX814).
The compound (L-53) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX815).
The compound (L-53) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX816).
A compound represented by formula (L-54):

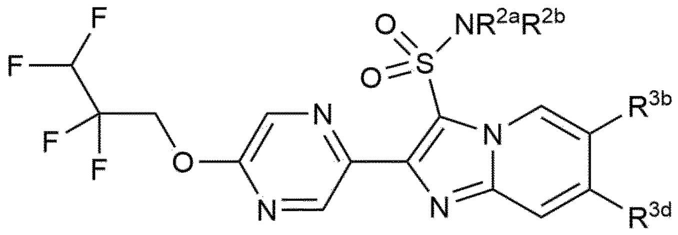

( L-54 )

(hereinafter referred to as compound (L-54)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX817).

The compound (L-54) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX818).

The compound (L-54) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX819).

The compound (L-54) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX820).

The compound (L-54) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX821).

The compound (L-54) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX822).

The compound (L-54) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX823).

The compound (L-54) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX824).

The compound (L-54) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX825).

The compound (L-54) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX826).

The compound (L-54) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX827).

The compound (L-54) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX828).

The compound (L-54) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX829).

The compound (L-54) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX830).

The compound (L-54) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX831).

The compound (L-54) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX832).

A compound represented by formula (L-55):

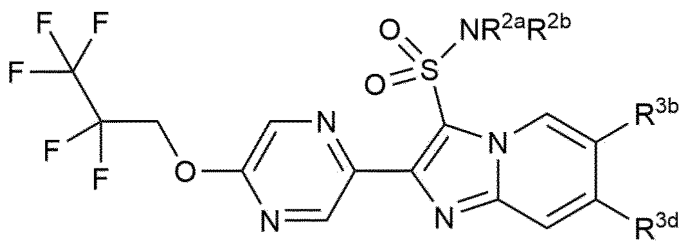

( L-55 )

(hereinafter referred to as compound (L-55)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX833).

The compound (L-55) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX834).

The compound (L-55) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX835).

The compound (L-55) wherein $R^{2a}$ is an ethyl group, $R^{2b}$ is a hydrogen atom, $R^{3b}$ is a hydrogen atom, and $R^{3d}$ is

any one substituent mentioned in [Table 7A] to [Table 15A] (hereinafter referred to as compound group SX836).

The compound (L-55) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX837).

The compound (L-55) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX838).

The compound (L-55) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX839).

The compound (L-55) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX840).

The compound (L-55) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX841).

The compound (L-55) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX842).

The compound (L-55) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX843).

The compound (L-55) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX844).

The compound (L-55) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX845).

The compound (L-55) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX846).

The compound (L-55) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX847).

The compound (L-55) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX848).

A compound represented by formula (L-56):

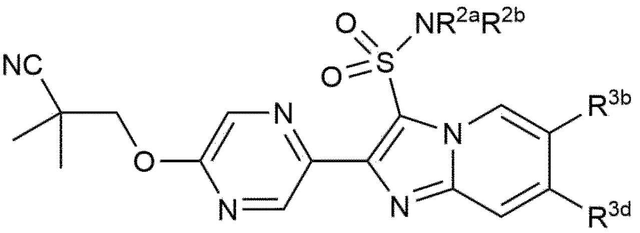

( L-56 )

(hereinafter referred to as compound (L-56)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX849).

The compound (L-56) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX850).

The compound (L-56) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX851).

The compound (L-56) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX852).

The compound (L-56) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX853).

The compound (L-56) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX854).

The compound (L-56) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX855).

The compound (L-56) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX856).

The compound (L-56) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX857).

The compound (L-56) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX858).

The compound (L-56) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX859).

The compound (L-56) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX860).
The compound (L-56) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX861).
The compound (L-56) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX862).
The compound (L-56) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX863).
The compound (L-56) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX864).
A compound represented by formula (L-57):

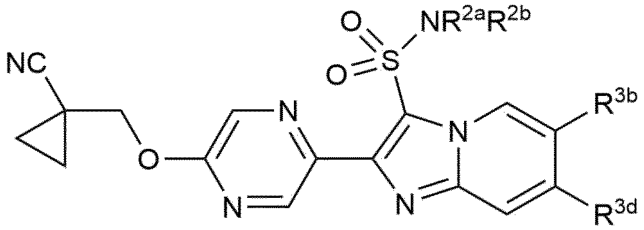

( L-57 )

(hereinafter referred to as compound (L-57)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX865).
The compound (L-57) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX866).
The compound (L-57) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX867).
The compound (L-57) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX868).
The compound (L-57) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX869).
The compound (L-57) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX870).
The compound (L-57) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX871).
The compound (L-57) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX872).
The compound (L-57) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX873).
The compound (L-57) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX874).
The compound (L-57) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX875).
The compound (L-57) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX876).
The compound (L-57) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX877).
The compound (L-57) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX878).
The compound (L-57) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX879).
The compound (L-57) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX880).
A compound represented by formula (L-58):

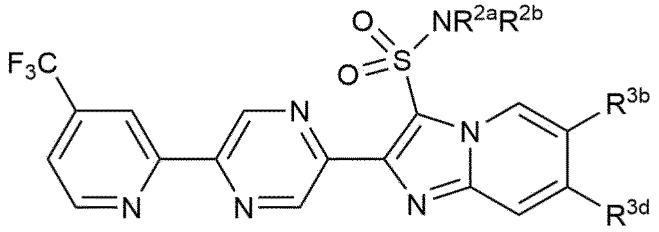

( L-58 )

(hereinafter referred to as compound (L-58)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX881).

The compound (L-58) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX882).

The compound (L-58) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX883).

The compound (L-58) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX884).

The compound (L-58) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX885).

The compound (L-58) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX886).

The compound (L-58) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX887).

The compound (L-58) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX888).

The compound (L-58) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX889).

The compound (L-58) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX890).

The compound (L-58) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX891).

The compound (L-58) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX892).

The compound (L-58) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX893).

The compound (L-58) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX894).

The compound (L-58) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX895).

The compound (L-58) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX896).

A compound represented by formula (L-59):

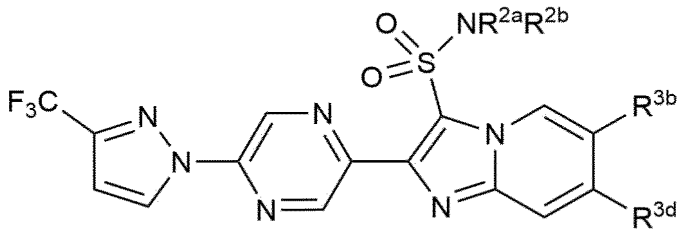

( L-59 )

(hereinafter referred to as compound (L-59)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX897).

The compound (L-59) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX898).

The compound (L-59) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX899).

The compound (L-59) wherein $R^{2a}$ is an ethyl group, $R^{2b}$ is a hydrogen atom, $R^{3b}$ is a hydrogen atom, and $R^{3d}$ is

any one substituent mentioned in [Table 7A] to [Table 15A] (hereinafter referred to as compound group SX900).

The compound (L-59) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX901).

The compound (L-59) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX902).

The compound (L-59) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX903).

The compound (L-59) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX904).

The compound (L-59) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX905).

The compound (L-59) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX906).

The compound (L-59) wherein $R^{2a}$ is a methyl group, $R^{2b}$ is a methyl group, $R^{3d}$ is a hydrogen atom, and $R^{3b}$ is any one substituent mentioned in [Table 7A] to [Table 15A] (hereinafter referred to as compound group SX907).

The compound (L-59) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX908).

The compound (L-59) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX909).

The compound (L-59) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX910).

The compound (L-59) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX911).

The compound (L-59) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX912).

A compound represented by formula (L-60):

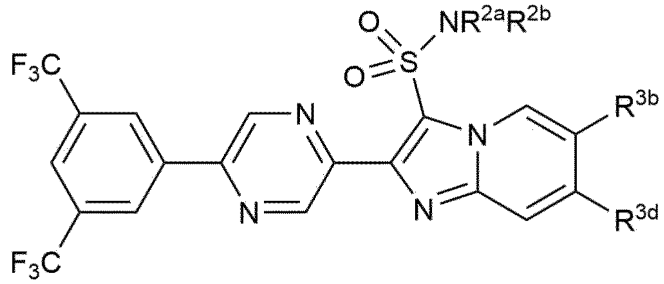

( L-60 )

(hereinafter referred to as compound (L-60)) wherein $R^{2a}$ and $R^{2b}$ are a hydrogen atom, $R^{3b}$ is a hydrogen atom, and $R^{3d}$ is any one substituent mentioned in [Table 7A] to [Table 15A] (hereinafter referred to as compound group SX913).

The compound (L-60) wherein $R^{2a}$ is a methyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX914).

The compound (L-60) wherein $R^{2a}$ is a methyl group, $R^{2b}$ is a methyl group, $R^{3b}$ is a hydrogen atom, and $R^{3d}$ is any one substituent mentioned in [Table 7A] to [Table 15A] (hereinafter referred to as compound group SX915).

The compound (L-60) wherein $R^{2a}$ is an ethyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX916).

The compound (L-60) wherein $R^{2a}$ is an ethyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX917).

The compound (L-60) wherein $R^{2a}$ is a propyl group, $R^{2b}$ is a hydrogen atom, $R^{3b}$ is a hydrogen atom, and $R^{3d}$ is any one substituent mentioned in [Table 7A] to [Table 15A] (hereinafter referred to as compound group SX918).

The compound (L-60) wherein $R^{2a}$ is a propyl group, $R^{2b}$ is a propyl group, $R^{3b}$ is a hydrogen atom, and $R^{3d}$ is any one substituent mentioned in [Table 7A] to [Table 15A] (hereinafter referred to as compound group SX919).

The compound (L-60) wherein $R^{2a}$ is a methyl group, $R^{2b}$ is an ethyl group, $R^{3b}$ is a hydrogen atom, and $R^{3d}$ is any one substituent mentioned in [Table 7A] to [Table 15A] (hereinafter referred to as compound group SX920).

The compound (L-60) wherein $R^{2a}$ and $R^{2b}$ are a hydrogen atom, $R^{3d}$ is a hydrogen atom, and $R^{3b}$ is any one substituent mentioned in [Table 7A] to [Table 15A] (hereinafter referred to as compound group SX921).

The compound (L-60) wherein $R^{2a}$ is a methyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX922).

The compound (L-60) wherein $R^{2a}$ is a methyl group, $R^{2b}$ is a methyl group, $R^{3d}$ is a hydrogen atom, and $R^{3b}$ is

any one substituent mentioned in [Table 7A] to [Table 15A] (hereinafter referred to as compound group SX923).

The compound (L-60) wherein $R^{2a}$ is an ethyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX924).

The compound (L-60) wherein $R^{2a}$ is an ethyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX925).

The compound (L-60) wherein $R^{2a}$ is a propyl group, $R^{2b}$ is a hydrogen atom, $R^{3d}$ is a hydrogen atom, and $R^{3b}$ is any one substituent mentioned in [Table 7A] to [Table 15A] (hereinafter referred to as compound group SX926).

The compound (L-60) wherein $R^{2a}$ is a propyl group, $R^{2b}$ is a propyl group, $R^{3d}$ is a hydrogen atom, and $R^{3b}$ is any one substituent mentioned in [Table 7A] to [Table 15A] (hereinafter referred to as compound group SX927).

The compound (L-60) wherein $R^{2a}$ is a methyl group, $R^{2b}$ is an ethyl group, $R^{3d}$ is a hydrogen atom, and $R^{3b}$ is any one substituent mentioned in [Table 7A] to [Table 15A] (hereinafter referred to as compound group SX928).

Next, Formulation Examples of the present compound are shown below. The "part(s)" represents "part(s) by weight". The present compound S represents the compounds mentioned in compound group SX1 to SX928.

Formulation Example 1

35 Parts of a mixture of a polyoxyethylene alkyl ether sulfate ammonium salt and silica (weight ratio: 1:1), 10 parts of any one of the present compound S, and 55 parts of water are mixed, followed by finely grinding by a wet grinding method to obtain a formulation.

Formulation Example 2

50 Parts of any one of the present compound S, 3 parts of calcium lignin sulfonate, 2 parts of sodium lauryl sulfate, and 45 parts of silica are subjected to mixing and grinding to obtain a formulation.

Formulation Example 3

5 Parts of any one of the present compound S, 9 parts of polyoxyethylene styryl phenyl ether, 5 parts of polyoxyethylene decyl ether (ethylene oxide addition number: 5), 6 parts of calcium dodecylbenzene sulfonate, and 75 parts of xylene are mixed to obtain a formulation.

Formulation Example 4

2 Parts of any one of the present compound S, 1 part of silica, 2 parts of calcium lignin sulfonate, 30 parts of bentonite, and 65 parts of kaolin clay are subjected to mixing and grinding and, after adding an appropriate amount of water, the mixture is kneaded, granulated by a granulator and then dried to obtain a formulation.

Formulation Example 5

10 Parts of any one of the present compound S is mixed with a mixture of 18 parts of benzyl alcohol and 9 parts of DMSO, and then 6.3 parts of GERONOL (registered trademark) TE250, 2.7 parts of Ethylan (registered trademark) NS-500LQ and 54 parts of solvent naphtha are added thereto, followed by mixing to obtain a formulation.

Formulation Example 6

0.1 Part of any one of the present compound S and 39.9 parts of kerosene are dissolved while mixing, and the mixture is charged in an aerosol container, and the aerosol container is filled with 60 parts of liquefied petroleum gas (mixture of propane, butane and isobutane; saturated vapor pressure: 0.47 MPa (25°C)) to obtain a formulation.

Formulation Example 7

0.2 Part of any one of the present compound S, 50 parts of pyrethrum extract dreg powder, 30 parts of Machilus thunbergii powder, and 19.8 parts of wood powder are mixed, and an appropriate amount of water is added thereto, and the mixture is kneaded and extracted by an extruder into a platelike sheet, and the resulting sheet is made a spiral-like form thereof by a punching machine to obtain a formulation

Next, Test Examples show an efficacy of the present compound on controlling harmful arthropods. The following test examples were carried out at 25°C.

Test Method 1

Each test compound is formulated according to the method mentioned in Formulation Example 1 to obtain each formulation, and water containing 0.03% by volume of Shindaine (registered trademark) is added thereto to prepare a diluted solution containing a prescribed concentration of each test compound.
Approximately 30 cotton aphids (Aphis gossypii) (all stages of life) are released onto cucumber (Cucumis sativus) seedlings (on the developmental stage of the second true leaf) planted in a container. After 1 day, each of the diluted solutions is sprayed over the seedlings in a ratio of 10 mL/seedling. After additional 5 days, the number of the surviving insects is examined and the control value is calculated by the following equation.

$$\text{Control value (\%)} = \{1 - (Cb \times Tai)/(Cai \times Tb)\} \times 100$$

where symbols in the equation represent the following meanings:

Cb: Number of test insects in non-treated area;
Cai: Number of surviving insects at the time of the investigation in non-treated area;
Tb: Number of test insects in treated area; and
Tai: Number of surviving insects at the time of the investigation in treated area.

Here, the "non-treated area" represents an area where the same treatment procedure as that of the treated area is carried out except for not using each test compound.

Test Example 1-1

The test was carried out by making the prescribed concentration 500 ppm and using the following present compounds as a test compound according to the test method 1. As a result of the test, the following present compounds showed 90% or more as the control value.

Present compounds: 1, 2, 3 and 4

Test Method 2

Each test compound is formulated according to the method mentioned in Formulation Example 5 to obtain each formulation, and water is added thereto to prepare a diluted solution containing a prescribed concentration of each test compound.
Each of the diluted solutions is irrigated into the plant foot of cucumber (Cucumis sativus) seedlings (on the developmental stage of the second true leaf) planted in a container in a ratio of 5 mL/seedlings. After 7 days, approximately 30 cotton aphids (Aphis gossypii) (all stages of life) are released onto the surfaces of leaves of the cucumber seedlings. After additional 6 days, the number of the surviving insects is examined and the control value is calculated by the following equation.

$$\text{Control value (\%)} = \{1 - (Cb \times Tai)/(Cai \times Tb)\} \times 100$$

where symbols in the equation represent the following meanings:

Cb: Number of test insects in non-treated area;
Cai: Number of surviving insects at the time of the investigation in non-treated area;
Tb: Number of test insects in treated area; and
Tai: Number of surviving insects at the time of the investigation in treated area.

Here, the "non-treated area" represents an area where the same treatment procedure as that of the treated area is carried out except for not using each test compound.

Test Example 2-1

The test is carried out by making the prescribed concentration 500 ppm or 200 ppm and using the following present compounds as a test compound according to the test method 2. As a result of the test, it is possible to confirm an

insecticidal effect on cotton aphid (Aphis gossypii).

Test Method 3

Each test compound is formulated according to the method mentioned in Formulation Example 1 to obtain each formulation, and water containing 0.03% by volume of Shindaine (registered trademark) is added thereto to prepare a diluted solution containing a prescribed concentration of each test compound.

Each of the diluted solutions is sprayed over cabbage (Brassicae oleracea) seedlings (on the developmental stage of the second to third true leaf) planted in a container in a ratio of 20 mL/seedling. Thereafter, the stem and leaf of the seedlings are cut out, and placed in a container lined with a filter paper. Five 2nd instar larvae of common cutworm (Spodoptera litura) are released into the container. After 5 days, the number of the surviving insects is counted, and the mortality of insects is calculated by the following equation.

Mortality (%) = (1 - Number of surviving insects/5) $\times$ 100

Test Example 3-1

The test was carried out by making the prescribed concentration 500 ppm and using the following present compounds as a test compound according to the test method 3. As a result of the test, the following present compounds showed 80% or more as the mortality.

Present compounds: 1, 2, 3 and 4

Test Example 4

Each test compound is formulated according to the method mentioned in Formulation Example 1 to obtain each formulation, and water containing 0.03% by volume of Shindaine (registered trademark) is added thereto to prepare a diluted solution containing a prescribed concentration of each test compound.

Each of the diluted solutions is sprayed over cabbage (Brassicae oleracea) seedlings (on the developmental stage of the second to third true leaf) planted in a container in a ratio of 20 mL/seedling. Thereafter, the stem and leaf of the seedlings are cut out, and placed in a container lined with a filter paper. Five 2nd instar larvae of diamondback moth (Plutella xylostella) are released into the container. After 5 days, the number of the surviving insects is counted, and the mortality of insects is calculated by the following equation.

Mortality (%) = (1 - Number of surviving insects/5) $\times$ 100

Test Example 4-1

The test was carried out by making the prescribed concentration 500 ppm and using the following present compounds as a test compound according to the test method 4. As a result of the test, the following present compounds showed 80% or more as the mortality.

Present compounds: 1, 2, 3 and 4

Test Method 5

Each test compound is dissolved in 50 $\mu$L of a mixed solution of polyoxyethylene sorbitan monococoate and acetone (at a volume ratio of 5:95) per 1 mg of the test compound. Water containing 0.03% by volume of Shindaine (registered trademark) is added thereto to prepare a diluted solution containing a prescribed concentration of each test compound.

Young seedlings of corns (Zea mays) are immersed in each of the diluted solutions for 30 seconds. Thereafter, two seedlings are placed in a petri dish (diameter: 90 mm), and ten 2nd instar larvae of western corn rootworm (Diabrotica virgifera virgifera) are released into the dish. After 5 days, the number of the dead insects is counted, and the mortality of insects is calculated by the following equation.

$$\text{Mortality (\%)} = (1 - \text{Number of dead insects}/10) \times 100$$

Test Example 5-1

The test was carried out by making the prescribed concentration 200 ppm and using the following present compounds as a test compound according to the test method 5. As a result of the test, the following present compounds showed 80% or more as the mortality.

Present compounds: 1, 2 and 3

Test Method 6

An acetone solution prepared by making the concentration of the test compound 800 ppm is poured into a 50 mL container, and the inner face of the container is uniformly coated with the test compound so as to make the coating amount of 40 $mg/m^2$, followed by drying.

Five German cockroach (Blattella germanica) male adults are released into the container, and the container is then covered with a lid. After the prescribed time is elapsed, the state of the German cockroach are examined. The mortality of insects is calculated by the following equation.

Mortality (%) = (Number of dead insects/Number of tested insects) $\times$ 100

Test Example 6-1

The test was carried out by making the prescribed elapsed time 3 days and using the following present compounds as a test compound according to the test method 6. As a result of the test, the following present compounds showed 80% or more as the mortality of insects.

Present compounds: 1 and 2

Test Method 7

An acetone solution prepared by making the concentration of the test compound 2,000 ppm is poured into a 20 mL container, and the inner face of the container is uniformly coated with the test compound so as to make the coating amount of 100 $mg/m^2$, followed by drying.

Five Asian longhorned tick nymphs (Haemaphysalis longicornis) are released into the container, and the container is then covered with a lid. After the prescribed time is elapsed, the state of the Asian longhorned tick nymphs are examined. The mortality of insects is calculated by the following equation.

Mortality (%) = (Number of dead insects/Number of tested insects) $\times$ 100

Test Example 7-1

The test was carried out by making the prescribed elapsed time 2 days and using the following present compounds as a test compound according to the test method 7. As a result of the test, it is possible to confirm an insecticidal effect on Asian longhorned tick (Haemaphysalis longicornis).

Test Method 8

An acetone solution prepared by making the concentration of the test compound 800 ppm is poured into a 20 mL container, and the inner face of the container is uniformly coated with the test compound so as to make the coating amount of 40 $mg/m^2$, followed by drying.

Five adult male houseflies (Musca domestica) are released into the container, and the container is then covered with a lid. After the prescribed time is elapsed, the state of the houseflies are examined. The mortality of insects is calculated by the following equation.

Mortality (%) = (Number of dead insects/Number of tested insects) $\times$ 100

Test Example 8-1

The test was carried out by making the prescribed elapsed time 1 day and using the following present compounds

as a test compound according to the test method 8. As a result of the test, the following present compounds showed 80% or more as the mortality of insects.

Present compounds: 1 and 2

Test Method 9

Each test compound is formulated according to the method mentioned in Formulation Example 5 to obtain each formulation, and water containing 0.03% by volume of Shindaine (registered trademark) is added thereto to prepare a diluted solution containing a prescribed concentration of each test compound.

Each of the diluted solutions is sprayed over cabbage (Brassicae oleracea) seedlings (on the developmental stage of the third to fourth true leaf) planted in a container in a ratio of 20 mL/seedling. Thereafter, ten 3rd instar larvae of common cutworm (Spodoptera litura) are released into the container. After 6 days, the number of the surviving insects is counted and the mortality of insects is calculated by the following equation.

Mortality (%) = (1 - Number of surviving insects/10) $\times$ 100

Test Example 9-1

The test was carried out by making the prescribed concentration 200 ppm and using the following present compounds as a test compound according to the test method 9. As a result of the test, the following present compounds showed 80% or more as the mortality.

Present compounds: 1, 2, 3 and 4

Test Example 9-2

The test is carried out by making the prescribed concentration 50 ppm and using the following present compounds as a test compound according to the test method 9. As a result of the test, it is possible to confirm an insecticidal effect on common cutworm (Spodoptera litura).

Test Method 10

Each test compound is formulated according to the method mentioned in Formulation Example 5 to obtain each formulation, and water containing 0.03% by volume of Shindaine (registered trademark) is added thereto to prepare a diluted solution containing a prescribed concentration of each test compound.

Each of the diluted solutions is sprayed over cabbage (Brassicae oleracea) seedlings (on the developmental stage of the third to fourth true leaf) planted in a container in a ratio of 20 mL/seedling. Thereafter, ten 3rd instar larvae of diamondback moth (Plutella xylostella) are released into the container. After 5 days, the number of the surviving insects is counted, and the mortality of insects is calculated by the following equation.

Mortality (%) = (1 - Number of surviving insects/10) $\times$ 100

Test Example 10-1

The test was carried out by making the prescribed concentration 200 ppm and using the following present compounds as a test compound according to the test method 10. As a result of the test, the following present compounds showed 80% or more as the mortality.

Present compounds: 1, 2, 3 and 4

Test Example 10-2

The test was carried out by making the prescribed concentration 50 ppm and using the following present compounds as a test compound according to the test method 10. As a result of the test, the following present compounds showed 80% or more as the mortality.

Present compounds: 1, 2, 3 and 4

Test Method 11

Each test compound is formulated according to the method mentioned in Formulation Example 5 to obtain each formulation, and water containing 0.03% by volume of Shindaine (registered trademark) is added thereto to prepare a diluted solution containing a prescribed concentration of each test compound.
Approximately thirty cotton aphids (Aphis gossypii) (all stages of life) are released onto cucumber (Cucumis sativus) seedlings (on the developmental stage of the second true leaf) planted in a container. After 1 day, each of the diluted solutions is sprayed over the seedlings in a ratio of 10 mL/seedling. After additional 5 days, the number of the surviving insects is examined and the control value is calculated by the following equation.

$$\text{Control value (\%)} = \{1 - (Cb \times Tai)/(Cai \times Tb)\} \times 100$$

where symbols in the equation represent the following meanings:

Cb: Number of test insects in non-treated area;
Cai: Number of surviving insects at the time of the investigation in non-treated area;
Tb: Number of test insects in treated area; and
Tai: Number of surviving insects at the time of the investigation in treated area.

Here, the "non-treated area" represents an area where the same treatment procedure as that of the treated area is carried out except for not using each test compound.

Test Example 11-1

The test was carried out by making the prescribed concentration 200 ppm and using the following present compounds as a test compound according to the test method 11. As a result of the test, the following present compounds showed 90% or more as the control value.

Present compounds: 1, 2 and 3

Test Example 11-2

The test was carried out by making the prescribed concentration 50 ppm and using the following present compounds as a test compound according to the test method 11. As a result of the test, the following present compounds showed 90% or more as the control value.

Present compounds: 1, 2 and 3

Test Method 12

Each test compound is formulated according to the method mentioned in Formulation Example 1 to obtain each formulation, and water is added thereto to prepare a diluted solution containing a prescribed concentration of each test compound.
Thirty final instar larvae of common house mosquito (Culex pipiens pallens) are released into the diluted solution and, after 1 day, the state of the common house mosquito larvae is examined and the mortality of insects is calculated by the following equation.

Mortality % = (Number of dead insects/Number of tested insects) $\times$ 100

Test Example 12-1

The test was carried out by making the prescribed concentration 3.5 ppm and using the following present compounds as a test compound according to the test method 12. As a result of the test, the following present compounds showed 90% or more as the control value.

Present compounds: 1 and 2

Test Method 13

Each present compound (1 mg) is dissolved in 10 μL of a mixed solution of xylene, DMF and a surfactant (xylene:DMF:surfactant = 4:4:1 (volume ratio)), followed by dilution with water containing 0.02% by volume of a spreader to prepare diluted solution A containing a prescribed concentration of the present compound.
Each present ingredient (1 mg) is dissolved in 10 μL of a mixed solution of xylene, DMF and a surfactant (xylene:DMF:surfactant = 4:4:1 (volume ratio)), followed by dilution with water containing 0.02% by volume of a spreader to prepare diluted solution B containing a prescribed concentration of the present ingredient.
The diluted solution A is mixed with the diluted solution B to prepare a diluted solution C.
Leaf discs of Cucumber (cucumber sativus) cotyledon (length 1.5 cm) are placed in each well of 24-well microplate. Two apterous adults and eight instar larvae of cotton aphids (Aphis gossypii) per one well are released and the diluted solution C is sprayed at 20 μL per one well. The group is defined as "treated area".
A well that is sprayed with 20 μL of water containing 0.02% by volume of a spreader instead of the diluted solution C is defined as "untreated area".
After drying the diluted solution C, the upper microplate is covered with a film sheet. After 5 days, the number of the surviving insects in each well is examined.
The controlling value is calculated by the following equation.

$$\text{Control value (\%)} = \{1 - (\text{Tai})/(\text{Cai})\} \times 100$$

where symbols in the equation represent the following meanings:

Cai: Number of surviving insects at the time of the examination in non-treated area;
Tai: Number of surviving insects at the time of the examination in treated area.

Specific diluted solutions C, which can confirm their effect according to the Test method 13, are shown in the following 1) to 5).

1) The diluted solution C comprises the combination mentioned in List A wherein the concentration of the present compound is 200 ppm and the concentration of the present ingredient is 2,000 ppm. In List A, Comp X represents any one compound selected from compound group SX1 to SX928.
List A:

Comp X + clothianidin; Comp X + thiamethoxam; Comp X + imidacloprid; Comp X + thiacloprid; Comp X + flupyradifurone;
Comp X + sulfoxaflor; Comp X + triflumezopyrim; Comp X + dicloromezotiaz; Comp X + beta-cyfluthrin; Comp X + tefluthrin; Comp X + fipronil; Comp X + chlorantraniliprole;
Comp X + cyantraniliprole; Comp X + tetraniliprole; Comp X + thiodicarb; Comp X + carbofuran; Comp X + fluxametamide;
Comp X + afoxolaner; Comp X + fluralaner; Comp X + broflanilide; Comp X + abamectin; Comp X + fluopyram; Comp X + fluensulfone; Comp X + fluazaindolizine; Comp X + tioxazafen; Comp X + flupyrimin; Comp X + Mycorrhizal Fungi;
Comp X + Bradyrhizobium japonicum TAll; Comp X + Bacillus firmus; Comp X + Bacillus firmus 1-1582; Comp X + Bacillus amyloliquefaciens; Comp X + Bacillus amyloliquefaciens FZB42;
Comp X + Bacillus amyloliquefaciens PTA4838; Comp X + Pasteuria nishizawae; Comp X + Pasteuria nishizawae Pn1; Comp X + Pasteuria penetrans; Comp X + tebuconazole; Comp X + prothioconazole; Comp X + metconazole;
Comp X + ipconazole; Comp X + triticonazole; Comp X + difenoconazole; Comp X + imazalil; Comp X + triadimenol;
Comp X + tetraconazole; Comp X + flutriafol; Comp X + mandestrobin; Comp X + azoxystrobin; Comp X + pyraclostrobin;
Comp X + trifloxystrobin; Comp X + fluoxastrobin; Comp X + picoxystrobin; Comp X + fenamidone; Comp X + metalaxyl; Comp X + metalaxyl-M; Comp X + fludioxonil; Comp X + sedaxane;
Comp X + penflufen; Comp X + fluxapyroxad; Comp X + benzovindiflupyr; Comp X + boscalid; Comp X + carboxin; Comp X + penthiopyrad; Comp X + flutolanil; Comp X + captan; Comp X + thiram; Comp X + tolclofos-methyl; Comp X + thiabendazole; Comp X + ethaboxam; Comp X + mancozeb; Comp X + picarbutrazox; Comp X + oxathiapiprolin; Comp X + silthiofam; Comp X + inpyrfluxam.

2) The diluted solution C comprises the combination recited in List A wherein a concentration of the present compound is 200 ppm, and a concentration of the present ingredient is 200 ppm.
3) The diluted solution C comprises the combination recited in List A wherein a concentration of the present compound is 500 ppm, and a concentration of the present ingredient is 50 ppm.
4) The diluted solution C comprises the combination recited in List A wherein a concentration of the present compound is 500 ppm, and a concentration of the present ingredient is 5 ppm.
5) The diluted solution C comprises the combination recited in List A wherein a concentration of the present compound is 500 ppm, and a concentration of the present ingredient is 0.5 ppm.

[Industrial Applicability]

The present compound exhibits excellent control effect on harmful arthropods.

## Claims

**1.** A compound represented by formula (I):

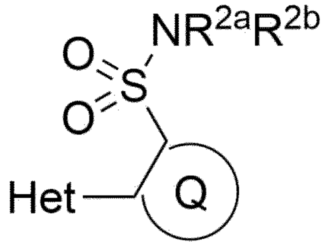

( I )

[wherein,

Q represented by the following formula:

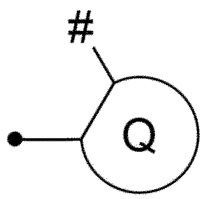

represents a group represented by formula Q1, a group represented by formula Q2, a group represented by formula Q4, a group represented by formula Q5, a group represented by formula Q6, or a group represented by formula Q7 (wherein # represents the binding site to the sulfur atom, and • represents the binding site to Het):

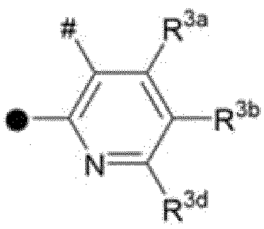

Q1

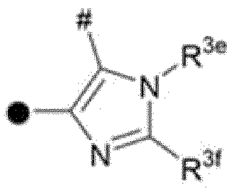

Q2

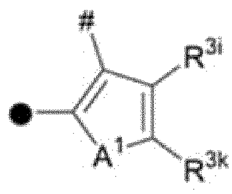

Q4

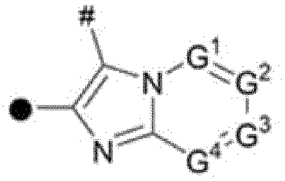
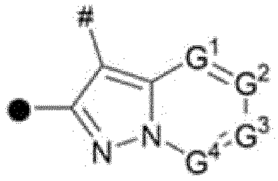
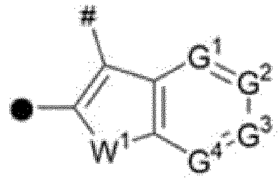

Q5 Q6 Q7

$A^1$ represents $NR^{5a}$, an oxygen atom or a sulfur atom, the combination of $G^1$, $G^2$, $G^3$ and $G^4$ represents:

a combination wherein $G^1$ is a nitrogen atom or $CR^{3a}$, $G^2$ is $CR^{3b}$, $G^3$ is $CR^{3d}$, and $G^4$ is $CR^{3c}$;
a combination wherein $G^1$ is $CR^{3a}$, $G^2$ is a nitrogen atom, $G^3$ is $CR^{3d}$, and $G^4$ is $CR^3$c;
a combination wherein $G^1$ is $CR^{3a}$, $G^2$ is $CR^{3b}$, $G^3$ is a nitrogen atom, and $G^4$ is $CR^3$; or
a combination wherein $G^1$ is $CR^{3a}$, $G^2$ is $CR^{3b}$, $G^3$ is $CR^{3d}$, and $G^4$ is a nitrogen atom,
$W^1$ represents an oxygen atom or a sulfur atom,
$R^{2a}$ and $R^{2b}$ are identical to or different from each other, and each represents a C1-C6 alkyl group optionally substituted with one or more halogen atoms, a C3-C6 cycloalkyl group optionally substituted with one or more halogen atoms, or a hydrogen atom,
$R^{2a}$ and $R^{2b}$ may be combined with the nitrogen atom to which they are attached to form an aziridinyl group, an azetidinyl group, a pyrrolidinyl group, or a piperidyl group,
$R^{3a}$, $R^{3b}$, $R^{3c}$, $R^{3d}$, $R^{3f}$, $R^{3i}$ and $R^{3k}$ are identical to or different from each other, and each represents a C1-C6 chain hydrocarbon group optionally substituted with one or more substituents selected from Group B, a C3-C7 cycloalkyl group optionally substituted with one or more substituents selected from Group E, a phenyl group optionally substituted with one or more substituents selected from Group H, a 5- or 6-membered aromatic heterocyclic group optionally substituted with one or more substituents selected from Group H, $OR^{12}$, $NR^{11}R^{12}$, $NR^{11a}R^{12a}$, $NR^{24}NR^{11}R^{12}$, $NR^{24}$ $OR^{11}$, $NR^{11}C(O)R^{13}$ $NR^{24}NR^{11}C(O)R^{13}$, $NR^{11}C(O)OR^{14}$, $NR^{24}$ $NR^{11}C(O)OR^{14}$ , $NR^{11}C(O)NR^{31}R^{32}$, $NR^{24}NR^{11}C(O)NR^{31}R^{32}$, $N=CHNR^{31}R^{32}$ $N=S(O)_pR^{15}R^{16}$, $C(O)R^{13}$, $C(O)OR^{17}$, $C(O)NR^{31}R^{32}$, $C(O)NR^{11}S(O)_2R^{23}$, $CR^{30}=NOR^{17}$, $NR^{11}CR^{24}=NOR^{17}$, $S(O)_qR^{23}$, a cyano group, a nitro group, a hydrogen atom, or a halogen atom,
$R^{3e}$ represents a C1-C6 alkyl group optionally substituted with one or more halogen atoms, a phenyl group optionally substituted with one or more substituents selected from Group H, or a hydrogen atom,
when Q is a group represented by formula Q1, the two adjacent substituents among $R^{3a}$, $R^{3b}$ and $R^{3d}$ may be combined with two carbon atoms to which they are attached to form a benzene ring, a pyrrole ring, a furan ring, a thiophene ring, a pyrazole ring, an imidazole ring, an oxazole ring, an isoxazole ring, a thiazole ring, an isothiazole ring, an oxadiazole ring, a thiadiazole ring, a pyridine ring, a pyridazine ring, a pyrimidine ring, a pyrazine ring {the benzene ring, the pyrrole ring, the furan ring, the thiophene ring, the pyrazole ring, the imidazole ring, the oxazole ring, the isoxazole ring, the thiazole ring, the isothiazole ring, the pyridine ring, the pyridazine ring, the pyrimidine ring and the pyrazine ring may be optionally substituted with one or more substituents selected from Group H}, or a triazole ring optionally substituted with one or more substituents selected from Group I,
p represents 0 or 1,
q represents 0, 1 or 2,
$R^{30}$ represents a C1-C6 chain hydrocarbon group optionally substituted with one or more halogen atoms, a halogen atom, $OR^{35}$, $NR^{36}R^{37}$, or a hydrogen atom,
$R^{17}$ represents a C1-C6 chain hydrocarbon group optionally substituted with one or more halogen atoms, a phenyl group optionally substituted with one or more substituents selected from Group D, or a hydrogen atom,
$R^{12}$ represents a C1-C6 chain hydrocarbon group optionally substituted with one or more substituents selected from Group F, a C3-C7 cycloalkyl group optionally substituted with one or more substituents selected from Group J, a C3-C7 cycloalkenyl group optionally substituted with one or more substituents selected from Group J, a phenyl group optionally substituted with one or more substituents selected from Group D, a 6-membered aromatic heterocyclic group optionally substituted with one or more substituents selected from Group D, a hydrogen atom, or $S(O)_2R^{23}$,
$R^{23}$ represents a C1-C6 chain hydrocarbon group optionally substituted with one or more halogen atoms, or a phenyl group optionally substituted with one or more substituents selected from Group D,
$R^{11a}$ and $R^{12a}$, together with the nitrogen atoms to which they are attached, represent a 3- to 7-membered

nonaromatic heterocyclic group optionally substituted with one or more substituents selected from Group E,

$R^{13}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, a phenyl group optionally substituted with one or more substituents selected from Group D, a 5- or 6-membered aromatic heterocyclic group optionally substituted with one or more substituents selected from Group D, or a hydrogen atom,

$R^{14}$ represents a C1-C6 chain hydrocarbon group optionally substituted with one or more halogen atoms, a C3-C7 cycloalkyl group optionally substituted with one or more halogen atoms, a (C3-C6 cycloalkyl) C1-C3 alkyl group optionally substituted with one or more halogen atoms, or a phenyl C1-C3 alkyl group {the phenyl moiety in the phenyl C1-C3 alkyl group may be optionally substituted with one or more substituents selected from Group D},

$R^{15}$ and $R^{16}$ are identical to or different from each other, and each represents a C1-C6 alkyl group optionally substituted with one or more halogen atoms,

$R^{31}$ represents a C1-C6 alkyl group optionally substituted with one or more halogen atoms, or a hydrogen atom,

$R^{32}$ represents a C1-C6 chain hydrocarbon group optionally substituted with one or more substituents selected from Group F, a C3-C7 cycloalkyl group optionally substituted with one or more substituents selected from Group J, or a hydrogen atom,

Het represents a group represented by formula Het1, a group represented by formula Het2, a group represented by formula Het3, a group represented by formula Het4, a group represented by formula Het5, or a group represented by formula Het6:

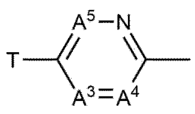
Het1

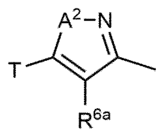
Het2

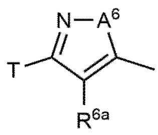
Het3

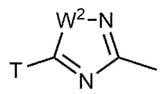
Het4

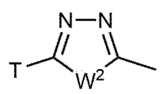
Het5

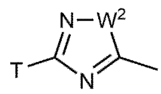
Het6

$A^2$ represents an oxygen atom, a sulfur atom, or $NR^{5b}$,

$A^3$ represents a nitrogen atom or $CR^{4b}$,

$A^4$ represents a nitrogen atom or $CR^{4c}$,

$A^5$ represents a nitrogen atom or $CR^{4d}$,

$A^6$ represents an oxygen atom, a sulfur atom, or $NR^{20}$,

$W^2$ represents an oxygen atom or a sulfur atom,

$R^{4b}$, $R^{4c}$, $R^{4d}$ and $R^{6a}$ are identical to or different from each other, and each represents a C1-C6 chain hydrocarbon group optionally substituted with one or more halogen atoms, a nitro group, $OR^{18}$, $NR^{18}R^{19}$, a cyano group, an amino group, a halogen atom, or a hydrogen atom,

$R^{20}$ is identical to or different from each other, and represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, or a (C3-C7 cycloalkyl) C1-C6 alkyl group optionally substituted with one or more halogen atoms,

T represents a C1-C10 chain hydrocarbon group, a (C1-C5 alkoxy) C2-C5 alkyl group, a (C3-C5 alkenyloxy) C2-C5 alkyl group, a (C3-C5 alkynyloxy) C2-C5 alkyl group, a (C1-C5 alkyl)-$S(O)_w$-(C2-C5 alkyl) group, a (C3-C5 alkenyl)-$S(O)_w$-(C2-C5 alkyl) group, a (C3-C5 alkynyl)-$S(O)_w$-(C2-C5 alkyl) group, a (C1-C5 alkyl)-C(O)-(C1-C5 alkyl) group {the C1-C10 chain hydrocarbon group, the (C1-C5 alkoxy) C2-C5 alkyl group, the (C3-C5 alkenyloxy) C2-C5 alkyl group, the (C3-C5 alkynyloxy) C2-C5 alkyl group, the (C1-C5 alkyl)-$S(O)_w$-(C2-C5 alkyl) group, the (C3-C5 alkenyl)-$S(O)_x$-(C2-C5 alkyl) group, the (C3-C5 alkynyl)-$S(O)_w$-(C2-C5 alkyl) group, and the (C1-C5 alkyl)-C(O)-(C1-C5 alkyl) group are substituted with one or more substituents selected from the group consisting of a cyano group and a halogen atom}, a (C3-C7 cycloalkyl) C1-C3 alkyl group which is substituted with one or more substituents selected from Group G, a C3-C7 cycloalkyl group which is substituted with one or more substituents selected from Group G, $OR^1$, $S(O)_vR^1$, $OS(O)_2R^1$, $CH_2OR^1$, $NR^1R^{29}$, $C(O)R^1$, $C(O)NR^1R^{29}$, $NR^{29}C(O)R^1$, $N=CR^1R^{30}$, a group represented by formula T-1, a group represented by formula T-2, a group represented by formula T-3, a group represented by formula T-4, a group represented by formula T-5, a group represented by formula T-6, a group represented by formula T-7, a group represented by formula T-8, a group represented by formula T-9, a group represented by formula T-10, a group represented by formula T-11, or a group represented by formula T-12:

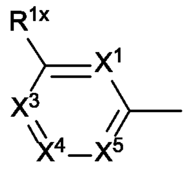
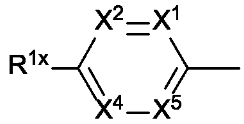
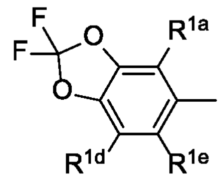
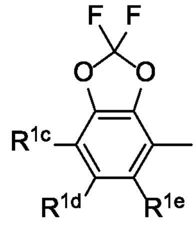

T-1 T-2 T-3 T-4

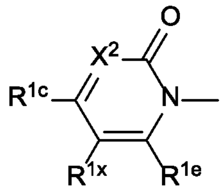
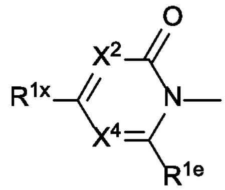
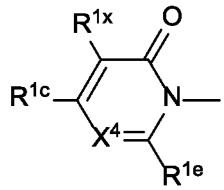

T-5 T-6 T-7

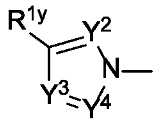
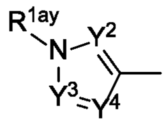
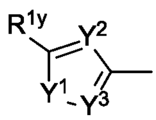
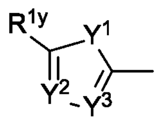
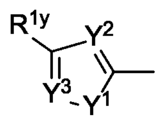

T-8 T-9 T-10 T-11 T-12

$X^1$ represents a nitrogen atom or $CR^{1a}$,
$X^2$ represents a nitrogen atom or $CR^{1b}$,
$X^3$ represents a nitrogen atom or $CR^{1c}$,
$X^4$ represents a nitrogen atom or $CR^{1d}$,
$X^5$ represents a nitrogen atom or $CR^{1e}$,
$R^{1x}$ represents a C1-C5 chain hydrocarbon group which is substituted with one or more halogen atoms, $OR^7$, $OS(O)_2R^7$, $S(O)_mR^7$, $NR^8S(O)_2R^7$, or a halogen atom,
$R^{1a}$ , $R^{1b}$, $R^{1c}$, $R^{1d}$ and $R^{1e}$ are identical to or different from each other, and each represents a C1-C6 chain hydrocarbon group optionally substituted with one or more halogen atoms, a C3-C6 cycloalkyl group optionally substituted with one or more halogen atoms, a halogen atom, or a hydrogen atom,
$Y^1$ represents $NR^{25}$, an oxygen atom or a sulfur atom,
$Y^2$ represents a nitrogen atom or $CR^{26}$,
$Y^3$ represents a nitrogen atom or $CR^{27}$,
$Y^4$ represents a nitrogen atom or $CR^{28}$,
$R^{5a}$, $R^{5b}$ and $R^{25}$ are identical to or different from each other, and each represents a C1-C6 chain hydrocarbon group optionally substituted with one or more halogen atoms, a C1-C6 alkoxy group optionally substituted with one or more halogen atoms, a C3-C7 cycloalkyl group optionally substituted with one or more halogen atoms, a (C3-C7 cycloalkyl) C1-C6 alkyl group optionally substituted with one or more halogen atoms, or a hydrogen atom,
$R^{26}$, $R^{27}$ and $R^{28}$ are identical to or different from each other, and each represents a C1-C6 chain hydrocarbon group optionally substituted with one or more halogen atoms, a C3-C6 cycloalkyl group optionally substituted with one or more halogen atoms, a halogen atom, or a hydrogen atom,
$R^{1y}$ represents a C1-C5 chain hydrocarbon group which is substituted with one or more halogen atoms, $OR^7$, $OS(O)_2R^7$, $S(O)_mR^7$, $NR^8S(O)_2R^7$, a cyano group, or a halogen atom,
$R^{1ay}$ and $R^7$ are identical to or different from each other, and each represents a C1-C6 chain hydrocarbon group which is substituted with one or more halogen atoms,
m and v are identical to or different from each other, and each represents 0, 1 or 2,
w and t are identical to or different from each other, and each represents 0, 1 or 2,
$R^1$ represents a C1-C10 chain hydrocarbon group, a (C1-C5 alkoxy) C2-C5 alkyl group, a (C3-C5 alkenyloxy) C2-C5 alkyl group, a (C3-C5 alkynyloxy) C2-C5 alkyl group, a (C1-C5 alkyl)-$S(O)_t$-(C2-C5 alkyl) group, a (C3-C5 alkenyl)-$S(O)_t$-(C2-C5 alkyl) group, a (C3-C5 alkynyl)-$S(O)_t$-(C2-C5 alkyl) group, a (C1-C5 alkyl)-C(O)-(C1-C5 alkyl) group {the C1-C10 chain hydrocarbon group, the (C1-C5 alkoxy) C2-C5 alkyl group, the (C3-C5 alkenyloxy) C2-C5 alkyl group, the (C3-C5 alkynyloxy) C2-C5 alkyl group, the (C1-C5 alkyl)-S(O)$_t$-(C2-C5 alkyl) group, the (C3-C5 alkenyl)-S(O)$_t$-(C2-C5 alkyl) group, the (C3-C5 alkynyl)-S(O)$_t$-(C2-C5 alkyl) group, and the (C1-C5 alkyl)-C(O)-(C1-C5 alkyl) group are substituted with one or more substituents selected from the group consisting of a cyano group and a halogen atom}, a (C3-C7 cycloalkyl) C1-C3 alkyl group which is substituted with one or more substituents selected from Group G, or a C3-C7 cycloalkyl group which is substituted with one or more substituents selected from Group G,

$R^{18}$ and $R^{35}$ are identical to or different from each other, and each represents a C1-C6 chain hydrocarbon group optionally substituted with one or more halogen atoms, and

$R^8$, $R^{11}$, $R^{19}$, $R^{24}$, $R^{29}$, $R^{36}$ and $R^{37}$ are identical to or different from each other, and each represents a C1-C6 chain hydrocarbon group optionally substituted with one or more halogen atoms, or a hydrogen atom.

Group B: the group consisting of a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a C3-C6 cycloalkyl group optionally substituted with one or more halogen atoms, a cyano group, a hydroxy group, and a halogen atom.

Group C: the group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, and a halogen atom.

Group D: the group consisting of a C1-C6 chain hydrocarbon group optionally substituted with one or more halogen atoms, a hydroxy group, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a sulfanyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, an amino group, $NHR^{21}$, $NR^{21}R^{22}$, $C(O)R^{21}$, $OC(O)R^{21}$, $C(O)OR^{21}$, a cyano group, a nitro group, and a halogen atom.

$R^{21}$ and $R^{22}$ are identical to or different from each other, and each represents a C1-C6 alkyl group optionally substituted with one or more halogen atoms.

Group E: the group consisting of a C1-C6 chain hydrocarbon group optionally substituted with one or more halogen atoms, a C1-C6 alkoxy group optionally substituted with one or more halogen atoms, a C3-C6 alkenyloxy group optionally substituted with one or more halogen atoms, a C3-C6 alkynyloxy group optionally substituted with one or more halogen atoms, a halogen atom, an oxo group, a hydroxy group, a cyano group, and a nitro group.

Group F: the group consisting of a C1-C6 alkoxy group optionally substituted with one or more halogen atoms, a phenyl group optionally substituted with one or more substituents selected from Group D, a 5- or 6-membered aromatic heterocyclic group optionally substituted with one or more substituents selected from Group D, a C3-C7 cycloalkyl group optionally substituted with one or more halogen atoms, a 3- to 7-membered nonaromatic heterocyclic group optionally substituted with one or more substituents selected from Group C, an amino group, $NHR^{21}$, $NR^{21}R^{22}$, a halogen atom, and a cyano group.

Group G: the group consisting of a halogen atom, a C1-C6 haloalkyl group and a cyano group.

Group H: the group consisting of a C1-C6 alkyl group optionally substituted with one or more halogen atoms, $OR^{10}$, $NR^9R^{10}$, $C(O)R^{10}$, $C(O)NR^9R^{10}$, $OC(O)R^9$, $OC(O)OR^9$, $NR^{10}C(O)R^9$, $NR^{10}C(O)OR^9$, $C(O)OR^{18}$, a halogen atom, a nitro group, a cyano group, an amino group, and a 5- or 6-membered aromatic heterocyclic group.

$R^9$ represents a C1-C6 alkyl group optionally substituted with one or more halogen atoms, or a C3-C6 cycloalkyl group optionally substituted with one or more halogen atoms, and

$R^{10}$ represents a C1-C6 alkyl group optionally substituted with one or more halogen atoms, a C3-C6 cycloalkyl group optionally substituted with one or more halogen atoms, or a hydrogen atom.

Group I: the group consisting of a C2-C6 chain hydrocarbon group optionally substituted with one or more halogen atoms, a C3-C6 cycloalkyl group optionally substituted with one or more halogen atoms, a phenyl group optionally substituted with one or more substituents selected from Group D, a 5- or 6-membered aromatic heterocyclic group optionally substituted with one or more substituents selected from Group D, a C2-C6 alkylcarbonyl group optionally substituted with one or more halogen atoms, a C2-C6 alkoxycarbonyl group optionally substituted with one or more halogen atoms, an aminocarbonyl group, a (C1-C6 alkyl) aminocarbonyl group optionally substituted with one or more halogen atoms, a methyl group, and a di(C1-C4 alkyl)aminocarbonyl group optionally substituted with one or more halogen atoms.

Group J: the group consisting of a C1-C6 alkyl group optionally substituted with one or more halogen atoms, a halogen atom, and a cyano group] (hereinafter referred to as present compound N) or an N-oxide thereof (hereinafter a compound represented by formula (I) or an N-oxide thereof is referred to as present compound).

**2.** The compound according to claim 1, wherein Q is a group represented by formula Q4 or a group represented by formula Q7, or an N-oxide thereof.

**3.** The compound according to claim 1, wherein Q is a group represented by formula Q5 or a group represented by formula Q6, or an N-oxide thereof.

**4.** The compound according to claim 1, wherein Q is a group represented by formula Q1 or a group represented by formula Q2, or an N-oxide thereof.

**5.** The compound according to claim 1, wherein Q is a group represented by formula Q5, or an N-oxide thereof.

**6.** The compound according to claim 5, wherein $G^1$ is $CR^{3a}$, $G^2$ is $CR^{3b}$, $G^3$ is $CR^{3d}$, and $G^4$ is $CR^{3c}$, or an N-oxide thereof.

**7.** The compound according to any one of claims 1 to 6, wherein Het is a group represented by formula Het1, $A^3$ is a nitrogen atom or $CR^{4b}$, $A^4$ is $CR^{4c}$, and $A^5$ is a nitrogen atom or $CR^{4d}$, or an N-oxide thereof.

**8.** A harmful arthropod control composition comprising the compound according to any one of claims 1 to 7, or an N-oxide thereof, and an inert carrier.

**9.** A composition comprising one or more ingredients selected from the group consisting of Group (a), Group (b), Group (c) and Group (d), and the compound according to any one of claims 1 to 7, or an N-oxide thereof:

Group (a): the group consisting of insecticidal active ingredients, miticidal active ingredients and nematicidal active ingredients;
Group (b): fungicidal active ingredients;
Group (c): plant growth regulatory ingredients; and
Group (d): repellent ingredients.

**10.** A method for controlling harmful arthropods, which comprises applying an effective amount of the compound according to any one of claims 1 to 7, or an N-oxide thereof, or an effective amount of the composition according to claim 9 to harmful arthropods or habitats where harmful arthropods live.

**11.** A seed or a vegetative reproduction organ holding an effective amount of the compound according to any one of claims 1 to 7, or an N-oxide thereof, or an effective amount of the composition according to claim 9.

**INTERNATIONAL SEARCH REPORT**

International application No. **PCT/JP2023/037148**

**A. CLASSIFICATION OF SUBJECT MATTER**

***C07D 471/04***(2006.01)i; ***A01M 1/20***(2006.01)i; ***A01N 43/40***(2006.01)i; ***A01N 43/50***(2006.01)i; ***A01N 43/54***(2006.01)i; ***A01N 43/56***(2006.01)i; ***A01N 43/58***(2006.01)i; ***A01N 43/60***(2006.01)i; ***A01N 43/653***(2006.01)i; ***A01N 43/90***(2006.01)i; ***A01N 47/02***(2006.01)i; ***A01N 53/14***(2006.01)i; ***A01P 7/02***(2006.01)i; ***A01P 7/04***(2006.01)i; ***A61K 31/497***(2006.01)i; ***A61K 31/501***(2006.01)i; ***A61P 33/14***(2006.01)i

FI: C07D471/04 108Q; A01M1/20; A01N43/40 102; A01N43/50 G; A01N43/54 B; A01N43/56 D; A01N43/58 A; A01N43/60; A01N43/653 G; A01N43/90 103; A01N47/02; A01N53/14; A01P7/02; A01P7/04; A61K31/497; A61K31/501; A61P33/14; C07D471/04 CSP

According to International Patent Classification (IPC) or to both national classification and IPC

**B. FIELDS SEARCHED**

Minimum documentation searched (classification system followed by classification symbols)

C07D471/04; A01M1/20; A01N43/40; A01N43/50; A01N43/54; A01N43/56; A01N43/58; A01N43/60; A01N43/653; A01N43/90; A01N47/02; A01N53/14; A01P7/02; A01P7/04; A61K31/497; A61K31/501; A61P33/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2022/186133 A1 (NIHON NOHYAKU CO., LTD.) 09 September 2022 (2022-09-09) entire text | 1-11 |
| A | JP 2018-523664 A (BAYER CROPSCIENCE AG) 23 August 2018 (2018-08-23) entire text | 1-11 |
| A | JP 2018-505881 A (BAYER CROPSCIENCE AG) 01 March 2018 (2018-03-01) entire text | 1-11 |
| A | JP 2017-512191 A (BAYER CROPSCIENCE AG) 18 May 2017 (2017-05-18) entire text | 1-11 |
| A | JP 2018-536639 A (BAYER CROPSCIENCE AG) 13 December 2018 (2018-12-13) entire text | 1-11 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 December 2023** | **26 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/037148**

| Patent document cited in search report | Publication date (day/month/year) | Patent family member(s) | Publication date (day/month/year) |
|---|---|---|---|
| WO 2022/186133 A1 | 09 September 2022 | (Family: none) | |
| JP 2018-523664 A | 23 August 2018 | US 2018/0271099 A1 | |
| | | US 2020/0029567 A1 | |
| | | WO 2017/025419 A2 | |
| | | EP 3331870 A2 | |
| | | EP 3896065 A1 | |
| | | EP 3896066 A2 | |
| | | KR 10-2018-0032640 A | |
| | | CN 108137548 A | |
| JP 2018-505881 A | 01 March 2018 | US 2018/0002345 A1 | |
| | | WO 2016/124563 A1 | |
| | | EP 3253210 A1 | |
| | | KR 10-2017-0124533 A | |
| | | CN 107428764 A | |
| JP 2017-512191 A | 18 May 2017 | US 2017/0073342 A1 | |
| | | US 2019/0241564 A1 | |
| | | US 2023/0087882 A1 | |
| | | WO 2015/121136 A1 | |
| | | EP 3107912 A1 | |
| | | KR 10-2016-0122809 A | |
| | | CN 106414441 A | |
| JP 2018-536639 A | 13 December 2018 | US 2018/0305353 A1 | |
| | | WO 2017/072039 A1 | |
| | | EP 3368521 A1 | |
| | | KR 10-2018-0069005 A | |
| | | CN 108430986 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022165593 A
- WO 2016052455 A
- WO 2018221720 A
- WO 2015163478 A
- WO 2016121969 A
- WO 2017069105 A
- WO 2017065228 A
- WO 2017077911 A
- WO 2016121970 A
- WO 2017164108 A
- WO 2017150209 A
- WO 2017169894 A
- WO 2018052119 A
- WO 2018101424 A
- WO 2019189731 A


### Non-patent literature cited in the description


- Manual on development and use of FAO and WHO Specifications for pesticides, FAO Plant Production and Protection Papers. FAO/WHO Joint Meeting on Pesticide Specifications. 2016, 271-276